# EUROPEAN PATENT APPLICATION

(11) **EP 2 813 849 A1**
(43) Date of publication of application: **17.12.2014**
(21) Application number: 13171260.6
(22) Date of filing: 10.06.2013
(51) Int. Cl.: G01N 33/569

(54) **EBV-specific immune signature as diagnostic markers in Chronic Fatigue Syndrome (CFS)**

(71) Applicant: Charité-Universitätsmedizin Berlin (Charité), 10117 Berlin (DE); JPT Peptide Technologies GmbH, 12489 Berlin (DE)
(72) Inventor: Prof. Dr. Scheibenbogen, Carmen, 12203 Berlin (DE); Prof. Dr. Volk, Hans-Dieter, 10117 Berlin (DE); Prof. Dr. Priller, Josef, 10435 Berlin (DE); PD Dr. Ruprecht, Klemens, 14109 Berlin (DE); Dr. Reimer, Ulf, 10437 Berlin (DE); Dr. Wenschuh, Holger, 12555 Berlin (DE)
(74) Representative: Schiweck, Weinzierl & Koch

(57) **Abstract**

The present invention relates to the development of an in vitro method for diagnosing Chronic Fatigue Syndrome by determining the presence, absence or the amount of at least one marker characteristic of an Epstein-Barr virus (EBV) infection in a sample obtained from the body of an individual. Specifically, the marker characteristic of EBV infection is selected from the group consisting of EBNA1 EBNA3, EBNA4, EBNA6, BZLF1, LMP1 and VP26. Furthermore, the invention relates to a device for the diagnosis of Chronic Fatigue Syndrome, wherein the device comprises a solid phase having immobilized thereon at least one marker or protein fragment thereof, wherein the marker is selected from the group consisting of EBNA1, EBNA3, EBNA4, EBNA6, BZLF1, LMP1 and VP26. In addition, the invention relates to the use of at least one marker or a protein fragment thereof for diagnosis of Chronic Fatigue, wherein the marker is selected from the group consisting of EBNA1 EBNA3, EBNA4, EBNA6, BZLF1, LMP1 and VP26.

SEQUENCE LISTING

<110>  Charité Berlin
       JPT

<120>  EBV-specific immune signature as diagnostic markers in Chronic
       Fatigue Syndrome

<130>  CHA14501EP

<160>  135

<170>  PatentIn version 3.5

<210>  1
<211>  245
<212>  PRT
<213>  Artificial

<220>
<223>  EBV BZLF1 Strain AG876

<400>  1

Met Met Asp Pro Asn Ser Thr Ser Glu Asp Val Lys Phe Thr Pro Asp
1               5                   10                  15

Pro Tyr Gln Val Pro Phe Val Gln Ala Phe Asp Gln Ala Thr Arg Val
            20                  25                  30

Tyr Gln Asp Leu Gly Gly Pro Ser Gln Ala Pro Leu Pro Cys Val Leu
        35                  40                  45

Trp Pro Val Leu Pro Glu Pro Leu Pro Gln Gly Gln Leu Thr Ala Tyr
    50                  55                  60

His Val Ser Ala Ala Pro Thr Gly Ser Trp Phe Pro Ala Pro Gln Pro
65                  70                  75                  80

Ala Pro Glu Asn Ala Tyr Gln Ala Tyr Ala Ala Pro Gln Leu Phe Pro
                85                  90                  95

Val Ser Asp Ile Thr Gln Asn Gln Gln Thr Asn Gln Ala Gly Gly Glu
            100                 105                 110

Ala Pro Gln Pro Gly Asp Asn Ser Thr Val Gln Pro Ala Ala Ala Val
        115                 120                 125

Val Phe Ala Cys Pro Gly Ala Asn Gln Gly Gln Gln Leu Ala Asp Ile
    130                 135                 140

Gly Ala Pro Gln Pro Ala Pro Ala Ala Ala Pro Ala Arg Arg Thr Arg
145                 150                 155                 160

Lys Pro Leu Gln Pro Glu Ser Leu Glu Glu Cys Asp Ser Glu Leu Asp
                165                 170                 175

Ile Lys Arg Tyr Lys Asn Arg Val Ala Ser Arg Lys Cys Arg Ala Lys
            180                 185                 190

Phe Lys His Leu Leu Gln His Tyr Arg Glu Val Ala Ser Ala Lys Ser
        195                 200                 205

Ser Glu Asn Asp Arg Leu Arg Leu Leu Leu Lys Gln Met Cys Pro Ser
    210                 215                 220

Leu Asp Val Asp Ser Ile Ile Pro Arg Thr Pro Asp Val Leu His Glu
225                 230                 235                 240

Asp Leu Leu Asn Phe
                245

<210>  2
<211>  245
<212>  PRT
<213>  Artificial

<220>
<223>  EBV BZLF1 Strain B95-8

<400>  2

Met Met Asp Pro Asn Ser Thr Ser Glu Asp Val Lys Phe Thr Pro Asp
1               5                   10                  15

Pro Tyr Gln Val Pro Phe Val Gln Ala Phe Asp Gln Ala Thr Arg Val
            20                  25                  30

Tyr Gln Asp Leu Gly Gly Pro Ser Gln Ala Pro Leu Pro Cys Val Leu
        35                  40                  45

Trp Pro Val Leu Pro Glu Pro Leu Pro Gln Gly Gln Leu Thr Ala Tyr
    50                  55                  60

His Val Ser Thr Ala Pro Thr Gly Ser Trp Phe Ser Ala Pro Gln Pro
65                  70                  75                  80

Ala Pro Glu Asn Ala Tyr Gln Ala Tyr Ala Ala Pro Gln Leu Phe Pro
                85                  90                  95

Val Ser Asp Ile Thr Gln Asn Gln Gln Thr Asn Gln Ala Gly Gly Glu
            100                 105                 110

Ala Pro Gln Pro Gly Asp Asn Ser Thr Val Gln Thr Ala Ala Ala Val
        115                 120                 125

Val Phe Ala Cys Pro Gly Ala Asn Gln Gly Gln Gln Leu Ala Asp Ile
    130                 135                 140

Gly Val Pro Gln Pro Ala Pro Val Ala Ala Pro Ala Arg Arg Thr Arg
145                 150                 155                 160

Lys Pro Gln Gln Pro Glu Ser Leu Glu Glu Cys Asp Ser Glu Leu Glu
                165                 170                 175

Ile Lys Arg Tyr Lys Asn Arg Val Ala Ser Arg Lys Cys Arg Ala Lys
            180                 185                 190

Phe Lys Gln Leu Leu Gln His Tyr Arg Glu Val Ala Ala Ala Lys Ser
        195                 200                 205

Ser Glu Asn Asp Arg Leu Arg Leu Leu Leu Lys Gln Met Cys Pro Ser
    210                 215                 220

Leu Asp Val Asp Ser Ile Ile Pro Arg Thr Pro Asp Val Leu His Glu
225                 230                 235                 240

Asp Leu Leu Asn Phe
                245

<210>  3
<211>  245
<212>  PRT
<213>  Artificial

<220>
<223>  EBV BZLF1 Strain GD1

<400>  3

Met Met Asp Pro Asn Ser Thr Ser Glu Asp Val Lys Phe Thr Pro Asp
1               5                   10                  15

Pro Tyr Gln Val Pro Phe Val Gln Ala Phe Asp Gln Ala Thr Arg Val
            20                  25                  30

Tyr Gln Asp Leu Gly Gly Pro Ser Gln Ala Pro Leu Pro Cys Val Leu
        35                  40                  45

Trp Pro Val Leu Pro Glu Pro Leu Pro Gln Gly Gln Leu Thr Ala Tyr
    50                  55                  60

His Val Ser Ala Ala Pro Thr Gly Ser Trp Phe Pro Ala Pro Gln Pro

65                  70                  75                  80

Ala Pro Glu Asn Ala Tyr Gln Ala Tyr Ala Ala Pro Gln Leu Phe Pro
                85                  90                  95

Val Ser Asp Ile Thr Gln Asn Gln Leu Thr Asn Gln Ala Gly Gly Glu
            100                 105                 110

Ala Pro Gln Pro Gly Asp Asn Ser Thr Val Gln Pro Ala Ala Ala Val
        115                 120                 125

Val Leu Ala Cys Pro Gly Ala Asn Gln Glu Gln Gln Leu Ala Asp Ile
    130                 135                 140

Gly Ala Pro Gln Pro Ala Pro Ala Ala Ala Pro Ala Arg Arg Thr Arg
145                 150                 155                 160

Lys Pro Leu Gln Pro Glu Ser Leu Glu Glu Cys Asp Ser Glu Leu Glu
                165                 170                 175

Ile Lys Arg Tyr Lys Asn Arg Val Ala Ser Arg Lys Cys Arg Ala Lys
            180                 185                 190

Phe Lys His Leu Leu Gln His Tyr Arg Glu Val Ala Ser Ala Lys Ser
        195                 200                 205

Ser Glu Asn Asp Arg Leu Arg Leu Leu Leu Lys Gln Met Cys Pro Ser
    210                 215                 220

Leu Asp Val Asp Ser Ile Ile Pro Arg Thr Pro Asp Val Leu His Glu
225                 230                 235                 240

Asp Leu Leu Asn Phe
                245

<210>  4
<211>  641
<212>  PRT
<213>  Artificial

<220>
<223>  EBV EBNA1 Strain AG876

<400>  4

Met Ser Asp Glu Gly Pro Gly Thr Gly Pro Gly Asn Gly Leu Gly Gln
1               5                   10                  15

Lys Glu Asp Thr Ser Gly Pro Asp Gly Ser Ser Gly Ser Gly Pro Gln
            20                  25                  30

Arg Arg Gly Gly Asp Asn His Gly Arg Gly Arg Gly Arg Gly Arg Gly
        35                  40                  45

Arg Gly Gly Gly Arg Pro Gly Ala Pro Gly Gly Ser Gly Ser Gly Pro
    50                  55                  60

Arg His Arg Asp Gly Val Arg Arg Pro Gln Lys Arg Pro Ser Cys Ile
65                  70                  75                  80

Gly Cys Lys Gly Ala His Gly Gly Thr Gly Ala Gly Gly Gly Ala Gly
                85                  90                  95

Ala Gly Gly Ala Gly Ala Gly Gly Ala Gly Ala Gly Gly Ala Gly Ala
            100                 105                 110

Gly Gly Ala Gly Ala Gly Gly Ala Gly Ala Gly Gly Ala Gly Ala Gly
        115                 120                 125

Gly Ala Gly Ala Gly Gly Ala Gly Ala Gly Gly Ala Gly Ala Gly Gly
    130                 135                 140

Gly Ala Gly Ala Gly Gly Ala Gly Ala Gly Gly Ala Gly Ala Gly Gly
145                 150                 155                 160

Gly Ala Gly Ala Gly Gly Gly Ala Gly Ala Gly Gly Gly Ala Gly Ala
                165                 170                 175

Gly Gly Gly Ala Gly Ala Gly Gly Gly Ala Gly Ala Gly Gly Gly Ala
            180                 185                 190

Gly Ala Gly Gly Gly Ala Gly Ala Gly Gly Gly Ala Gly Ala Gly Gly
        195                 200                 205

Gly Ala Gly Ala Gly Gly Ala Gly Ala Gly Gly Ala Gly Ala Gly Gly
    210                 215                 220

Gly Ala Gly Ala Gly Gly Gly Ala Gly Ala Gly Gly Gly Ala Gly Ala
225                 230                 235                 240

Gly Gly Gly Ala Gly Ala Gly Gly Gly Ala Gly Ala Gly Gly Gly Ala
                245                 250                 255

Gly Ala Gly Gly Gly Ala Gly Ala Gly Gly Gly Ala Gly Ala Gly Gly
            260                 265                 270

Gly Ala Gly Ala Gly Gly Gly Ala Gly Ala Gly Gly Gly Ala Gly Ala

275                 280                 285

Gly Gly Gly Ala Gly Ala Gly Gly Gly Ala Gly Ala Gly Gly Gly Ala
    290                 295                 300

Gly Ala Gly Gly Gly Ala Gly Ala Gly Gly Gly Ala Gly Ala Gly Gly
305                 310                 315                 320

Gly Ala Gly Ala Gly Gly Gly Gly Arg Gly Arg Gly Gly Ser Gly Gly
                325                 330                 335

Arg Gly Arg Gly Gly Ser Gly Gly Arg Gly Arg Gly Gly Ser Gly Gly
            340                 345                 350

Arg Arg Gly Arg Gly Arg Glu Arg Ala Arg Gly Gly Ser Arg Glu Arg
        355                 360                 365

Ala Arg Gly Arg Gly Arg Gly Arg Gly Glu Lys Arg Pro Arg Ser Pro
    370                 375                 380

Ser Ser Gln Ser Ser Ser Ser Gly Ser Pro Pro Arg Arg Pro Pro Pro
385                 390                 395                 400

Gly Arg Arg Pro Phe Phe His Pro Val Ala Glu Ala Asp Tyr Phe Glu
                405                 410                 415

Tyr His Gln Glu Gly Gly Pro Asp Gly Glu Pro Asp Met Pro Pro Gly
            420                 425                 430

Ala Ile Glu Gln Gly Pro Ala Asp Asp Pro Gly Glu Gly Pro Ser Thr
        435                 440                 445

Gly Pro Arg Gly Gln Gly Asp Gly Gly Arg Arg Lys Lys Gly Gly Trp
    450                 455                 460

Tyr Gly Lys His Arg Gly Glu Gly Gly Ser Ser Gln Lys Phe Glu Asn
465                 470                 475                 480

Ile Ala Glu Gly Leu Arg Leu Leu Leu Ala Arg Cys His Val Glu Arg
                485                 490                 495

Thr Thr Glu Asp Gly Asn Trp Val Ala Gly Val Phe Val Tyr Gly Gly
            500                 505                 510

Ser Lys Thr Ser Leu Tyr Asn Leu Arg Arg Gly Ile Gly Leu Ala Ile
        515                 520                 525

Pro Gln Cys Arg Leu Thr Pro Leu Ser Arg Leu Pro Phe Gly Met Ala
    530                 535                 540

Pro Gly Pro Gly Pro Gln Pro Gly Pro Leu Arg Glu Ser Ile Val Cys
545                 550                 555                 560

Tyr Phe Ile Val Phe Leu Gln Thr His Ile Phe Ala Glu Gly Leu Lys
                565                 570                 575

Asp Ala Ile Lys Asp Leu Val Leu Pro Lys Pro Ala Pro Thr Cys Asn
            580                 585                 590

Ile Lys Val Thr Val Cys Ser Phe Asp Asp Gly Val Asp Leu Pro Pro
        595                 600                 605

Trp Phe Pro Pro Met Val Glu Gly Ala Ala Ala Glu Gly Asp Asp Gly
    610                 615                 620

Asp Asp Gly Asp Glu Gly Gly Asp Gly Asp Glu Gly Glu Glu Gly Gln
625                 630                 635                 640

Glu

<210>  5
<211>  641
<212>  PRT
<213>  Artificial

<220>
<223>  EBV EBNA1 Strain B95-8

<400>  5

Met Ser Asp Glu Gly Pro Gly Thr Gly Pro Gly Asn Gly Leu Gly Glu
1               5                   10                  15

Lys Gly Asp Thr Ser Gly Pro Glu Gly Ser Gly Gly Ser Gly Pro Gln
            20                  25                  30

Arg Arg Gly Gly Asp Asn His Gly Arg Gly Arg Gly Arg Gly Arg Gly
        35                  40                  45

Arg Gly Gly Gly Arg Pro Gly Ala Pro Gly Gly Ser Gly Ser Gly Pro
    50                  55                  60

Arg His Arg Asp Gly Val Arg Arg Pro Gln Lys Arg Pro Ser Cys Ile
65                  70                  75                  80

Gly Cys Lys Gly Thr His Gly Gly Thr Gly Ala Gly Ala Gly Ala Gly

                85                  90                  95

Gly Ala Gly Ala Gly Gly Ala Gly Ala Gly Gly Gly Ala Gly Ala Gly
            100                 105                 110

Gly Gly Ala Gly Gly Ala Gly Gly Ala Gly Gly Ala Gly Ala Gly Gly
        115                 120                 125

Gly Ala Gly Ala Gly Gly Gly Ala Gly Gly Ala Gly Gly Ala Gly Ala
    130                 135                 140

Gly Gly Gly Ala Gly Ala Gly Gly Gly Ala Gly Gly Ala Gly Ala Gly
145                 150                 155                 160

Gly Gly Ala Gly Gly Ala Gly Gly Ala Gly Ala Gly Gly Gly Ala Gly
                165                 170                 175

Ala Gly Gly Gly Ala Gly Gly Ala Gly Ala Gly Gly Gly Ala Gly Gly
            180                 185                 190

Ala Gly Gly Ala Gly Ala Gly Gly Gly Ala Gly Ala Gly Gly Ala Gly
        195                 200                 205

Gly Ala Gly Gly Ala Gly Ala Gly Gly Ala Gly Ala Gly Gly Gly Ala
    210                 215                 220

Gly Gly Ala Gly Gly Ala Gly Ala Gly Gly Ala Gly Ala Gly Gly Ala
225                 230                 235                 240

Gly Ala Gly Gly Ala Gly Ala Gly Gly Ala Gly Gly Ala Gly Ala Gly
                245                 250                 255

Gly Ala Gly Gly Ala Gly Ala Gly Gly Ala Gly Gly Ala Gly Ala Gly
            260                 265                 270

Gly Gly Ala Gly Gly Ala Gly Ala Gly Gly Gly Ala Gly Gly Ala Gly
        275                 280                 285

Ala Gly Gly Ala Gly Gly Ala Gly Ala Gly Gly Ala Gly Gly Ala Gly
    290                 295                 300

Ala Gly Gly Ala Gly Gly Ala Gly Ala Gly Gly Gly Ala Gly Ala Gly
305                 310                 315                 320

Gly Ala Gly Ala Gly Gly Gly Gly Arg Gly Arg Gly Gly Ser Gly Gly
                325                 330                 335

Arg Gly Arg Gly Gly Ser Gly Gly Arg Gly Arg Gly Gly Ser Gly Gly
        340                 345                 350

Arg Arg Gly Arg Gly Arg Glu Arg Ala Arg Gly Gly Ser Arg Glu Arg
    355                 360                 365

Ala Arg Gly Arg Gly Arg Gly Arg Gly Glu Lys Arg Pro Arg Ser Pro
    370                 375                 380

Ser Ser Gln Ser Ser Ser Ser Gly Ser Pro Pro Arg Arg Pro Pro Pro
385                 390                 395                 400

Gly Arg Arg Pro Phe Phe His Pro Val Gly Glu Ala Asp Tyr Phe Glu
                405                 410                 415

Tyr His Gln Glu Gly Gly Pro Asp Gly Glu Pro Asp Val Pro Pro Gly
            420                 425                 430

Ala Ile Glu Gln Gly Pro Ala Asp Asp Pro Gly Glu Gly Pro Ser Thr
        435                 440                 445

Gly Pro Arg Gly Gln Gly Asp Gly Gly Arg Arg Lys Lys Gly Gly Trp
    450                 455                 460

Phe Gly Lys His Arg Gly Gln Gly Gly Ser Asn Pro Lys Phe Glu Asn
465                 470                 475                 480

Ile Ala Glu Gly Leu Arg Ala Leu Leu Ala Arg Ser His Val Glu Arg
                485                 490                 495

Thr Thr Asp Glu Gly Thr Trp Val Ala Gly Val Phe Val Tyr Gly Gly
            500                 505                 510

Ser Lys Thr Ser Leu Tyr Asn Leu Arg Arg Gly Thr Ala Leu Ala Ile
        515                 520                 525

Pro Gln Cys Arg Leu Thr Pro Leu Ser Arg Leu Pro Phe Gly Met Ala
    530                 535                 540

Pro Gly Pro Gly Pro Gln Pro Gly Pro Leu Arg Glu Ser Ile Val Cys
545                 550                 555                 560

Tyr Phe Met Val Phe Leu Gln Thr His Ile Phe Ala Glu Val Leu Lys
                565                 570                 575

Asp Ala Ile Lys Asp Leu Val Met Thr Lys Pro Ala Pro Thr Cys Asn
            580                 585                 590

Ile Arg Val Thr Val Cys Ser Phe Asp Asp Gly Val Asp Leu Pro Pro
        595                 600                 605

Trp Phe Pro Pro Met Val Glu Gly Ala Ala Ala Glu Gly Asp Asp Gly
    610                 615                 620

Asp Asp Gly Asp Glu Gly Gly Asp Gly Asp Glu Gly Glu Glu Gly Gln
625                 630                 635                 640

Glu

<210>  6
<211>  641
<212>  PRT
<213>  Artificial

<220>
<223>  EBV EBNA1 Strain GD1

<400>  6

Met Ser Asp Glu Gly Pro Gly Thr Gly Pro Gly Asn Gly Leu Gly Gln
1               5                   10                  15

Lys Glu Asp Ser Ser Gly Pro Glu Gly Ser Gly Gly Ser Gly Pro Gln
            20                  25                  30

Arg Arg Gly Gly Asp Asn His Gly Arg Gly Arg Gly Arg Gly Arg Gly
        35                  40                  45

Arg Gly Gly Gly Arg Pro Gly Ala Pro Gly Gly Ser Gly Ser Gly Pro
    50                  55                  60

Arg His Arg Asp Gly Val Arg Arg Pro Gln Lys Arg Pro Ser Cys Ile
65                  70                  75                  80

Gly Cys Lys Gly Ala His Gly Gly Thr Gly Ser Gly Ala Gly Ala Gly
                85                  90                  95

Gly Ala Gly Ala Gly Gly Ala Gly Ala Gly Gly Gly Ala Gly Ala Gly
            100                 105                 110

Gly Gly Ala Gly Gly Ala Gly Gly Ala Gly Gly Ala Gly Ala Gly Gly
        115                 120                 125

Gly Ala Gly Ala Gly Gly Gly Ala Gly Gly Ala Gly Gly Ala Gly Ala
    130                 135                 140

Gly Gly Gly Ala Gly Ala Gly Gly Gly Ala Gly Gly Ala Gly Ala Gly
145                 150                 155                 160

Gly Gly Ala Gly Gly Ala Gly Gly Ala Gly Ala Gly Gly Gly Ala Gly
                165                 170                 175

Ala Gly Gly Gly Ala Gly Gly Ala Gly Ala Gly Gly Gly Ala Gly Gly
            180                 185                 190

Ala Gly Gly Ala Gly Ala Gly Gly Gly Ala Gly Ala Gly Gly Ala Gly
        195                 200                 205

Gly Ala Gly Gly Ala Gly Ala Gly Gly Ala Gly Ala Gly Gly Gly Ala
    210                 215                 220

Gly Gly Ala Gly Gly Ala Gly Ala Gly Gly Ala Gly Ala Gly Gly Ala
225                 230                 235                 240

Gly Ala Gly Gly Ala Gly Ala Gly Gly Ala Gly Gly Ala Gly Ala Gly
                245                 250                 255

Gly Ala Gly Gly Ala Gly Ala Gly Gly Ala Gly Gly Ala Gly Ala Gly
            260                 265                 270

Glu Glu Ala Gly Gly Ala Gly Ala Gly Gly Gly Ala Gly Gly Ala Gly
        275                 280                 285

Ala Gly Gly Ala Gly Gly Ala Gly Ala Gly Gly Ala Gly Gly Ala Gly
    290                 295                 300

Ala Gly Gly Ala Gly Gly Ala Gly Ala Gly Gly Gly Ala Gly Ala Gly
305                 310                 315                 320

Gly Ala Gly Ala Gly Gly Gly Gly Arg Gly Arg Gly Gly Ser Gly Gly
                325                 330                 335

Arg Gly Arg Gly Gly Ser Gly Gly Arg Gly Arg Gly Gly Ser Gly Gly
            340                 345                 350

Arg Arg Gly Arg Gly Arg Glu Arg Ala Arg Gly Arg Ser Arg Glu Arg
        355                 360                 365

Ala Arg Gly Arg Gly Arg Gly Arg Gly Glu Lys Arg Pro Arg Ser Pro
    370                 375                 380

Ser Ser Gln Ser Ser Ser Ser Gly Ser Pro Pro Arg Arg Pro Pro Pro
385                 390                 395                 400

Gly Arg Arg Pro Phe Phe His Pro Val Gly Asp Ala Asp Tyr Phe Glu
            405                 410                 415

Tyr Leu Gln Glu Gly Gly Pro Asp Gly Glu Pro Asp Val Pro Pro Gly
        420                 425                 430

Ala Ile Glu Gln Gly Pro Thr Asp Asp Pro Gly Glu Gly Pro Ser Thr
        435                 440                 445

Gly Pro Arg Gly Gln Gly Asp Gly Gly Arg Arg Lys Lys Gly Gly Trp
    450                 455                 460

Phe Gly Lys His Arg Gly Gln Gly Gly Ser Asn Pro Lys Phe Glu Asn
465                 470                 475                 480

Ile Ala Glu Gly Leu Arg Val Leu Leu Ala Arg Ser His Val Glu Arg
            485                 490                 495

Thr Thr Glu Glu Gly Asn Trp Val Ala Gly Val Phe Val Tyr Gly Gly
        500                 505                 510

Ser Lys Thr Ser Leu Tyr Asn Leu Arg Arg Gly Ile Ala Leu Ala Val
        515                 520                 525

Pro Gln Cys Arg Ile Thr Pro Leu Ser Arg Leu Pro Phe Gly Met Ala
    530                 535                 540

Pro Gly Pro Gly Pro Gln Pro Gly Pro Leu Arg Glu Ser Ile Val Cys
545                 550                 555                 560

Tyr Phe Met Val Phe Leu Gln Thr His Ile Phe Ala Glu Val Leu Lys
            565                 570                 575

Asp Ala Ile Lys Asp Leu Val Met Thr Lys Pro Ala Pro Thr Cys Asn
        580                 585                 590

Ile Lys Val Thr Val Cys Ser Phe Asp Asp Gly Val Asp Leu Pro Pro
        595                 600                 605

Trp Phe Pro Pro Met Val Glu Gly Ala Ala Ala Glu Gly Asp Asp Gly
    610                 615                 620

Asp Asp Gly Asp Glu Gly Gly Asp Gly Asp Glu Gly Glu Glu Gly Gln
625                 630                 635                 640

Glu

<210> 7
<211> 925
<212> PRT
<213> Artificial

<220>
<223> EBNA3 Strain AG876

<400> 7

Met Asp Lys Asp Arg Pro Gly Leu Pro Ala Pro Asp Asp Asn Ile Glu
1               5                   10                  15

Glu Val Pro Ser Thr Ser Gly Val Gln Glu Arg Ala Ser Glu Gly Asp
            20                  25                  30

Trp Glu Asn Val Leu Ile Glu Ile Ser Asp Ser Ser Ser Glu Glu Glu
        35                  40                  45

Ala Glu Asp Ala His Leu Glu Pro Ser Gln Arg Gly Lys Lys Arg Lys
    50                  55                  60

Arg Val Asp Asp Asp Ala Gly Gly Ser Ala Pro Ala Gln His Val Pro
65                  70                  75                  80

Pro Pro Gln Leu Asp His Pro Gly Arg Glu Ala Ile Leu Tyr Arg Phe
                85                  90                  95

Pro Leu Asp Leu Arg Arg Phe Ile Gln Ala Ile Gly Ala Ala Ala Thr
            100                 105                 110

His Pro Asp Thr Arg Ala Ile Asp Gln Phe Phe Gly Ser Gln Ile Ser
        115                 120                 125

Asn Thr Asp Leu Tyr Val Met Tyr Ala Met Ala Ile Arg Gln Ala Ile
    130                 135                 140

Arg Asp Arg Arg Arg Asn Pro Ala Ser Arg Arg Ser Gln Val Lys Trp
145                 150                 155                 160

Arg Met Thr Thr Leu Ala Ala Gly Trp Pro Met Gly Tyr Gln Ala Tyr
                165                 170                 175

Ser Ser Trp Met Tyr Ser Tyr Thr Asp Pro Gln Val Thr Ala Thr Ile
            180                 185                 190

Ile His Leu Gln Ala Thr Leu Gly Cys Ala Ser Gly Arg Arg Cys His
        195                 200                 205

Val Thr Phe Ser Ala Gly Thr Phe Arg Pro Pro Arg Cys Ser Pro Gly
    210                 215                 220

Asp Arg Gln Trp Leu Tyr Val Gln Ser Arg Val Gly Asp Leu Val Gln
225                 230                 235                 240

Ser Ser Asn Pro Cys Tyr Ser Ile Phe Phe Asp Tyr Met Ala Ile His
                245                 250                 255

Arg Ser Leu Thr Lys Ile Trp Asp Glu Val Val Thr Pro Asp Gln Arg
            260                 265                 270

Val Thr Phe Met Glu Phe Leu Gly Phe Leu Gln Arg Thr Glu Leu Val
        275                 280                 285

Tyr Ile Lys Ser Phe Val Ser Tyr Ala Leu Gly Thr Thr Ser Ile Glu
    290                 295                 300

Thr Pro Trp Met Asp Glu Asn Pro Ser Thr Glu Thr Ala Gln Ala Trp
305                 310                 315                 320

Asn Ala Gly Leu Leu Arg Gly Arg Ala Tyr Gly Gln Asp Leu Leu Arg
                325                 330                 335

Thr Glu Gly Glu His Gly Glu Gly Ala Thr Cys Glu Thr Arg Glu Glu
            340                 345                 350

Ser Glu Asp Thr Glu Ser Asp Gly Asp Asp Glu Glu Leu Pro Arg Val
        355                 360                 365

Val Ser Arg Asp Gly Thr Lys His Arg Arg Pro Pro Ile Phe Leu Arg
    370                 375                 380

Arg Leu His Arg Leu Leu Leu Met Arg Ala Gly Lys Gly Lys Glu Arg
385                 390                 395                 400

Ala Arg Glu Thr Leu Ala Lys Ala Pro Arg Arg Thr Tyr Gly Thr Pro
                405                 410                 415

Arg Pro Pro Val Gln Lys Pro Arg Pro Glu Val Pro Gln Ser Tyr Glu
            420                 425                 430

Thr Ala Thr Ser His Gly Ser Ala Gln Val Pro Glu Pro Pro Pro Thr
        435                 440                 445

His Pro Leu His Gln Gln His Ser Met Ala Pro Cys Met Val Ala Gln
    450                 455                 460

Asn Pro Arg Ala Pro Leu Gly Asp Gln Leu Pro Gly Val Pro Lys Asp
465                 470                 475                 480

Gly Arg Gly Ala Cys Ala Pro Val Pro Ala Leu Ala Gly Pro Ile Val
                485                 490                 495

Arg Pro Trp Glu Ser Ser Leu Leu Gln Ser Pro Gly Arg Ala Phe Ala
            500                 505                 510

Pro Val Ser Pro Gln Pro Met Pro Val Glu Pro Val Pro Val Pro Thr
        515                 520                 525

Val Ala Leu Glu Arg Pro Val Cys Pro Ala Pro Pro Glu Ile Ala Met
    530                 535                 540

Gln Gly Pro Gly Glu Pro Ser Gly Ile Lys Arg Thr Arg Glu Arg Trp
545                 550                 555                 560

Arg Pro Ala Pro Trp Thr Pro Asn Pro Pro Arg Ser Pro Ser Gln Met
                565                 570                 575

Ser Val Arg Asp Arg Leu Ala Arg Leu Arg Ala Glu Ala Gln Ala Arg
            580                 585                 590

Gln Ala Ser Val Glu Val Gln Pro Thr Gln Leu Thr Gln Val Ser Pro
        595                 600                 605

Gln Gln Pro Met Glu Arg Pro Leu Glu Pro Glu Gln Gln Met Phe Pro
    610                 615                 620

Gly Ser Pro Phe Ser Gln Val Ala Asp Val Ala Arg Glu Ser Gly Val
625                 630                 635                 640

Pro Ala Met Gln Pro Gln Tyr Phe Asp Leu Pro Leu Thr Gln Pro Ile
                645                 650                 655

Ser Gln Gly Ala Pro Ala Ala Pro Leu Arg Ala Ser Met Gly Pro Val
            660                 665                 670

Pro Pro Val Pro Ala Thr Gln Pro Gln Tyr Phe Asp Ile Pro Leu Thr
        675                 680                 685

Glu Pro Ile Asn Gln Gly Ala Ser Ala Ala His Phe Leu Pro Gln Gln
    690                 695                 700

Pro Met Glu Gly Pro Leu Val Pro Glu Arg Trp Met Phe Gln Gly Ala

705                 710                 715                 720

Thr Leu Ser Gln Ser Val Arg Pro Gly Val Ala Gln Ser Gln Tyr Phe
                725                 730                 735

Asp Leu Pro Leu Thr Gln Pro Ile Asn His Gly Ala Pro Ala Ala His
            740                 745                 750

Phe Leu His Gln Pro Pro Met Glu Gly Pro Trp Val Pro Glu Gln Trp
        755                 760                 765

Met Phe Gln Gly Ala Pro Pro Ser Gln Gly Thr Asp Val Val Gln His
    770                 775                 780

Gln Leu Asp Asp Leu Gly Tyr Pro Leu His Asp Leu Asn His Pro Gly
785                 790                 795                 800

Val Pro Val Ser Pro Ala Val Asn Gln Tyr His Phe Ser Gln Ala Ala
                805                 810                 815

Phe Gly Leu Pro Ile Asp Glu Asp Glu Ser Gly Glu Arg Ser Asp Thr
            820                 825                 830

Ser Glu Pro Tyr Glu Ala Leu Asp Leu Ser Ile His Gly Arg Pro Cys
        835                 840                 845

Pro Gln Ala Pro Glu Trp Pro Val Gln Gly Glu Gly Gly Gln Asp Ala
    850                 855                 860

Thr Glu Val Leu Asp Leu Ser Ile His Gly Arg Pro Arg Pro Arg Thr
865                 870                 875                 880

Pro Glu Trp Pro Val Gln Gly Glu Ser Gly Gln Asn Val Thr Asp His
                885                 890                 895

Glu Pro Arg Arg Val Val Val Ser Ala Ile Val His Met Cys Gln Asp
            900                 905                 910

Asp Glu Phe Pro Asp Leu Gln Asp Pro Pro Asp Glu Ala
        915                 920                 925

<210>  8
<211>  944
<212>  PRT
<213>  Artificial

<220>
<223>  EBV EBNA3 Strain B95-8

<400>  8

Met Asp Lys Asp Arg Pro Gly Pro Pro Ala Leu Asp Asp Asn Met Glu
1               5                   10                  15

Glu Glu Val Pro Ser Thr Ser Val Val Gln Glu Gln Val Ser Ala Gly
            20                  25                  30

Asp Trp Glu Asn Val Leu Ile Glu Leu Ser Asp Ser Ser Ser Glu Lys
        35                  40                  45

Glu Ala Glu Asp Ala His Leu Glu Pro Ala Gln Lys Gly Thr Lys Arg
    50                  55                  60

Lys Arg Val Asp His Asp Ala Gly Gly Ser Ala Pro Ala Arg Pro Met
65                  70                  75                  80

Leu Pro Pro Gln Pro Asp Leu Pro Gly Arg Glu Ala Ile Leu Arg Arg
                85                  90                  95

Phe Pro Leu Asp Leu Arg Thr Leu Leu Gln Ala Ile Gly Ala Ala Ala
            100                 105                 110

Thr Arg Ile Asp Thr Arg Ala Ile Asp Gln Phe Phe Gly Ser Gln Ile
        115                 120                 125

Ser Asn Thr Glu Met Tyr Ile Met Tyr Ala Met Ala Ile Arg Gln Ala
    130                 135                 140

Ile Arg Asp Arg Arg Arg Asn Pro Ala Ser Arg Arg Asp Gln Ala Lys
145                 150                 155                 160

Trp Arg Leu Gln Thr Leu Ala Ala Gly Trp Pro Met Gly Tyr Gln Ala
                165                 170                 175

Tyr Ser Ser Trp Met Tyr Ser Tyr Thr Asp His Gln Thr Thr Pro Thr
            180                 185                 190

Phe Val His Leu Gln Ala Thr Leu Gly Cys Thr Gly Gly Arg Arg Cys
        195                 200                 205

His Val Thr Phe Ser Ala Gly Thr Phe Lys Leu Pro Arg Cys Thr Pro
    210                 215                 220

Gly Asp Arg Gln Trp Leu Tyr Val Gln Ser Ser Val Gly Asn Ile Val
225                 230                 235                 240

Gln Ser Cys Asn Pro Arg Tyr Ser Ile Phe Phe Asp Tyr Met Ala Ile

                245                 250                 255

His Arg Ser Leu Thr Lys Ile Trp Glu Glu Val Leu Thr Pro Asp Gln
            260                 265                 270

Arg Val Ser Phe Met Glu Phe Leu Gly Phe Leu Gln Arg Thr Asp Leu
        275                 280                 285

Ser Tyr Ile Lys Ser Phe Val Ser Asp Ala Leu Gly Thr Thr Ser Ile
    290                 295                 300

Gln Thr Pro Trp Ile Asp Asp Asn Pro Ser Thr Glu Thr Ala Gln Ala
305                 310                 315                 320

Trp Asn Ala Gly Phe Leu Arg Gly Arg Ala Tyr Gly Ile Asp Leu Leu
                325                 330                 335

Arg Thr Glu Gly Glu His Val Glu Gly Ala Thr Gly Glu Thr Arg Glu
            340                 345                 350

Glu Ser Glu Asp Thr Glu Ser Asp Gly Asp Asp Glu Asp Leu Pro Cys
        355                 360                 365

Ile Val Ser Arg Gly Gly Pro Lys Val Lys Arg Pro Pro Ile Phe Ile
    370                 375                 380

Arg Arg Leu His Arg Leu Leu Leu Met Arg Ala Gly Lys Arg Thr Glu
385                 390                 395                 400

Gln Gly Lys Glu Val Leu Glu Lys Ala Arg Gly Ser Thr Tyr Gly Thr
                405                 410                 415

Pro Arg Pro Pro Val Pro Lys Pro Arg Pro Glu Val Pro Gln Ser Asp
            420                 425                 430

Glu Thr Ala Thr Ser His Gly Ser Ala Gln Val Pro Glu Pro Pro Thr
        435                 440                 445

Ile His Leu Ala Ala Gln Gly Met Ala Tyr Pro Leu His Glu Gln His
    450                 455                 460

Gly Met Ala Pro Cys Pro Val Ala Gln Ala Pro Pro Thr Pro Leu Pro
465                 470                 475                 480

Pro Val Ser Pro Gly Asp Gln Leu Pro Gly Val Phe Ser Asp Gly Arg
                485                 490                 495

Val Ala Cys Ala Pro Val Pro Ala Pro Ala Gly Pro Ile Val Arg Pro
        500                 505                 510

Trp Glu Pro Ser Leu Thr Gln Ala Ala Gly Gln Ala Phe Ala Pro Val
    515                 520                 525

Arg Pro Gln His Met Pro Val Glu Pro Val Pro Val Pro Thr Val Ala
    530                 535                 540

Leu Glu Arg Pro Val Tyr Pro Lys Pro Val Arg Pro Ala Pro Pro Lys
545                 550                 555                 560

Ile Ala Met Gln Gly Pro Gly Glu Thr Ser Gly Ile Arg Arg Ala Arg
                565                 570                 575

Glu Arg Trp Arg Pro Ala Pro Trp Thr Pro Asn Pro Pro Arg Ser Pro
            580                 585                 590

Ser Gln Met Ser Val Arg Asp Arg Leu Ala Arg Leu Arg Ala Glu Ala
        595                 600                 605

Gln Val Lys Gln Ala Ser Val Glu Val Gln Pro Pro Gln Leu Thr Gln
    610                 615                 620

Val Ser Pro Gln Gln Pro Met Glu Gly Pro Leu Val Pro Glu Gln Gln
625                 630                 635                 640

Met Phe Pro Gly Ala Pro Phe Ser Gln Val Ala Asp Val Val Arg Ala
                645                 650                 655

Pro Gly Val Pro Ala Met Gln Pro Gln Tyr Phe Asp Leu Pro Leu Ile
            660                 665                 670

Gln Pro Ile Ser Gln Gly Ala Pro Val Ala Pro Leu Arg Ala Ser Met
        675                 680                 685

Gly Pro Val Pro Pro Val Pro Ala Thr Gln Pro Gln Tyr Phe Asp Ile
    690                 695                 700

Pro Leu Thr Glu Pro Ile Asn Gln Gly Ala Ser Ala Ala His Phe Leu
705                 710                 715                 720

Pro Gln Gln Pro Met Glu Gly Pro Leu Val Pro Glu Gln Trp Met Phe
                725                 730                 735

Pro Gly Ala Ala Leu Ser Gln Ser Val Arg Pro Gly Val Ala Gln Ser
            740                 745                 750

Gln Tyr Phe Asp Leu Pro Leu Thr Gln Pro Ile Asn His Gly Ala Pro
        755                 760                 765

Ala Ala His Phe Leu His Gln Pro Pro Met Glu Gly Pro Trp Val Pro
    770                 775                 780

Glu Gln Trp Met Phe Gln Gly Ala Pro Pro Ser Gln Gly Thr Asp Val
785                 790                 795                 800

Val Gln His Gln Leu Asp Ala Leu Gly Tyr Thr Leu His Gly Leu Asn
                805                 810                 815

His Pro Gly Val Pro Val Ser Pro Ala Val Asn Gln Tyr His Leu Ser
            820                 825                 830

Gln Ala Ala Phe Gly Leu Pro Ile Asp Glu Asp Glu Ser Gly Glu Gly
        835                 840                 845

Ser Asp Thr Ser Glu Pro Cys Glu Ala Leu Asp Leu Ser Ile His Gly
    850                 855                 860

Arg Pro Cys Pro Gln Ala Pro Glu Trp Pro Val Gln Glu Glu Gly Gly
865                 870                 875                 880

Gln Asp Ala Thr Glu Val Leu Asp Leu Ser Ile His Gly Arg Pro Arg
                885                 890                 895

Pro Arg Thr Pro Glu Trp Pro Val Gln Gly Glu Gly Gly Gln Asn Val
            900                 905                 910

Thr Gly Pro Glu Thr Arg Arg Val Val Val Ser Ala Val Val His Met
        915                 920                 925

Cys Gln Asp Asp Glu Phe Pro Asp Leu Gln Asp Pro Pro Asp Glu Ala
    930                 935                 940

<210>  9
<211>  935
<212>  PRT
<213>  Artificial

<220>
<223>  EBV EBNA3 Strain GD1

<400>  9

Met Asp Lys Asp Arg Pro Gly Asp Pro Ala Leu Asp Asp Asn Met Glu
1               5                   10                  15

Glu Glu Val Pro Ser Thr Ser Val Val Gln Glu Gln Val Ser Ala Gly
            20                  25                  30

Asp Trp Glu Asn Val Leu Ile Glu Leu Ser Asp Ser Ser Ser Glu Lys
        35                  40                  45

Glu Ala Glu Asp Ala Gln Leu Glu Pro Ala Gln Lys Gly Thr Lys Arg
    50                  55                  60

Lys Arg Val Asp His Asp Ala Gly Gly Ser Ala Pro Ala Arg Pro Met
65                  70                  75                  80

Leu Pro Pro Gln Pro Asp Leu Pro Gly Arg Glu Ala Ile Leu Arg Arg
                85                  90                  95

Phe Pro Leu Asp Leu Arg Thr Leu Leu Gln Ala Ile Gly Ala Ala Ala
            100                 105                 110

Thr Arg Ile Asp Thr Arg Ala Ile Asp Gln Phe Phe Gly Ser Gln Ile
        115                 120                 125

Ser Asn Thr Glu Met Tyr Ile Met Tyr Ala Met Ala Ile Arg Gln Ala
    130                 135                 140

Ile Arg Asp Arg Arg Arg Asn Pro Ala Ser Arg Arg Asp Gln Ala Lys
145                 150                 155                 160

Trp Arg Leu Gln Thr Leu Ala Ala Gly Trp Pro Met Gly Tyr Gln Ala
                165                 170                 175

Tyr Ser Ser Trp Met Tyr Ser Tyr Thr Asp His Gln Thr Ala Pro Thr
            180                 185                 190

Phe Val Gln Leu Gln Ala Thr Leu Gly Cys Thr Gly Gly Arg Arg Cys
        195                 200                 205

His Val Thr Phe Ser Ala Gly Thr Phe Lys Pro Pro Arg Cys Thr Pro
    210                 215                 220

Gly Asp Arg Gln Trp Leu Tyr Val Gln Ser Ser Val Gly Asn Ile Val
225                 230                 235                 240

Gln Ser Cys Asn Pro Arg Tyr Ser Ile Phe Phe Asp Tyr Met Ala Ile
                245                 250                 255

His Arg Ser Leu Thr Lys Ile Trp Glu Glu Val Leu Thr Pro Asp Gln
            260                 265                 270

Arg Val Ser Phe Met Glu Phe Leu Gly Phe Leu Gln Arg Thr Asp Leu
        275                 280                 285

Ser Tyr Ile Lys Ser Phe Val Ser Asp Ala Leu Gly Thr Thr Ser Ile
    290                 295                 300

Gln Thr Pro Trp Ile Asp Asp Asn Ser Ser Thr Glu Thr Ala Gln Ala
305                 310                 315                 320

Trp Asn Ala Gly Phe Leu Arg Gly Arg Ala Tyr Gly Leu Asp Leu Leu
                325                 330                 335

Arg Thr Glu Gly Glu His Val Glu Gly Ala Thr Gly Glu Thr Arg Glu
            340                 345                 350

Glu Ser Glu Asp Thr Glu Ser Asp Gly Asp Asp Glu Asp Leu Pro Cys
        355                 360                 365

Ile Val Ser Arg Gly Gly Pro Lys Val Lys Arg Pro Pro Ile Phe Ile
    370                 375                 380

Arg Arg Leu His Arg Leu Leu Leu Met Arg Ala Gly Lys Arg Thr Glu
385                 390                 395                 400

Gln Gly Lys Glu Val Leu Glu Lys Ala Arg Gly Ser Thr Tyr Gly Thr
                405                 410                 415

Pro Arg Pro Pro Val Pro Lys Pro Arg Pro Glu Val Pro Gln Ser Asp
            420                 425                 430

Glu Thr Ala Thr Ser His Gly Ser Ala Gln Val Pro Glu Pro Pro Thr
        435                 440                 445

Ile His Leu Ala Ala Gln Gly Met Ala Tyr Pro Leu His Glu Gln Arg
    450                 455                 460

Gly Met Ala Pro Cys Pro Val Ala Gln Ala Pro His Thr Pro Leu Pro
465                 470                 475                 480

Pro Val Ser Pro Gly Asp Gln Leu Pro Gly Val Ser Ser Asp Gly Arg
                485                 490                 495

Val Ala Cys Ala Pro Val Pro Ala Pro Ala Gly Pro Ile Val Arg Pro
            500                 505                 510

Trp Glu Pro Ser Leu Thr Gln Ala Ala Gly Gln Ala Phe Ala Pro Val
        515                 520                 525

Arg Pro Gln His Met Pro Val Glu Pro Val Pro Val Pro Thr Val Ala
    530                 535                 540

Leu Glu Arg Pro Val Tyr Pro Lys Pro Val Arg Pro Ala Pro Pro Lys
545                 550                 555                 560

Ile Ala Met Gln Gly Pro Gly Glu Thr Ser Gly Ile Arg Arg Ala Arg
                565                 570                 575

Glu Arg Trp Arg Pro Ala Pro Trp Thr Pro Asn Pro Pro Arg Ser Pro
            580                 585                 590

Ser Gln Met Ser Val Arg Asp Arg Leu Ala Arg Leu Arg Ala Glu Ala
        595                 600                 605

Gln Val Lys Gln Ala Ser Val Glu Val Gln Pro Pro Gln Val Thr Gln
    610                 615                 620

Val Ser Pro Gln Gln Pro Met Glu Gly Pro Gly Ala Pro Phe Ser Gln
625                 630                 635                 640

Val Ala Asp Val Val His Thr Pro Gly Val Pro Ala Met Gln Pro Gln
                645                 650                 655

Tyr Phe Asp Leu Pro Leu Ile Gln Pro Ile Ser Gln Gly Ala Pro Val
            660                 665                 670

Ala Pro Leu Arg Ala Ser Met Gly Pro Val Pro Pro Val Pro Ala Thr
        675                 680                 685

Gln Pro Gln Tyr Phe Asp Ile Pro Leu Thr Glu Pro Ile Asn Gln Gly
    690                 695                 700

Ala Ser Ala Ala His Phe Leu Pro Gln Gln Pro Met Glu Gly Pro Leu
705                 710                 715                 720

Val Pro Glu Gln Trp Met Phe Pro Gly Ala Ala Leu Ser Gln Ser Val
                725                 730                 735

Arg Pro Gly Val Ala Gln Ser Gln Tyr Phe Asp Leu Pro Leu Thr Gln
            740                 745                 750

Pro Ile Thr His Gly Ala Pro Ala Ala His Phe Leu His Gln Pro Pro
        755                 760                 765

Met Glu Gly Pro Trp Val Pro Glu Gln Trp Met Phe Gln Gly Ala Pro

770                 775                 780

Pro Ser Gln Gly Thr Asp Val Val Gln His Gln Leu Asp Ala Leu Gly
785                 790                 795                 800

Tyr Pro Leu His Ala Leu Asn His Pro Gly Val Pro Val Ser Pro Ala
                805                 810                 815

Val Asn Gln Tyr His Leu Ser Gln Ala Ala Phe Gly Leu Pro Ile Asp
            820                 825                 830

Glu Asp Glu Ser Gly Glu Gly Ser Asp Thr Ser Glu Pro Cys Glu Ala
        835                 840                 845

Leu Asp Leu Ser Ile His Gly Arg Pro Cys Pro Gln Ala Pro Glu Trp
    850                 855                 860

Pro Val Gln Gly Glu Gly Gly Gln Asp Ala Thr Glu Val Leu Asp Leu
865                 870                 875                 880

Ser Ile His Gly Arg Pro Arg Pro Arg Thr Pro Glu Trp Pro Val Gln
                885                 890                 895

Gly Glu Ser Gly Gln Asn Val Thr Asp His Glu Pro Arg Arg Val Val
            900                 905                 910

Val Ser Ala Ile Val His Met Cys Gln Asp Asp Glu Phe Pro Asp Leu
        915                 920                 925

Gln Asp Pro Pro Asp Glu Ala
    930                 935

<210>  10
<211>  946
<212>  PRT
<213>  Artificial

<220>
<223>  EBV EBNA4 Strain AG876

<400>  10

<210>  11
<211>  938
<212>  PRT
<213>  Artificial

<220>
<223>  EBV EBNA4 Strain B95-8

<400>  11

Met Lys Lys Ala Trp Leu Ser Arg Ala Gln Gln Ala Asp Ala Gly Gly
1               5                   10                  15

Ala Ser Gly Ser Glu Asp Pro Pro Asp Tyr Gly Asp Gln Gly Asn Val
            20                  25                  30

Thr Gln Val Gly Ser Glu Pro Ile Ser Pro Glu Ile Gly Pro Phe Glu
        35                  40                  45

Leu Ser Ala Ala Ser Glu Asp Asp Pro Gln Ser Gly Pro Val Glu Glu
    50                  55                  60

Asn Leu Asp Ala Ala Ala Arg Glu Glu Glu Glu Pro His Glu Gln Glu
65                  70                  75                  80

His Asn Gly Gly Asp Asp Pro Leu Asp Val His Thr Arg Gln Pro Arg
                85                  90                  95

Phe Val Asp Val Asn Pro Thr Gln Ala Pro Val Ile Gln Leu Val His
            100                 105                 110

Ala Val Tyr Asp Ser Met Leu Gln Ser Asp Leu Arg Pro Leu Gly Ser
        115                 120                 125

Leu Phe Leu Glu Gln Asn Leu Asn Ile Glu Glu Phe Ile Trp Met Cys
    130                 135                 140

Met Thr Val Arg His Arg Cys Gln Ala Ile Arg Lys Lys Pro Leu Pro
145                 150                 155                 160

Ile Val Lys Gln Arg Arg Trp Lys Leu Leu Ser Ser Cys Arg Ser Trp
                165                 170                 175

Arg Met Gly Tyr Arg Thr His Asn Leu Lys Val Asn Ser Phe Glu Ser
            180                 185                 190

Gly Gly Asp Asn Val His Pro Val Leu Val Thr Ala Thr Leu Gly Cys
        195                 200                 205

Asp Glu Gly Thr Arg His Ala Thr Thr Tyr Ser Ala Gly Ile Val Gln
    210                 215                 220

Ile Pro Arg Ile Ser Asp Gln Asn Gln Lys Ile Glu Thr Ala Phe Leu
225                 230                 235                 240

Met Ala Arg Arg Ala Arg Ser Leu Ser Ala Glu Arg Tyr Thr Leu Phe
                245                 250                 255

Phe Asp Leu Val Ser Ser Gly Asn Thr Leu Tyr Ala Ile Trp Ile Gly
            260                 265                 270

Leu Gly Thr Lys Asn Arg Val Ser Phe Ile Glu Phe Val Gly Trp Leu
        275                 280                 285

Cys Lys Lys Asp His Thr His Ile Arg Glu Trp Phe Arg Gln Cys Thr
    290                 295                 300

Gly Arg Pro Lys Ala Ala Lys Pro Trp Leu Arg Ala His Pro Val Ala
305                 310                 315                 320

Ile Pro Tyr Asp Asp Pro Leu Thr Asn Glu Glu Ile Asp Leu Ala Tyr
                325                 330                 335

Ala Arg Gly Gln Ala Met Asn Ile Glu Ala Pro Arg Leu Pro Asp Asp
            340                 345                 350

Pro Ile Ile Val Glu Asp Asp Asp Glu Ser Glu Glu Ile Glu Ala Glu
        355                 360                 365

Ser Asp Glu Glu Glu Asp Lys Ser Gly Met Glu Ser Leu Lys Asn Ile
    370                 375                 380

Pro Gln Thr Leu Pro Tyr Asn Pro Thr Val Tyr Gly Arg Pro Ala Val
385                 390                 395                 400

Phe Asp Arg Lys Ser Asp Ala Lys Ser Thr Lys Lys Cys Arg Ala Ile
                405                 410                 415

Val Thr Asp Phe Ser Val Ile Lys Ala Ile Glu Glu Glu His Arg Lys
            420                 425                 430

Lys Lys Ala Ala Arg Thr Glu Gln Pro Arg Ala Thr Pro Glu Ser Gln
        435                 440                 445

Ala Pro Thr Val Val Leu Gln Arg Pro Pro Thr Gln Gln Glu Pro Gly
    450                 455                 460

Pro Val Gly Pro Leu Ser Val Gln Ala Arg Leu Glu Pro Trp Gln Pro
465                 470                 475                 480

Leu Pro Gly Pro Gln Val Thr Ala Val Leu Leu His Glu Glu Ser Met
                485                 490                 495

Gln Gly Val Gln Val His Gly Ser Met Leu Asp Leu Leu Glu Lys Asp
            500                 505                 510

Asp Glu Val Met Glu Gln Arg Val Met Ala Thr Leu Leu Pro Pro Val
        515                 520                 525

Pro Gln Gln Pro Arg Ala Gly Arg Arg Gly Pro Cys Val Phe Thr Gly
    530                 535                 540

Asp Leu Gly Ile Glu Ser Asp Glu Pro Ala Ser Thr Glu Pro Val His
545                 550                 555                 560

Asp Gln Leu Leu Pro Ala Pro Gly Pro Asp Pro Leu Glu Ile Gln Pro
                565                 570                 575

Leu Thr Ser Pro Thr Thr Ser Gln Leu Ser Ser Ser Ala Pro Ser Cys
            580                 585                 590

Ala Gln Thr Pro Trp Pro Val Val Gln Pro Ser Gln Thr Pro Asp Asp
        595                 600                 605

Pro Thr Lys Gln Ser Arg Pro Pro Glu Thr Ala Ala Pro Arg Gln Trp
    610                 615                 620

Pro Met Pro Leu Arg Pro Ile Pro Met Arg Pro Leu Arg Met Gln Pro
625                 630                 635                 640

Ile Pro Phe Asn His Pro Val Gly Pro Thr Pro His Gln Thr Pro Gln
                645                 650                 655

Val Glu Ile Thr Pro Tyr Lys Pro Thr Trp Ala Gln Ile Gly His Ile
            660                 665                 670

Pro Tyr Gln Pro Thr Pro Thr Gly Pro Ala Thr Met Leu Leu Arg Gln
        675                 680                 685

Trp Ala Pro Ala Thr Met Gln Thr Pro Pro Arg Ala Pro Thr Pro Met
    690                 695                 700

Ser Pro Pro Glu Val Pro Pro Val Pro Arg Gln Arg Pro Arg Gly Ala
705                 710                 715                 720

Pro Thr Pro Thr Pro Pro Pro Gln Val Pro Pro Val Pro Arg Gln Arg
                725                 730                 735

Pro Arg Gly Ala Pro Thr Pro Thr Pro Pro Pro Gln Val Leu Pro Thr
            740                 745                 750

Pro Met Gln Leu Ala Leu Arg Ala Pro Ala Gly Gln Gln Gly Pro Thr
        755                 760                 765

Lys Gln Ile Leu Arg Gln Leu Leu Thr Gly Gly Val Lys Lys Gly Arg
    770                 775                 780

Pro Ser Leu Lys Leu Gln Ala Ala Leu Glu Arg Gln Ala Ala Ala Gly
785                 790                 795                 800

Trp Gln Pro Ser Pro Gly Ser Gly Thr Ser Asp Lys Ile Val Gln Ala

805                 810                 815

Pro Ile Phe Tyr Pro Pro Val Leu Gln Pro Ile Gln Val Met Gly Gln
            820                 825                 830

Gly Gly Ser Pro Thr Ala Met Ala Ala Ser Ala Val Thr Gln Ala Pro
        835                 840                 845

Thr Glu Tyr Thr Arg Glu Arg Arg Gly Val Gly Pro Met Pro Pro Thr
    850                 855                 860

Asp Ile Pro Pro Ser Lys Arg Ala Lys Ile Glu Ala Tyr Thr Glu Pro
865                 870                 875                 880

Glu Met Pro His Gly Gly Ala Ser His Ser Pro Val Val Ile Leu Glu
                885                 890                 895

Asn Val Gly Gln Gly Gln Gln Gln Thr Leu Glu Cys Gly Gly Thr Ala
            900                 905                 910

Lys Gln Glu Arg Asp Met Leu Gly Leu Gly Asp Ile Ala Val Ser Ser
        915                 920                 925

Pro Ser Ser Ser Glu Thr Ser Asn Asp Glu
    930                 935

<210>  12
<211>  938
<212>  PRT
<213>  Artificial

<220>
<223>  EBV EBNA4 Strain GD1

<400>  12

Met Lys Lys Ala Trp Leu Ser Arg Ala Arg Gln Ala Asp Ala Gly Gly
1               5                   10                  15

Ala Ser Gly Ser Glu Asp Pro Pro Asp Tyr Gly Asp Gln Gly Asn Val
            20                  25                  30

Gln Gln Val Gly Ser Gly Pro Ile Ser Pro Glu Ile Gly Pro Phe Glu
        35                  40                  45

Leu Ser Ala Ala Ser Glu Asp Asp Pro Gln Ser Gly Pro Val Glu Glu
    50                  55                  60

Asn Leu Asp Ala Ala Ala Arg Glu Glu Gln Glu Pro His Glu Gln Glu
65                  70                  75                  80

His Asn Gly Gly Asp Asp Pro Leu Asp Val His Thr Arg Gln Pro Arg
                85                  90                  95

Phe Val Asp Val Asn Pro Thr Gln Ala Pro Val Ile Gln Leu Val His
            100                 105                 110

Ala Val Tyr Asp Ser Met Leu Gln Ser Asp Leu Arg Pro Leu Gly Ser
        115                 120                 125

Leu Phe Leu Glu Gln Asn Leu Asn Ile Glu Glu Phe Ile Trp Met Cys
    130                 135                 140

Met Thr Val Arg His Arg Cys Gln Ala Ile Arg Lys Lys Pro Leu Pro
145                 150                 155                 160

Ile Val Lys Gln Arg Arg Trp Lys Leu Leu Ser Ser Cys Arg Ser Trp
                165                 170                 175

Arg Met Gly Tyr Arg Thr His Asn Leu Lys Val Asn Ser Phe Glu Ser
            180                 185                 190

Gly Gly Asp Asn Val His Pro Val Leu Val Thr Ala Thr Leu Gly Cys
        195                 200                 205

Asp Glu Gly Thr Arg His Ala Thr Thr Tyr Ser Ala Gly Ile Val Gln
    210                 215                 220

Ile Pro Arg Ile Ser Asp Gln Asn Gln Lys Ile Glu Thr Ala Phe Leu
225                 230                 235                 240

Met Ala Arg Arg Ala Arg Ser Leu Ser Ala Glu Arg Tyr Thr Leu Phe
                245                 250                 255

Phe Asp Leu Val Ser Ser Gly Asn Thr Leu Tyr Ala Ile Trp Ile Gly
            260                 265                 270

Leu Gly Thr Lys Asn Arg Val Ser Phe Ile Glu Phe Val Gly Trp Leu
        275                 280                 285

Cys Lys Lys Asp His Thr His Ile Arg Glu Trp Phe Arg Gln Cys Thr
    290                 295                 300

Gly Arg Pro Lys Ala Ala Lys Pro Trp Leu Arg Ala His Pro Val Ala
305                 310                 315                 320

Ile Pro Tyr Asp Asp Pro Leu Thr Asn Glu Glu Ile Asp Leu Ala Tyr

325                 330                 335

Ala Arg Gly Gln Ala Met Asn Ile Glu Ala Pro Arg Leu Pro Asp Asp
            340                 345                 350

Pro Ile Ile Val Glu Asp Asp Asp Glu Ser Glu Glu Ile Glu Ala Glu
        355                 360                 365

Ser Asp Glu Glu Glu Asp Lys Ser Gly Met Glu Ser Leu Lys Asn Ile
    370                 375                 380

Pro Gln Thr Leu Pro Tyr Asn Pro Thr Val Tyr Gly Arg Pro Ala Val
385                 390                 395                 400

Phe Asp Arg Lys Ser Asp Ala Lys Ser Thr Lys Lys Cys Arg Ala Ile
                405                 410                 415

Leu Thr Asp Phe Ser Val Ile Lys Ala Ile Glu Glu Glu His Arg Lys
            420                 425                 430

Lys Lys Ala Ala Arg Thr Glu Gln Pro Arg Ala Thr Pro Glu Ser Gln
        435                 440                 445

Ala Pro Thr Val Val Leu Gln Arg Pro Pro Thr Gln His Glu Pro Gly
    450                 455                 460

Pro Ala Gly Pro Leu Ser Val Gln Ala Arg Leu Glu Pro Trp Gln Pro
465                 470                 475                 480

Leu Pro Gly Pro Gln Val Thr Thr Val Leu Leu His Glu Glu Ser Met
                485                 490                 495

Gln Gly Val Gln Val His Gly Ser Met Leu Asp Leu Leu Glu Lys Asp
            500                 505                 510

Asp Glu Val Met Glu Gln Arg Val Met Ala Thr Leu Leu Pro Pro Val
        515                 520                 525

Pro Gln Gln Pro Gln Ala Gly Arg Arg Gly Pro Cys Val Tyr Thr Gly
    530                 535                 540

Asp Leu Gly Ile Glu Ser Asp Glu Leu Ala Ser Thr Glu Pro Val His
545                 550                 555                 560

Asp Gln Leu Leu Pro Ala Pro Gly Pro Asp Pro Leu Glu Ile Gln Pro
                565                 570                 575

Leu Thr Ser Pro Thr Thr Ser Gln Leu Ser Ser Ser Ala Pro Ser Cys
            580                 585                 590

Ala Gln Thr Pro Trp Pro Val Val Gln Pro Ser Gln Thr Pro Asp Asp
        595                 600                 605

Pro Met Lys Gln Ser Arg Pro Pro Glu Thr Ala Ala Pro Arg Gln Trp
    610                 615                 620

Pro Met Pro Leu Arg Pro Ile Pro Met Arg Pro Leu Arg Met Gln Pro
625                 630                 635                 640

Ile Pro Phe Asn His Pro Val Gly Pro Thr Pro His Gln Thr Pro Gln
                645                 650                 655

Val Glu Leu Thr Pro Tyr Lys Pro Thr Trp Ala Gln Met Gly His Ile
            660                 665                 670

Pro Tyr Gln Pro Thr Pro Thr Gly Pro Ala Thr Met Leu Leu Arg Gln
        675                 680                 685

Trp Ala Pro Ala Thr Met Gln Thr Pro Pro Arg Ala Pro Thr Pro Met
    690                 695                 700

Ser Pro Pro Glu Val Pro Pro Val Pro Arg Gln Arg Ser Arg Gly Ala
705                 710                 715                 720

Pro Thr Pro Thr Pro Pro Pro Gln Val Pro Pro Val Pro Arg Gln Arg
                725                 730                 735

Pro Arg Gly Ala Pro Thr Pro Thr Pro Pro Pro Gln Val Leu Pro Thr
            740                 745                 750

Pro Met Gln Leu Ala Pro Arg Ala Pro Ala Gly Gln Gln Gly Pro Thr
        755                 760                 765

Lys Gln Ile Leu Arg Gln Leu Leu Thr Gly Gly Val Lys Lys Gly Arg
    770                 775                 780

Pro Ser Leu Lys Leu Gln Ala Ala Leu Glu Arg Gln Ala Ala Ala Gly
785                 790                 795                 800

Trp Gln Pro Ser Thr Gly Ser Gly Thr Ser Ala Lys Ile Val Arg Ala
                805                 810                 815

Pro Val Phe Tyr Pro Pro Val Leu Gln Pro Ile Gln Val Met Gly Gln
            820                 825                 830

Gly Gly Ser Pro Thr Ala Met Ala Ala Ser Thr Val Thr Gln Glu Pro
        835                 840                 845

Thr Glu Tyr Thr Arg Glu Arg Arg Gly Val Gly Pro Met Pro Pro Thr
    850                 855                 860

Asp Ile Pro Pro Ser Lys Arg Ala Lys Ile Glu Ala Tyr Thr Glu Pro
865                 870                 875                 880

Glu Met Pro His Gly Gly Ala Ser His Ser Pro Val Val Ile Leu Glu
                885                 890                 895

Asn Val Gly Gln Gly Gln Gln Gln Thr Leu Glu Cys Gly Gly Thr Ala
            900                 905                 910

Lys Gln Glu Arg Asp Val Leu Gly Leu Gly Asp Ile Ala Val Ser Ser
        915                 920                 925

Pro Ser Ser Ser Glu Thr Ser Asn Asp Glu
    930                 935

<210>  13
<211>  1069
<212>  PRT
<213>  Artificial

<220>
<223>  EBV EBNA6 Strain AG876

<400>  13

Met Glu Ser Phe Glu Gly Glu Gly Asp Ser Ile Gln Ser Pro Asp Asn
1               5                   10                  15

Ala Arg Gly Asp Asp Val Gln Asn Thr Gly Glu His Ile Gln Asp Pro
            20                  25                  30

Gly Pro Gly Pro Ser Thr Gly Gly Ala Ser Glu Gly Leu Val Gln Asn
        35                  40                  45

Glu Pro Asp Ser Arg Asp Gln Gln Ser Arg Gly Gln Arg Arg Gly Asp
    50                  55                  60

Glu Asn Arg Gly Trp Met Gln Arg Ile Arg Arg Arg Arg Arg Arg Arg
65                  70                  75                  80

Ala Ala Leu Ser Gly His Leu Leu Asp Met Glu Asp Asn Val Pro Pro
                85                  90                  95

Trp Phe Pro Pro His Asp Ile Thr Pro Tyr Val Ala Arg Asn Ile Arg
            100                 105                 110

Asp Ala Ala Cys Gln Ala Val Lys His Ser His Leu Gln Ala Leu Ser
        115                 120                 125

Asn Leu Ile Leu Asp Ser Gly Leu Asp Thr Gln His Leu Leu Cys Phe
    130                 135                 140

Val Met Ala Ala Arg Gln Arg Leu Gln Asp Ile Arg Arg Gly Pro Leu
145                 150                 155                 160

Val Val Glu Gly Gly Val Gly Trp Arg His Trp Leu Leu Thr Ser Pro
                165                 170                 175

Ser Arg Ser Trp Ser Met Gly Tyr Arg Thr Ala Thr Leu Arg Thr Leu
            180                 185                 190

Thr Pro Val Pro Asn Arg Val Gly Ala Asp Ser Ile Met Leu Thr Ala
        195                 200                 205

Thr Phe Gly Cys Gln Asn Gly Ala Leu Ala Ile Asn Thr Phe Ser Ala
    210                 215                 220

Thr Val Trp Ile Pro Pro Pro Ala Gly Pro Arg Glu Gln Glu Arg Tyr
225                 230                 235                 240

Ala Arg Glu Ala Glu Val Arg Phe Leu Arg Gly Lys Trp Gln Arg Arg
                245                 250                 255

Phe Arg Arg Ile Phe Asp Leu Ile Glu Leu Cys Gly Ser Leu His His
            260                 265                 270

Val Trp Gln Asn Met Leu Gln Thr Glu Glu Asn Leu Leu Asp Phe Val
        275                 280                 285

Arg Phe Met Gly Val Met Ser Ser Cys Asn Ser Ser Ser Val Asn Tyr
    290                 295                 300

Trp Phe His Lys Thr Ile Gly Asn Phe Lys Pro Tyr Tyr Pro Trp Asn
305                 310                 315                 320

Ala Pro Pro Asn Glu Asn Pro Tyr His Ala Arg Arg Gly Ile Lys Glu
                325                 330                 335

Gln Val Ile Gln Lys Ala Phe Leu Lys Ala Gln Arg Gln Gly Leu Ser
            340                 345                 350

Met Leu Ala Thr Gly Gly Gly Pro Arg Gly Asp Ala Thr Ser Glu Thr
        355                 360                 365

Ser Ser Asp Glu Asp Thr Gly Arg Gln Gly Ser Asp Val Glu Leu Glu
    370                 375                 380

Ser Ser Asp Asp Glu Leu Pro Tyr Ile Asp Pro Asn Met Glu Pro Val
385                 390                 395                 400

Gln Gln Arg Pro Val Met Phe Val Ser Arg Val Pro Val Arg Lys Pro
                405                 410                 415

Arg Thr Leu Pro Trp Pro Thr Pro Lys Thr His Pro Val Lys Arg Thr
            420                 425                 430

Ile Val Lys Thr Ser Tyr Arg Ser Asp Glu Ala Glu Glu Ala Gln Ser
        435                 440                 445

Thr Pro Glu Arg Pro Gly Pro Ser Lys Gln Pro Ser Glu Pro Val Glu
    450                 455                 460

Pro Ala His Thr Thr Pro Ala Gly Arg Ser Thr Val Ile Leu His Glu
465                 470                 475                 480

Pro Pro Arg Glu Pro Glu Ala Val Ser Phe Lys Pro Pro Pro Pro Pro
                485                 490                 495

Ser Arg Arg Arg Arg Gly Ala Cys Val Val Tyr Asp Asp Asp Ile Ile
            500                 505                 510

Glu Val Ile Asp Val Glu Thr Thr Glu Glu Glu Thr Thr Ser Met Gln
        515                 520                 525

Arg Gln Pro Pro Leu Gly Gln Gln Pro Pro Pro Pro Val Ile Ser Thr
    530                 535                 540

Gly Ser Ala Met Ser Ser Ser His Thr Asp Pro Ser Val Thr Gln Pro
545                 550                 555                 560

Ser Lys Pro His Arg Lys Pro Gln Asp Gly Phe Gln Arg Ser Gly Arg
                565                 570                 575

Arg Gln Lys Arg Ala Met Pro Pro Pro Val Ser Pro Ser Asp Ala Gly
            580                 585                 590

Pro Pro Ser Thr Arg Pro Arg Val Met Ala Pro Pro Ser Thr Gly Pro
        595                 600                 605

Arg Val Met Ala Thr Pro Ser Thr Gly Pro Arg Asp Met Ala Pro Pro
    610                 615                 620

Ser Thr Gly Pro Arg Asp Met Ala Pro Pro Ser Thr Gly Pro Arg Asp
625                 630                 635                 640

Met Ala Pro Pro Ser Thr Gly Pro Arg Asp Met Ala Pro Thr Val Val
                645                 650                 655

His Met Phe Thr Arg Glu Arg Leu Leu Thr Gln Ser Thr Gly Pro Ala
            660                 665                 670

Pro Arg Ser Phe Trp Glu Met Arg Ala Gly Arg Asp Ala Pro Lys Ile
        675                 680                 685

Gln Gln Glu Pro Ser Ser Gln Gln Gln Pro Ala Thr Gln Ser Thr Pro
    690                 695                 700

Pro Cys Gln Ser Trp Val Pro Ser Val Tyr Val Leu Pro Ala Val Asp
705                 710                 715                 720

Ala Gly Asn Ala Gln Pro Leu Gln Ile Ser His Leu Ser Ser Met Ser
                725                 730                 735

Pro Thr Gln Pro Ile Ser His Glu Glu Gln Pro Arg Tyr Glu Asp Pro
            740                 745                 750

Asp Thr Pro Leu Asp Leu Ser Leu His Pro Asp Thr Ala Thr Leu Pro
        755                 760                 765

Pro Thr Gln Asp Leu Tyr Pro Gly Arg Glu Asp Leu Gln Ala Thr Gln
    770                 775                 780

Ala Pro Tyr Pro Gly Tyr Glu Glu Pro Arg Pro Pro Gln Ala Pro Phe
785                 790                 795                 800

Val Gly Asp Tyr Gly Phe Val Gln Ile Pro Ser Ala Gln Trp Glu Pro
                805                 810                 815

His Pro Ser Gln Gly Thr Tyr Gln Gly His Ile Asp Pro Gln Leu Pro
            820                 825                 830

Ala Ala Leu Asp Leu Gly Pro Glu Gln Pro Arg Phe Pro Gln Asp Pro
        835                 840                 845

Tyr Val Tyr Ser Gly Gly Gln Leu Ser Ser Cys Pro Gly Tyr Ala Gly

850                 855                 860

Pro Trp Pro Ser Arg Pro Gln His Pro Arg Tyr Arg His Thr Leu Ala
865                 870                 875                 880

Leu Trp Pro Arg Glu Pro Arg His Gly His Ser Gln Gly Pro Trp Lys
                885                 890                 895

Pro Trp Ser Ala His Leu Pro Pro Gln Trp Asp Gly Ser Ala Gly His
            900                 905                 910

Gly Gln Asp Gln Val Ser Gln Phe Pro His Leu His Ser Glu Thr Gly
        915                 920                 925

Pro Pro Arg Leu Gln Leu Ser Ser Val Pro Gln Val Leu Tyr Pro Gln
    930                 935                 940

Pro Leu Val Ser Ser Ser Ala Pro Ser Trp Ser Ser Pro Gln Pro Arg
945                 950                 955                 960

Ala Pro Ile Arg Pro Ile Pro Thr Arg Phe Pro Pro Pro Pro Met Pro
                965                 970                 975

Leu Gln Asp Ser Met Ala Val Gly Cys Asp Ser Ser Gly Thr Ala Cys
            980                 985                 990

Pro Ser Met Pro Phe Ala Ser Asp  Tyr Ser Gln Gly Ala  Phe Thr Pro
        995                 1000                1005

Leu Asp  Ile Asn Ala Pro Thr  Pro Lys Ser Pro Arg  Val Glu Glu
    1010                1015                1020

Ser Ser  His Gly Pro Ala Arg  Cys Ser Gln Ala Thr  Ser Glu Ala
    1025                1030                1035

Gln Glu  Ile Leu Ser Asp Asn  Ser Glu Ile Ser Val  Phe Pro Lys
    1040                1045                1050

Asp Ala  Lys Gln Thr Asp Tyr  Asp Ala Ser Thr Glu  Ser Glu Leu
    1055                1060                1065

Asp

<210>  14
<211>  992
<212>  PRT
<213>  Artificial

<220>
<223>  EBV EBNA6 Strain B95-8

<400>  14

Met Glu Ser Phe Glu Gly Gln Gly Asp Ser Arg Gln Ser Pro Asp Asn
1               5                   10                  15

Glu Arg Gly Asp Asn Val Gln Thr Thr Gly Glu His Asp Gln Asp Pro
            20                  25                  30

Gly Pro Gly Pro Pro Ser Ser Gly Ala Ser Glu Arg Leu Val Pro Glu
        35                  40                  45

Glu Ser Tyr Ser Arg Asp Gln Gln Pro Trp Gly Gln Ser Arg Gly Asp
    50                  55                  60

Glu Asn Arg Gly Trp Met Gln Arg Ile Arg Arg Arg Arg Arg Arg Arg
65                  70                  75                  80

Ala Ala Leu Ser Gly His Leu Leu Asp Thr Glu Asp Asn Val Pro Pro
                85                  90                  95

Trp Leu Pro Pro His Asp Ile Thr Pro Tyr Thr Ala Arg Asn Ile Arg
            100                 105                 110

Asp Ala Ala Cys Arg Ala Val Lys Gln Ser His Leu Gln Ala Leu Ser
        115                 120                 125

Asn Leu Ile Leu Asp Ser Gly Leu Asp Thr Gln His Ile Leu Cys Phe
    130                 135                 140

Val Met Ala Ala Arg Gln Arg Leu Gln Asp Ile Arg Arg Gly Pro Leu
145                 150                 155                 160

Val Ala Glu Gly Gly Val Gly Trp Arg His Trp Leu Leu Thr Ser Pro
                165                 170                 175

Ser Gln Ser Trp Pro Met Gly Tyr Arg Thr Ala Thr Leu Arg Thr Leu
            180                 185                 190

Thr Pro Val Pro Asn Arg Val Gly Ala Asp Ser Ile Met Leu Thr Ala
        195                 200                 205

Thr Phe Gly Cys Gln Asn Ala Ala Arg Thr Leu Asn Thr Phe Ser Ala
    210                 215                 220

Thr Val Trp Thr Pro Pro His Ala Gly Pro Arg Glu Gln Glu Arg Tyr

225                 230                 235                 240

Ala Arg Glu Ala Glu Val Arg Phe Leu Arg Gly Lys Trp Gln Arg Arg
                245                 250                 255

Tyr Arg Arg Ile Tyr Asp Leu Ile Glu Leu Cys Gly Ser Leu His His
            260                 265                 270

Ile Trp Gln Asn Leu Leu Gln Thr Glu Glu Asn Leu Leu Asp Phe Val
        275                 280                 285

Arg Phe Met Gly Val Met Ser Ser Cys Asn Asn Pro Ala Val Asn Tyr
    290                 295                 300

Trp Phe His Lys Thr Ile Gly Asn Phe Lys Pro Tyr Tyr Pro Trp Asn
305                 310                 315                 320

Ala Pro Pro Asn Glu Asn Pro Tyr His Ala Arg Arg Gly Ile Lys Glu
                325                 330                 335

His Val Ile Gln Asn Ala Phe Arg Lys Ala Gln Ile Gln Gly Leu Ser
            340                 345                 350

Met Leu Ala Thr Gly Gly Glu Pro Arg Gly Asp Ala Thr Ser Glu Thr
        355                 360                 365

Ser Ser Asp Glu Asp Thr Gly Arg Gln Gly Ser Asp Val Glu Leu Glu
    370                 375                 380

Ser Ser Asp Asp Glu Leu Pro Tyr Ile Asp Pro Asn Met Glu Pro Val
385                 390                 395                 400

Gln Gln Arg Pro Val Met Phe Val Ser Arg Val Pro Ala Lys Lys Pro
                405                 410                 415

Arg Lys Leu Pro Trp Pro Thr Pro Lys Thr His Pro Val Lys Arg Thr
            420                 425                 430

Asn Val Lys Thr Ser Asp Arg Ser Asp Lys Ala Glu Ala Gln Ser Thr
        435                 440                 445

Pro Glu Arg Pro Gly Pro Ser Glu Gln Ser Ser Val Thr Val Glu Pro
    450                 455                 460

Ala His Pro Thr Pro Val Glu Met Pro Met Val Ile Leu His Gln Pro
465                 470                 475                 480

Pro Pro Val Pro Lys Pro Val Pro Val Lys Pro Thr Pro Pro Pro Ser
                485                 490                 495

Arg Arg Arg Arg Gly Ala Cys Val Val Tyr Asp Asp Asp Val Ile Glu
            500                 505                 510

Val Ile Asp Val Glu Thr Thr Glu Asp Ser Ser Ser Val Ser Gln Pro
        515                 520                 525

Asn Lys Pro His Arg Lys His Gln Asp Gly Phe Gln Arg Ser Gly Arg
    530                 535                 540

Arg Gln Lys Arg Ala Ala Pro Pro Thr Val Ser Pro Ser Asp Thr Gly
545                 550                 555                 560

Pro Pro Ala Val Gly Pro Pro Ala Ala Gly Pro Pro Ala Ala Gly Pro
                565                 570                 575

Pro Ala Ala Gly Pro Pro Ala Ala Gly Pro Pro Ala Ala Gly Pro Pro
            580                 585                 590

Ala Ala Gly Pro Arg Ile Leu Ala Pro Leu Ser Ala Gly Pro Pro Ala
        595                 600                 605

Ala Gly Pro His Ile Val Thr Pro Pro Ser Ala Arg Pro Arg Ile Met
    610                 615                 620

Ala Pro Pro Val Val Arg Met Phe Met Arg Glu Arg Gln Leu Pro Gln
625                 630                 635                 640

Ser Thr Gly Arg Lys Pro Gln Cys Phe Trp Glu Met Arg Ala Gly Arg
                645                 650                 655

Glu Ile Thr Gln Met Gln Gln Glu Pro Ser Ser His Leu Gln Ser Ala
            660                 665                 670

Thr Gln Pro Thr Thr Pro Arg Pro Ser Trp Ala Pro Ser Val Cys Ala
        675                 680                 685

Leu Ser Val Met Asp Ala Gly Lys Ala Gln Pro Ile Glu Ser Ser His
    690                 695                 700

Leu Ser Ser Met Ser Pro Thr Gln Pro Ile Ser His Glu Glu Gln Pro
705                 710                 715                 720

Arg Tyr Glu Asp Pro Asp Ala Pro Leu Asp Leu Ser Leu His Pro Asp
                725                 730                 735

Val Ala Ala Gln Pro Ala Pro Gln Ala Pro Tyr Gln Gly Tyr Gln Glu
        740                 745                 750

Pro Pro Ala Pro Gln Ala Pro Tyr Gln Gly Tyr Gln Glu Pro Pro Pro
    755                 760                 765

Pro Gln Ala Pro Tyr Gln Gly Tyr Gln Glu Pro Pro Ala His Gly Leu
    770                 775                 780

Gln Ser Ser Ser Tyr Pro Gly Tyr Ala Gly Pro Trp Thr Pro Arg Ser
785                 790                 795                 800

Gln His Pro Cys Tyr Arg His Pro Trp Ala Pro Trp Ser Gln Asp Pro
                805                 810                 815

Val His Gly His Thr Gln Gly Pro Trp Asp Pro Arg Ala Pro His Leu
            820                 825                 830

Pro Pro Gln Trp Asp Gly Ser Ala Gly His Gly Gln Asp Gln Val Ser
        835                 840                 845

Gln Phe Pro His Leu Gln Ser Glu Thr Gly Pro Pro Arg Leu Gln Leu
    850                 855                 860

Ser Leu Val Pro Leu Val Ser Ser Ser Ala Pro Ser Trp Ser Ser Pro
865                 870                 875                 880

Gln Pro Arg Ala Pro Ile Arg Pro Ile Pro Thr Arg Phe Pro Pro Pro
                885                 890                 895

Pro Met Pro Leu Gln Asp Ser Met Ala Val Gly Cys Asp Ser Ser Gly
            900                 905                 910

Thr Ala Cys Pro Ser Met Pro Phe Ala Ser Asp Tyr Ser Gln Gly Ala
        915                 920                 925

Phe Thr Pro Leu Asp Ile Asn Ala Thr Thr Pro Lys Arg Pro Arg Val
    930                 935                 940

Glu Glu Ser Ser His Gly Pro Ala Arg Cys Ser Gln Ala Thr Ala Glu
945                 950                 955                 960

Ala Gln Glu Ile Leu Ser Asp Asn Ser Glu Ile Ser Val Phe Pro Lys
                965                 970                 975

Asp Ala Lys Gln Thr Asp Tyr Asp Ala Ser Thr Glu Ser Glu Leu Asp
            980                 985                 990

<210>  15
<211>  1009
<212>  PRT
<213>  Artificial

<220>
<223>  EBV EBNA6 Strain GD1

<400>  15

Met Glu Ser Phe Glu Gly Gln Gly Asp Ser Arg Gln Ser Pro Asp Asn
1               5                   10                  15

Glu Arg Gly Asp Asn Val Gln Thr Thr Gly Glu His Asp Gln Asp Pro
            20                  25                  30

Gly Pro Gly Pro Pro Ser Ser Gly Ala Ser Glu Arg Leu Val Pro Glu
        35                  40                  45

Glu Ser Tyr Ser Arg Asp Gln Gln Pro Trp Gly Gln Ser Arg Gly Asp
    50                  55                  60

Glu Asn Arg Gly Trp Met Gln Arg Ile Arg Arg Arg Arg Arg Arg Arg
65                  70                  75                  80

Ala Ala Leu Ser Gly His Leu Leu Asp Thr Glu Asp Asn Val Pro Pro
                85                  90                  95

Trp Leu Pro Pro His Asp Ile Ala Pro Tyr Val Ala Arg Asn Ile Arg
            100                 105                 110

Asp Ala Ala Cys Arg Ala Val Lys Gln Ser His Leu Gln Ala Leu Ser
        115                 120                 125

Asn Leu Ile Leu Asp Ser Gly Leu Asp Thr Gln His Ile Leu Cys Phe
    130                 135                 140

Val Met Ala Ala Arg Gln Arg Leu Gln Asp Ile Arg Arg Gly Pro Leu
145                 150                 155                 160

Val Val Glu Gly Gly Val Gly Trp Arg His Trp Leu Leu Thr Ser Pro
                165                 170                 175

Ser Gln Ser Trp Pro Met Gly Tyr Arg Thr Ala Thr Leu Arg Thr Leu
            180                 185                 190

Thr Pro Val Pro Asn Arg Val Gly Ala Asp Ser Ile Met Leu Thr Ala
        195                 200                 205

Thr Phe Gly Cys Gln Asn Ala Ala Arg Thr Leu Asn Thr Phe Ser Ala
    210                 215                 220

Thr Val Trp Thr Pro Pro His Ala Gly Pro Arg Glu Gln Glu Arg Tyr
225                 230                 235                 240

Ala Arg Glu Ala Glu Val Arg Phe Leu Arg Gly Lys Trp Gln Arg Arg
                245                 250                 255

Tyr Arg Arg Ile Tyr Asp Leu Ile Glu Leu Cys Gly Ser Leu His His
            260                 265                 270

Ile Trp Gln Asn Leu Leu Gln Thr Glu Glu Asn Leu Leu Asp Phe Val
        275                 280                 285

Arg Phe Met Gly Val Met Ser Ser Cys Asn Asn Pro Ala Val Asn Tyr
    290                 295                 300

Trp Phe His Lys Thr Ile Gly Asn Phe Lys Pro Tyr Tyr Pro Trp Asn
305                 310                 315                 320

Ala Pro Pro Asn Glu Asn Pro Tyr His Ala Arg Arg Gly Ile Lys Glu
                325                 330                 335

His Val Ile Gln Asn Ala Phe Arg Lys Ala Gln Ile Gln Gly Leu Ser
            340                 345                 350

Met Leu Ala Thr Val Gly Glu Pro Arg Gly Asp Ala Thr Ser Glu Thr
        355                 360                 365

Ser Ser Asp Glu Asp Thr Gly Arg Gln Gly Ser Asp Val Glu Leu Glu
    370                 375                 380

Ser Ser Asp Asp Glu Leu Pro Tyr Ile Asp Pro Asn Met Glu Pro Val
385                 390                 395                 400

Gln Gln Arg Pro Val Met Phe Val Ser Arg Val Pro Ala Lys Lys Pro
                405                 410                 415

Arg Lys Leu Pro Trp Pro Thr Pro Lys Thr His Pro Val Lys Arg Thr
            420                 425                 430

Asn Val Lys Thr Ser Asp Arg Ser Asp Lys Ala Glu Ala Gln Ser Thr
        435                 440                 445

Pro Glu Arg Pro Gly Pro Ser Glu Gln Ser Ser Val Thr Val Glu Pro
    450                 455                 460

Ala His Pro Thr Pro Val Glu Met Pro Met Val Ile Leu His Gln Pro
465                 470                 475                 480

Pro Pro Val Pro Lys Pro Val Pro Val Lys Pro Thr Pro Pro Pro Ser
                485                 490                 495

Arg Arg Arg Arg Gly Ala Cys Val Val Tyr Asp Asp Asp Val Ile Glu
            500                 505                 510

Val Ile Asp Val Glu Thr Thr Glu Asp Ser Ser Ser Val Ser Gln Pro
        515                 520                 525

Asn Lys Pro His Arg Lys His Gln Asp Gly Phe Gln Arg Ser Gly Arg
    530                 535                 540

Arg Gln Lys Arg Ala Ala Pro Pro Thr Val Ser Pro Ser Asp Thr Gly
545                 550                 555                 560

Pro Pro Ala Ala Gly Pro Pro Ala Ala Gly Pro Pro Ala Ala Gly Pro
                565                 570                 575

His Ile Leu Thr Pro Pro Ser Ala Arg Pro Arg Ile Met Ala Pro Pro
            580                 585                 590

Val Val Arg Met Phe Met Arg Glu Arg Gln Leu Pro Gln Ser Thr Gly
        595                 600                 605

Arg Lys Pro Gln Cys Phe Trp Glu Met Arg Ala Ser Arg Glu Ile Thr
    610                 615                 620

Gln Met Gln Gln Glu Pro Ser Ser His Leu Gln Ser Ala Thr Gln Pro
625                 630                 635                 640

Thr Met Pro Arg Pro Ser Trp Val Pro Ser Val Cys Ala Leu Ser Val
                645                 650                 655

Met Asp Ala Gly Lys Ala Gln Pro Ile Gln Ser Ser His Leu Ser Ser
            660                 665                 670

Met Ser Pro Thr Gln Pro Ile Ser His Glu Glu Gln Pro Arg Tyr Glu
        675                 680                 685

Asp Pro Asp Ala Pro Leu Asp Leu Ser Leu His Pro Asp Val Ala Ala
    690                 695                 700

Pro Pro Ala Pro Arg Ala Pro Tyr Gln Gly Tyr Gln Glu Gln Pro Ala

705                 710                 715                 720

Pro Gln Ala Pro Tyr Gln Gly Tyr Gln Glu Gln Pro Ala Pro Gln Ala
                725                 730                 735

Pro Tyr Gln Gly Tyr Gln Glu Gln Pro Ala Pro Gln Ala Pro Tyr Gln
            740                 745                 750

Gly Tyr Gln Glu Gln Pro Ala Pro Gln Ala Pro Tyr Gln Gly Tyr Gln
        755                 760                 765

Glu Gln Pro Pro Pro Gln Ala Pro Tyr Gln Gly Tyr Gln Glu Gln Pro
    770                 775                 780

Ala Pro Gln Ala Pro Tyr Gln Gly Tyr Gln Glu Pro Pro Ala His Gly
785                 790                 795                 800

Leu Gln Ser Ser Ser Tyr Pro Gly Tyr Ala Gly Pro Trp Thr Pro Arg
                805                 810                 815

Ser Gln His Pro Cys Tyr Arg His Pro Trp Ala Pro Trp Ser Gln Asp
            820                 825                 830

Pro Val His Gly His Thr Gln Gly Pro Trp Asp Pro Arg Ala Pro His
        835                 840                 845

Leu Pro Pro Gln Trp Asp Gly Ser Ala Gly His Gly Gln Asp Gln Val
    850                 855                 860

Ser Gln Phe Pro His Leu Gln Ser Glu Thr Gly Pro Pro Arg Leu Gln
865                 870                 875                 880

Leu Ser Ser Val Pro Leu Val Ser Ser Ser Ala Pro Ser Trp Ser Ser
                885                 890                 895

Pro Gln Pro Arg Ala Pro Ile Arg Pro Ile Pro Thr Arg Phe Pro Pro
            900                 905                 910

Pro Pro Met Pro Leu Gln Asp Ser Met Ala Val Gly Cys Asp Ser Ser
        915                 920                 925

Gly Thr Ala Cys Pro Ser Met Pro Phe Ala Ser Asp Tyr Ser Gln Gly
    930                 935                 940

Ala Phe Thr Pro Leu Asp Ile Asn Ala Thr Thr Pro Lys Arg Pro Arg
945                 950                 955                 960

Val Glu Glu Ser Ser His Gly Pro Ala Arg Cys Ser Gln Ala Thr Ala
                965                 970                 975

Glu Ala Gln Glu Ile Leu Ser Asp Asn Ser Glu Ile Ser Val Phe Pro
            980                 985                 990

Lys Asp Ala Lys Gln Thr Asp Tyr  Asp Ala Ser Thr Glu  Ser Glu Leu
        995                 1000                1005

Asp

<210>  16
<211>  371
<212>  PRT
<213>  Artificial

<220>
<223>  EBV LMP1 Strain AG876

<400>  16

Met Glu His Asp Leu Glu Arg Gly Pro Pro Gly Pro Arg Arg Pro Pro
1               5                   10                  15

Arg Gly Pro Pro Leu Ser Ser Ser Leu Gly Leu Ala Leu Leu Leu Leu
            20                  25                  30

Leu Leu Ala Leu Leu Phe Trp Leu Tyr Ile Val Met Ser Asp Trp Thr
        35                  40                  45

Gly Gly Ala Leu Leu Val Leu Tyr Ser Phe Ala Leu Ile Leu Ile Ile
    50                  55                  60

Ile Ile Leu Ile Ile Phe Ile Phe Arg Arg Asp Leu Leu Cys Pro Leu
65                  70                  75                  80

Gly Ala Leu Cys Leu Leu Leu Leu Met Ile Thr Leu Leu Leu Ile Ala
                85                  90                  95

Leu Trp Asn Leu His Gly Gln Ala Leu Tyr Leu Gly Ile Val Leu Phe
            100                 105                 110

Ile Phe Gly Cys Leu Leu Val Leu Gly Leu Trp Ile Tyr Leu Leu Glu
        115                 120                 125

Ile Leu Trp Arg Leu Gly Ala Thr Ile Trp Gln Leu Leu Ala Phe Phe
    130                 135                 140

Leu Ala Phe Phe Leu Asp Leu Ile Leu Leu Ile Ile Ala Leu Tyr Leu

145                 150                 155                 160

Gln Gln Asn Trp Trp Thr Leu Leu Val Asp Leu Leu Trp Leu Leu Leu
                165                 170                 175

Phe Leu Ala Ile Leu Ile Trp Met Tyr Tyr His Gly Gln Arg His Ser
            180                 185                 190

Asp Glu His His His Asp Asp Ser Leu Pro His Pro Gln Gln Ala Thr
        195                 200                 205

Asp Asp Ser Gly His Glu Ser Asp Ser Asn Ser Asn Glu Gly Arg His
    210                 215                 220

His Leu Leu Val Thr Gly Ala Gly Asp Gly Pro Pro Leu Cys Ser Gln
225                 230                 235                 240

Asn Leu Gly Ala Pro Gly Gly Gly Pro Asp Asn Gly Pro Gln Asp Pro
                245                 250                 255

Asp Asn Thr Asp Asp Asn Gly Pro Gln Asp Pro Asp Asn Thr Asp Asp
            260                 265                 270

Asn Gly Pro Gln Asp Pro Asp Asn Thr Asp Asp Asn Gly Pro Gln Asp
        275                 280                 285

Pro Asp Asn Thr Ala Asp Asn Gly Pro His Asp Pro Leu Pro His Asn
    290                 295                 300

Pro Ser Asp Ser Ala Gly Asn Asp Gly Gly Pro Pro Asn Leu Thr Glu
305                 310                 315                 320

Glu Val Glu Asn Lys Gly Gly Asp Arg Gly Pro Pro Ser Met Thr Asp
                325                 330                 335

Gly Gly Gly Gly Asp Pro His Leu Pro Thr Leu Leu Leu Gly Thr Ser
            340                 345                 350

Gly Ser Gly Gly Asp Asp Asp Asp Pro His Gly Pro Val Gln Leu Ser
        355                 360                 365

Tyr Tyr Asp
    370

<210>  17
<211>  386
<212>  PRT
<213>  Artificial

<220>
<223>  EBV LMP1 Strain B95-8

<400>  17

Met Glu His Asp Leu Glu Arg Gly Pro Pro Gly Pro Arg Arg Pro Pro
1               5                   10                  15

Arg Gly Pro Pro Leu Ser Ser Ser Leu Gly Leu Ala Leu Leu Leu Leu
            20                  25                  30

Leu Leu Ala Leu Leu Phe Trp Leu Tyr Ile Val Met Ser Asp Trp Thr
        35                  40                  45

Gly Gly Ala Leu Leu Val Leu Tyr Ser Phe Ala Leu Met Leu Ile Ile
    50                  55                  60

Ile Ile Leu Ile Ile Phe Ile Phe Arg Arg Asp Leu Leu Cys Pro Leu
65                  70                  75                  80

Gly Ala Leu Cys Ile Leu Leu Leu Met Ile Thr Leu Leu Leu Ile Ala
                85                  90                  95

Leu Trp Asn Leu His Gly Gln Ala Leu Phe Leu Gly Ile Val Leu Phe
            100                 105                 110

Ile Phe Gly Cys Leu Leu Val Leu Gly Ile Trp Ile Tyr Leu Leu Glu
        115                 120                 125

Met Leu Trp Arg Leu Gly Ala Thr Ile Trp Gln Leu Leu Ala Phe Phe
    130                 135                 140

Leu Ala Phe Phe Leu Asp Leu Ile Leu Leu Ile Ile Ala Leu Tyr Leu
145                 150                 155                 160

Gln Gln Asn Trp Trp Thr Leu Leu Val Asp Leu Leu Trp Leu Leu Leu
                165                 170                 175

Phe Leu Ala Ile Leu Ile Trp Met Tyr Tyr His Gly Gln Arg His Ser
            180                 185                 190

Asp Glu His His His Asp Asp Ser Leu Pro His Pro Gln Gln Ala Thr
        195                 200                 205

Asp Asp Ser Gly His Glu Ser Asp Ser Asn Ser Asn Glu Gly Arg His
    210                 215                 220

His Leu Leu Val Ser Gly Ala Gly Asp Gly Pro Pro Leu Cys Ser Gln

225                 230                 235                 240

Asn Leu Gly Ala Pro Gly Gly Gly Pro Asp Asn Gly Pro Gln Asp Pro
                245                 250                 255

Asp Asn Thr Asp Asp Asn Gly Pro Gln Asp Pro Asp Asn Thr Asp Asp
            260                 265                 270

Asn Gly Pro His Asp Pro Leu Pro Gln Asp Pro Asp Asn Thr Asp Asp
        275                 280                 285

Asn Gly Pro Gln Asp Pro Asp Asn Thr Asp Asp Asn Gly Pro His Asp
    290                 295                 300

Pro Leu Pro His Ser Pro Ser Asp Ser Ala Gly Asn Asp Gly Gly Pro
305                 310                 315                 320

Pro Gln Leu Thr Glu Glu Val Glu Asn Lys Gly Gly Asp Gln Gly Pro
                325                 330                 335

Pro Leu Met Thr Asp Gly Gly Gly Gly His Ser His Asp Ser Gly His
            340                 345                 350

Gly Gly Gly Asp Pro His Leu Pro Thr Leu Leu Leu Gly Ser Ser Gly
        355                 360                 365

Ser Gly Gly Asp Asp Asp Asp Pro His Gly Pro Val Gln Leu Ser Tyr
    370                 375                 380

Tyr Asp
385

<210>  18
<211>  404
<212>  PRT
<213>  Artificial

<220>
<223>  EBV LMP1 Strain Cao

<400>  18

Met Glu Arg Asp Leu Glu Ser Ala Pro Pro Ser Ala Pro Arg Pro Pro
1               5                   10                  15

Leu Gly Pro Pro Leu Ser Ser Ser Ile Gly Leu Ala Leu Leu Leu Leu
            20                  25                  30

Leu Leu Ala Leu Leu Phe Trp Leu Tyr Ile Val Met Ser Asp Trp Thr
        35                  40                  45

Gly Gly Ala Leu Leu Val Leu Tyr Ser Phe Ala Leu Met Leu Ile Ile
    50                  55                  60

Ile Ile Leu Ile Ile Phe Ile Phe Arg Arg Asp Leu Leu Cys Pro Leu
65                  70                  75                  80

Gly Gly Leu Gly Leu Leu Leu Leu Met Ile Thr Leu Leu Leu Ile Ala
                85                  90                  95

Leu Trp Asn Leu His Gly Gln Ala Leu Tyr Leu Gly Ile Val Leu Phe
            100                 105                 110

Ile Phe Gly Cys Leu Leu Val Phe Gly Ile Trp Ile Tyr Phe Leu Glu
        115                 120                 125

Ile Leu Trp Arg Leu Gly Ala Thr Leu Trp Gln Leu Leu Ala Phe Ile
    130                 135                 140

Leu Ala Phe Phe Leu Ala Ile Ile Leu Leu Ile Ile Ala Leu Tyr Leu
145                 150                 155                 160

Gln Gln Asn Trp Trp Thr Leu Leu Val Asp Leu Leu Trp Leu Leu Leu
                165                 170                 175

Phe Met Ala Ile Leu Ile Trp Met Tyr Tyr His Gly Pro Arg His Thr
            180                 185                 190

Asp Glu His His His Asp Asp Ser Leu Pro His Pro Gln Gln Ala Thr
        195                 200                 205

Asp Asp Ser Ser His Glu Ser Asp Ser Asn Ser Asn Glu Gly Arg His
    210                 215                 220

His Leu Leu Val Ser Gly Ala Gly Asp Gly Pro Pro Leu Cys Ser Gln
225                 230                 235                 240

Asn Leu Gly Ala Pro Gly Gly Gly Pro Asp Asn Gly Pro Gln Asp Pro
                245                 250                 255

Asp Asn Thr Asp Asp Asn Gly Pro Gln Asp Pro Asp Asn Thr Asp Asp
            260                 265                 270

Asn Gly Pro Gln Asp Pro Asp Asn Thr Asp Asp Asn Gly Pro Gln Asp
        275                 280                 285

Pro Asp Asn Thr Asp Asp Asn Gly Pro Gln Asp Pro Asp Asn Thr Asp

290                 295                 300

Asp Asn Gly Pro Gln Asp Pro Asp Asn Thr Asp Asp Asn Gly Pro Gln
305                 310                 315                 320

Asp Pro Asp Asn Thr Asp Asp Asn Gly Pro His Asp Pro Leu Pro His
                325                 330                 335

Ser Pro Ser Asp Ser Ala Gly Asn Asp Gly Gly Pro Pro Asn Leu Thr
            340                 345                 350

Glu Glu Val Ala Asn Lys Gly Gly Asp Arg Gly Pro Pro Ser Met Thr
        355                 360                 365

Asp Gly Gly Gly Gly Asp Pro His Leu Pro Thr Leu Leu Leu Gly Thr
    370                 375                 380

Ser Gly Ser Gly Gly Asp Asp Asp Asp Pro His Gly Pro Val Gln Leu
385                 390                 395                 400

Ser Tyr Tyr Asp

<210>  19
<211>  366
<212>  PRT
<213>  Artificial

<220>
<223>  EBV LMP1 Strain GD1

<400>  19

Met Glu Arg Asp Leu Glu Arg Gly Pro Pro Gly Pro Pro Arg Pro Pro
1               5                   10                  15

Leu Gly Pro Pro Leu Ser Ser Ser Ile Gly Leu Ala Leu Leu Leu Leu
            20                  25                  30

Leu Leu Ala Leu Leu Phe Trp Leu Tyr Ile Val Leu Ser Asn Trp Thr
        35                  40                  45

Gly Gly Ala Leu Leu Val Leu Tyr Ser Phe Ala Leu Met Leu Ile Ile
    50                  55                  60

Ile Ile Leu Ile Ile Phe Ile Phe Arg Arg Asp Leu Leu Cys Pro Leu
65                  70                  75                  80

Gly Gly Leu Gly Leu Leu Leu Leu Met Val Thr Leu Leu Leu Ile Ala
                85                  90                  95

Leu Trp Asn Leu His Gly Gln Ala Leu Tyr Leu Gly Ile Val Leu Phe
            100                 105                 110

Ile Phe Gly Cys Leu Leu Val Leu Gly Leu Trp Ile Tyr Phe Leu Glu
        115                 120                 125

Ile Leu Trp Arg Leu Gly Ala Thr Ile Trp Gln Leu Leu Ala Phe Ile
    130                 135                 140

Leu Ala Phe Phe Leu Ala Ile Ile Leu Leu Ile Ile Ala Leu Tyr Leu
145                 150                 155                 160

Gln Gln Asn Trp Trp Thr Leu Leu Val Asp Leu Leu Trp Leu Leu Leu
                165                 170                 175

Phe Met Ala Ile Leu Ile Trp Met Tyr Phe His Gly Pro Arg His Thr
            180                 185                 190

Asp Glu His His His Asp Asp Ser Leu Pro His Pro Gln Gln Ala Thr
        195                 200                 205

Asp Asp Ser Ser His Glu Ser Asp Ser Asn Ser Asn Glu Gly Arg His
    210                 215                 220

His Leu Leu Val Ser Gly Ala Gly Asp Gly Pro Pro Leu Cys Ser Gln
225                 230                 235                 240

Asn Leu Gly Ala Pro Gly Gly Gly Pro Asp Asn Gly Pro Gln Asp Pro
                245                 250                 255

Asp Asn Thr Asp Asp Asn Gly Pro Gln Asp Pro Asp Asn Thr Asp Asp
            260                 265                 270

Asn Gly Asn Thr Asp Asp Asn Gly Pro Gln Asp Pro Asp Asn Thr Asp
        275                 280                 285

Asp Asn Gly Pro His Asp Pro Leu Pro His Asn Pro Ser Asp Ser Ala
    290                 295                 300

Gly Asn Asp Gly Gly Pro Pro Asn Leu Thr Glu Glu Val Glu Asn Lys
305                 310                 315                 320

Gly Gly Asp Arg Gly Pro Pro Ser Met Thr Asp Gly Gly Gly Gly Asp
                325                 330                 335

Pro His Leu Pro Thr Leu Leu Leu Gly Thr Ser Gly Ser Gly Gly Asp

340                 345                 350

Asp Asp Asp Pro His Gly Pro Val Gln Leu Ser Tyr Tyr Asp
        355                 360                 365

<210>  20
<211>  386
<212>  PRT
<213>  Artificial

<220>
<223>  EBV LMP1 Strain Raji

<400>  20

Met Asp Leu Asp Leu Glu Arg Gly Pro Pro Gly Pro Arg Arg Pro Pro
1               5                   10                  15

Arg Gly Pro Pro Leu Ser Ser Ser Ile Gly Leu Ala Leu Leu Leu Leu
            20                  25                  30

Leu Leu Ala Leu Leu Phe Trp Leu Tyr Ile Ile Met Ser Asn Trp Thr
        35                  40                  45

Gly Gly Ala Leu Leu Val Leu Tyr Ala Phe Ala Leu Met Leu Val Ile
    50                  55                  60

Ile Ile Leu Ile Ile Phe Ile Phe Arg Arg Asp Leu Leu Cys Pro Leu
65                  70                  75                  80

Gly Ala Leu Cys Leu Leu Leu Leu Met Ile Thr Leu Leu Leu Ile Ala
                85                  90                  95

Leu Trp Asn Leu His Gly Gln Ala Leu Tyr Leu Gly Ile Val Leu Phe
            100                 105                 110

Ile Phe Gly Cys Leu Leu Val Leu Gly Leu Trp Ile Tyr Leu Leu Glu
        115                 120                 125

Ile Leu Trp Arg Leu Gly Ala Thr Ile Trp Gln Leu Leu Ala Phe Phe
    130                 135                 140

Leu Ala Phe Phe Leu Asp Ile Ile Leu Leu Ile Ile Ala Leu Tyr Leu
145                 150                 155                 160

Gln Gln Asn Trp Trp Thr Leu Leu Val Asp Leu Leu Trp Leu Leu Leu
                165                 170                 175

Phe Leu Ala Ile Leu Ile Trp Met Tyr Tyr His Gly Gln Arg His Ser
            180                 185                 190

Asp Glu His His His Asp Asp Ser Leu Pro His Pro Gln Gln Ala Thr
        195                 200                 205

Asp Asp Ser Ser Asn Gln Ser Asp Ser Asn Ser Asn Glu Gly Arg His
    210                 215                 220

Leu Leu Leu Val Ser Gly Ala Gly Asp Gly Pro Pro Leu Cys Ser Gln
225                 230                 235                 240

Asn Leu Gly Ala Pro Gly Gly Gly Pro Asn Asn Gly Pro Gln Asp Pro
                245                 250                 255

Asp Asn Thr Asp Asp Asn Gly Pro Gln Asp Pro Asp Asn Thr Asp Asp
            260                 265                 270

Asn Gly Pro His Asp Pro Leu Pro Gln Asp Pro Asp Asn Thr Asp Asp
        275                 280                 285

Asn Gly Pro Gln Asp Pro Asp Asn Thr Asp Asp Asn Gly Pro His Asp
    290                 295                 300

Pro Leu Pro His Asn Pro Ser Asp Ser Ala Gly Asn Asp Gly Gly Pro
305                 310                 315                 320

Pro Gln Leu Thr Glu Glu Val Glu Asn Lys Gly Gly Asp Gln Gly Pro
                325                 330                 335

Pro Leu Met Thr Asp Gly Gly Gly Gly His Ser His Asp Ser Gly His
            340                 345                 350

Asp Gly Ile Asp Pro His Leu Pro Thr Leu Leu Leu Gly Thr Ser Gly
        355                 360                 365

Ser Gly Gly Asp Asp Asp Asp Pro His Gly Pro Val Gln Leu Ser Tyr
    370                 375                 380

Tyr Asp
385

<210>  21
<211>  176
<212>  PRT
<213>  Artificial

<220>
<223>  EBV VP26 Strain B95-8

<400>  21

Met Ala Arg Arg Leu Pro Lys Pro Thr Leu Gln Gly Arg Leu Glu Ala
1               5                   10                  15

Asp Phe Pro Asp Ser Pro Leu Leu Pro Lys Phe Gln Glu Leu Asn Gln
            20                  25                  30

Asn Asn Leu Pro Asn Asp Val Phe Arg Glu Ala Gln Arg Ser Tyr Leu
        35                  40                  45

Val Phe Leu Thr Ser Gln Phe Cys Tyr Glu Glu Tyr Val Gln Arg Thr
    50                  55                  60

Phe Gly Val Pro Arg Arg Gln Arg Ala Ile Asp Lys Arg Gln Arg Ala
65                  70                  75                  80

Ser Val Ala Gly Ala Gly Ala His Ala His Leu Gly Gly Ser Ser Ala
                85                  90                  95

Thr Pro Val Gln Gln Ala Gln Ala Ala Ala Ser Ala Gly Thr Gly Ala
            100                 105                 110

Leu Ala Ser Ser Ala Pro Ser Thr Ala Val Ala Gln Ser Ala Thr Pro
        115                 120                 125

Ser Val Ser Ser Ser Ile Ser Ser Leu Arg Ala Ala Thr Ser Gly Ala
    130                 135                 140

Thr Ala Ala Ala Ser Ala Ala Ala Ala Val Asp Thr Gly Ser Gly Gly
145                 150                 155                 160

Gly Gly Gln Pro His Asp Thr Ala Pro Arg Gly Ala Arg Lys Lys Gln
                165                 170                 175

<210>  22
<211>  15
<212>  PRT
<213>  Unknown

<220>
<223>  Peptide

<400>  22

Ala Tyr Gln Ala Tyr Ala Ala Pro Gln Leu Phe Pro Val Ser Asp
1               5                   10                  15

<210>  23
<211>  15
<212>  PRT
<213>  Unknown

<220>
<223>  Peptide

<400>  23

Gln Gln Pro Glu Ser Leu Glu Glu Cys Asp Ser Glu Leu Glu Ile
1               5                   10                  15


<210>  24
<211>  15
<212>  PRT
<213>  Unknown

<220>
<223>  Peptide

<400>  24

Glu Glu Cys Asp Ser Glu Leu Glu Ile Lys Arg Tyr Lys Asn Arg
1               5                   10                  15


<210>  25
<211>  15
<212>  PRT
<213>  Unknown

<220>
<223>  Peptide

<400>  25

Asp Ser Glu Leu Glu Ile Lys Arg Tyr Lys Asn Arg Val Ala Ser
1               5                   10                  15


<210>  26
<211>  15
<212>  PRT
<213>  Unknown

<220>
<223>  Peptide

<400>  26

Phe Lys Gln Leu Leu Gln His Tyr Arg Glu Val Ala Ala Ala Lys
1               5                   10                  15


<210>  27
<211>  15
<212>  PRT
<213>  Unknown

<220>
<223>  Peptide

<400>  27

Leu Leu Gln His Tyr Arg Glu Val Ala Ala Ala Lys Ser Ser Glu

1               5                   10                  15


<210>  28
<211>  15
<212>  PRT
<213>  Unknown

<220>
<223>  Peptide

<400>  28

Pro Ser Leu Asp Val Asp Ser Ile Ile Pro Arg Thr Pro Asp Val
1               5                   10                  15


<210>  29
<211>  15
<212>  PRT
<213>  Unknown

<220>
<223>  Peptide

<400>  29

Asp Val Asp Ser Ile Ile Pro Arg Thr Pro Asp Val Leu His Glu
1               5                   10                  15


<210>  30
<211>  15
<212>  PRT
<213>  Unknown

<220>
<223>  Peptide

<400>  30

Gly Leu Arg Ala Leu Leu Ala Arg Ser His Val Glu Arg Thr Thr
1               5                   10                  15


<210>  31
<211>  15
<212>  PRT
<213>  Unknown

<220>
<223>  Peptide

<400>  31

Lys Thr Ser Leu Tyr Asn Leu Arg Arg Gly Thr Ala Leu Ala Ile
1               5                   10                  15


<210>  32
<211>  15
<212>  PRT
<213>  Unknown

<220>
<223>  Peptide

<400>  32

Leu Tyr Asn Leu Arg Arg Gly Thr Ala Leu Ala Ile Pro Gln Cys
1               5                   10                  15


<210>  33
<211>  15
<212>  PRT
<213>  Unknown

<220>
<223>  Peptide

<400>  33

Gly Thr Ala Leu Ala Ile Pro Gln Cys Arg Leu Thr Pro Leu Ser
1               5                   10                  15


<210>  34
<211>  15
<212>  PRT
<213>  Unknown

<220>
<223>  Peptide

<400>  34

Leu Ala Ile Pro Gln Cys Arg Leu Thr Pro Leu Ser Arg Leu Pro
1               5                   10                  15


<210>  35
<211>  15
<212>  PRT
<213>  Unknown

<220>
<223>  Peptide

<400>  35

Arg Leu Thr Pro Leu Ser Arg Leu Pro Phe Gly Met Ala Pro Gly
1               5                   10                  15


<210>  36
<211>  15
<212>  PRT
<213>  Unknown

<220>
<223>  Peptide

<400>  36

Pro Leu Ser Arg Leu Pro Phe Gly Met Ala Pro Gly Pro Gly Pro

1               5                   10                  15


<210>  37
<211>  15
<212>  PRT
<213>  Unknown

<220>
<223>  Peptide

<400>  37

Asp Arg Pro Gly Pro Pro Ala Leu Asp Asp Asn Met Glu Glu Glu
1               5                   10                  15


<210>  38
<211>  15
<212>  PRT
<213>  Unknown

<220>
<223>  Peptide

<400>  38

Ala Leu Asp Asp Asn Met Glu Glu Glu Val Pro Ser Thr Ser Val
1               5                   10                  15


<210>  39
<211>  15
<212>  PRT
<213>  Unknown

<220>
<223>  Peptide

<400>  39

Pro Pro Gln Pro Asp Leu Pro Gly Arg Glu Ala Ile Leu Arg Arg
1               5                   10                  15


<210>  40
<211>  15
<212>  PRT
<213>  Unknown

<220>
<223>  Peptide

<400>  40

Pro Gly Arg Glu Ala Ile Leu Arg Arg Phe Pro Leu Asp Leu Arg
1               5                   10                  15


<210>  41
<211>  15
<212>  PRT
<213>  Unknown

<220>
<223>  Peptide

<400>  41

Glu Ala Ile Leu Arg Arg Phe Pro Leu Asp Leu Arg Thr Leu Leu
1               5                   10                  15


<210>  42
<211>  15
<212>  PRT
<213>  Unknown

<220>
<223>  Peptide

<400>  42

Lys Trp Arg Leu Gln Thr Leu Ala Ala Gly Trp Pro Met Gly Tyr
1               5                   10                  15


<210>  43
<211>  15
<212>  PRT
<213>  Unknown

<220>
<223>  Peptide

<400>  43

Leu Gln Thr Leu Ala Ala Gly Trp Pro Met Gly Tyr Gln Ala Tyr
1               5                   10                  15


<210>  44
<211>  15
<212>  PRT
<213>  Unknown

<220>
<223>  Peptide

<400>  44

Tyr Thr Asp His Gln Thr Thr Pro Thr Phe Val His Leu Gln Ala
1               5                   10                  15


<210>  45
<211>  15
<212>  PRT
<213>  Unknown

<220>
<223>  Peptide

<400>  45

Leu Gln Ala Thr Leu Gly Cys Thr Gly Gly Arg Arg Cys His Val

1               5                   10                  15


<210>  46
<211>  15
<212>  PRT
<213>  Unknown

<220>
<223>  Peptide

<400>  46

Thr Leu Gly Cys Thr Gly Gly Arg Arg Cys His Val Thr Phe Ser
1               5                   10                  15


<210>  47
<211>  15
<212>  PRT
<213>  Unknown

<220>
<223>  Peptide

<400>  47

Thr Phe Ser Ala Gly Thr Phe Lys Leu Pro Arg Cys Thr Pro Gly
1               5                   10                  15


<210>  48
<211>  15
<212>  PRT
<213>  Unknown

<220>
<223>  Peptide

<400>  48

Pro Arg Cys Thr Pro Gly Asp Arg Gln Trp Leu Tyr Val Gln Ser
1               5                   10                  15


<210>  49
<211>  15
<212>  PRT
<213>  Unknown

<220>
<223>  Peptide

<400>  49

Asn Pro Arg Tyr Ser Ile Phe Phe Asp Tyr Met Ala Ile His Arg
1               5                   10                  15


<210>  50
<211>  15
<212>  PRT
<213>  Unknown

<220>
<223>  Peptide

<400>  50

Tyr Ser Ile Phe Phe Asp Tyr Met Ala Ile His Arg Ser Leu Thr
1               5                   10                  15


<210>  51
<211>  15
<212>  PRT
<213>  Unknown

<220>
<223>  Peptide

<400>  51

Phe Phe Asp Tyr Met Ala Ile His Arg Ser Leu Thr Lys Ile Trp
1               5                   10                  15


<210>  52
<211>  15
<212>  PRT
<213>  Unknown

<220>
<223>  Peptide

<400>  52

Leu Gly Phe Leu Gln Arg Thr Asp Leu Ser Tyr Ile Lys Ser Phe
1               5                   10                  15


<210>  53
<211>  15
<212>  PRT
<213>  Unknown

<220>
<223>  Peptide

<400>  53

Leu Gln Arg Thr Asp Leu Ser Tyr Ile Lys Ser Phe Val Ser Asp
1               5                   10                  15


<210>  54
<211>  15
<212>  PRT
<213>  Unknown

<220>
<223>  Peptide

<400>  54

Gln Ala Trp Asn Ala Gly Phe Leu Arg Gly Arg Ala Tyr Gly Ile

1               5                   10                  15


<210>  55
<211>  15
<212>  PRT
<213>  Unknown

<220>
<223>  Peptide

<400>  55

Phe Leu Arg Gly Arg Ala Tyr Gly Ile Asp Leu Leu Arg Thr Glu
1               5                   10                  15


<210>  56
<211>  15
<212>  PRT
<213>  Unknown

<220>
<223>  Peptide

<400>  56

Tyr Gly Ile Asp Leu Leu Arg Thr Glu Gly Glu His Val Glu Gly
1               5                   10                  15


<210>  57
<211>  15
<212>  PRT
<213>  Unknown

<220>
<223>  Peptide

<400>  57

Val Ser Arg Gly Gly Pro Lys Val Lys Arg Pro Pro Ile Phe Ile
1               5                   10                  15


<210>  58
<211>  15
<212>  PRT
<213>  Unknown

<220>
<223>  Peptide

<400>  58

Gly Gly Pro Lys Val Lys Arg Pro Pro Ile Phe Ile Arg Arg Leu
1               5                   10                  15


<210>  59
<211>  15
<212>  PRT
<213>  Unknown

<220>
<223>  Peptide

<400>  59

Ser Thr Tyr Gly Thr Pro Arg Pro Pro Val Pro Lys Pro Arg Pro
1               5                   10                  15


<210>  60
<211>  15
<212>  PRT
<213>  Unknown

<220>
<223>  Peptide

<400>  60

Pro Ile Val Arg Pro Trp Glu Pro Ser Leu Thr Gln Ala Ala Gly
1               5                   10                  15


<210>  61
<211>  15
<212>  PRT
<213>  Unknown

<220>
<223>  Peptide

<400>  61

Ala Leu Glu Arg Pro Val Tyr Pro Lys Pro Val Arg Pro Ala Pro
1               5                   10                  15


<210>  62
<211>  15
<212>  PRT
<213>  Unknown

<220>
<223>  Peptide

<400>  62

Arg Pro Val Tyr Pro Lys Pro Val Arg Pro Ala Pro Pro Lys Ile
1               5                   10                  15


<210>  63
<211>  15
<212>  PRT
<213>  Unknown

<220>
<223>  Peptide

<400>  63

Leu Gly Tyr Thr Leu His Gly Leu Asn His Pro Gly Val Pro Val

1               5                   10                  15


<210>  64
<211>  15
<212>  PRT
<213>  Unknown

<220>
<223>  Peptide

<400>  64

Arg Trp Lys Leu Leu Ser Ser Cys Arg Ser Trp Arg Met Gly Tyr
1               5                   10                  15


<210>  65
<211>  15
<212>  PRT
<213>  Unknown

<220>
<223>  Peptide

<400>  65

Leu Leu Ser Ser Cys Arg Ser Trp Arg Met Gly Tyr Arg Thr His
1               5                   10                  15


<210>  66
<211>  15
<212>  PRT
<213>  Unknown

<220>
<223>  Peptide

<400>  66

Met Gly Tyr Arg Thr His Asn Leu Lys Val Asn Ser Phe Glu Ser
1               5                   10                  15


<210>  67
<211>  15
<212>  PRT
<213>  Unknown

<220>
<223>  Peptide

<400>  67

Asn Leu Lys Val Asn Ser Phe Glu Ser Gly Gly Asp Asn Val His
1               5                   10                  15


<210>  68
<211>  15
<212>  PRT
<213>  Unknown

<220>
<223>  Peptide

<400>  68

Ile Glu Thr Ala Phe Leu Met Ala Arg Arg Ala Arg Ser Leu Ser
1               5                   10                  15


<210>  69
<211>  15
<212>  PRT
<213>  Unknown

<220>
<223>  Peptide

<400>  69

Ala Phe Leu Met Ala Arg Arg Ala Arg Ser Leu Ser Ala Glu Arg
1               5                   10                  15


<210>  70
<211>  15
<212>  PRT
<213>  Unknown

<220>
<223>  Peptide

<400>  70

His Ile Arg Glu Trp Phe Arg Gln Cys Thr Gly Arg Pro Lys Ala
1               5                   10                  15


<210>  71
<211>  15
<212>  PRT
<213>  Unknown

<220>
<223>  Peptide

<400>  71

Pro Lys Ala Ala Lys Pro Trp Leu Arg Ala His Pro Val Ala Ile
1               5                   10                  15


<210>  72
<211>  15
<212>  PRT
<213>  Unknown

<220>
<223>  Peptide

<400>  72

Ala Lys Pro Trp Leu Arg Ala His Pro Val Ala Ile Pro Tyr Asp

1               5                   10                  15


<210>  73
<211>  15
<212>  PRT
<213>  Unknown

<220>
<223>  Peptide

<400>  73

Glu Ile Asp Leu Ala Tyr Ala Arg Gly Gln Ala Met Asn Ile Glu
1               5                   10                  15


<210>  74
<211>  15
<212>  PRT
<213>  Unknown

<220>
<223>  Peptide

<400>  74

Leu Pro Tyr Asn Pro Thr Val Tyr Gly Arg Pro Ala Val Phe Asp
1               5                   10                  15


<210>  75
<211>  15
<212>  PRT
<213>  Unknown

<220>
<223>  Peptide

<400>  75

Val Tyr Gly Arg Pro Ala Val Phe Asp Arg Lys Ser Asp Ala Lys
1               5                   10                  15


<210>  76
<211>  15
<212>  PRT
<213>  Unknown

<220>
<223>  Peptide

<400>  76

His Arg Lys Lys Lys Ala Ala Arg Thr Glu Gln Pro Arg Ala Thr
1               5                   10                  15


<210>  77
<211>  15
<212>  PRT
<213>  Unknown

<220>
<223>  Peptide

<400>  77

Pro Gln Gln Pro Arg Ala Gly Arg Arg Gly Pro Cys Val Phe Thr
1               5                   10                  15


<210>  78
<211>  15
<212>  PRT
<213>  Unknown

<220>
<223>  Peptide

<400>  78

Ala Ala Pro Arg Gln Trp Pro Met Pro Leu Arg Pro Ile Pro Met
1               5                   10                  15


<210>  79
<211>  15
<212>  PRT
<213>  Unknown

<220>
<223>  Peptide

<400>  79

Arg Gln Trp Pro Met Pro Leu Arg Pro Ile Pro Met Arg Pro Leu
1               5                   10                  15


<210>  80
<211>  15
<212>  PRT
<213>  Unknown

<220>
<223>  Peptide

<400>  80

Leu Arg Pro Ile Pro Met Arg Pro Leu Arg Met Gln Pro Ile Pro
1               5                   10                  15


<210>  81
<211>  15
<212>  PRT
<213>  Unknown

<220>
<223>  Peptide

<400>  81

Ile Pro Met Arg Pro Leu Arg Met Gln Pro Ile Pro Phe Asn His

1               5                   10                  15


<210>  82
<211>  15
<212>  PRT
<213>  Unknown

<220>
<223>  Peptide

<400>  82

Arg Pro Leu Arg Met Gln Pro Ile Pro Phe Asn His Pro Val Gly
1               5                   10                  15


<210>  83
<211>  15
<212>  PRT
<213>  Unknown

<220>
<223>  Peptide

<400>  83

Pro Thr Trp Ala Gln Ile Gly His Ile Pro Tyr Gln Pro Thr Pro
1               5                   10                  15


<210>  84
<211>  15
<212>  PRT
<213>  Unknown

<220>
<223>  Peptide

<400>  84

Pro Ala Thr Met Gln Thr Pro Pro Arg Ala Pro Thr Pro Met Ser
1               5                   10                  15


<210>  85
<211>  15
<212>  PRT
<213>  Unknown

<220>
<223>  Peptide

<400>  85

Leu Lys Leu Gln Ala Ala Leu Glu Arg Gln Ala Ala Ala Gly Trp
1               5                   10                  15


<210>  86
<211>  15
<212>  PRT
<213>  Unknown

<220>
<223>  Peptide

<400>  86

Leu Gly Leu Gly Asp Ile Ala Val Ser Ser Pro Ser Ser Ser Glu
1               5                   10                  15


<210>  87
<211>  15
<212>  PRT
<213>  Unknown

<220>
<223>  Peptide

<400>  87

Trp Gly Gln Ser Arg Gly Asp Glu Asn Arg Gly Trp Met Gln Arg
1               5                   10                  15


<210>  88
<211>  15
<212>  PRT
<213>  Unknown

<220>
<223>  Peptide

<400>  88

Asp Glu Asn Arg Gly Trp Met Gln Arg Ile Arg Arg Arg Arg Arg
1               5                   10                  15


<210>  89
<211>  15
<212>  PRT
<213>  Unknown

<220>
<223>  Peptide

<400>  89

Ile Arg Arg Arg Arg Arg Arg Arg Ala Ala Leu Ser Gly His Leu
1               5                   10                  15


<210>  90
<211>  15
<212>  PRT
<213>  Unknown

<220>
<223>  Peptide

<400>  90

Arg Arg Arg Arg Arg Ala Ala Leu Ser Gly His Leu Leu Asp Thr

1               5                   10                  15


<210>  91
<211>  15
<212>  PRT
<213>  Unknown

<220>
<223>  Peptide

<400>  91

Leu Asp Thr Glu Asp Asn Val Pro Pro Trp Leu Pro Pro His Asp
1               5                   10                  15


<210>  92
<211>  15
<212>  PRT
<213>  Unknown

<220>
<223>  Peptide

<400>  92

Trp Leu Pro Pro His Asp Ile Thr Pro Tyr Thr Ala Arg Asn Ile
1               5                   10                  15


<210>  93
<211>  15
<212>  PRT
<213>  Unknown

<220>
<223>  Peptide

<400>  93

Ile Thr Pro Tyr Thr Ala Arg Asn Ile Arg Asp Ala Ala Cys Arg
1               5                   10                  15


<210>  94
<211>  15
<212>  PRT
<213>  Unknwon

<400>  94

Arg Asn Ile Arg Asp Ala Ala Cys Arg Ala Val Lys Gln Ser His
1               5                   10                  15


<210>  95
<211>  15
<212>  PRT
<213>  Unknown

<220>
<223>  Peptide

<400>  95

Val Met Ala Ala Arg Gln Arg Leu Gln Asp Ile Arg Arg Gly Pro
1               5                   10                  15


<210>  96
<211>  15
<212>  PRT
<213>  Unknown

<220>
<223>  Peptide

<400>  96

Leu Val Ala Glu Gly Gly Val Gly Trp Arg His Trp Leu Leu Thr
1               5                   10                  15


<210>  97
<211>  15
<212>  PRT
<213>  Unknown

<220>
<223>  Peptide

<400>  97

Glu Gly Gly Val Gly Trp Arg His Trp Leu Leu Thr Ser Pro Ser
1               5                   10                  15


<210>  98
<211>  15
<212>  PRT
<213>  Unknown

<220>
<223>  Peptide

<400>  98

Arg His Trp Leu Leu Thr Ser Pro Ser Gln Ser Trp Pro Met Gly
1               5                   10                  15


<210>  99
<211>  15
<212>  PRT
<213>  Unknown

<220>
<223>  Peptide

<400>  99

Gln Ser Trp Pro Met Gly Tyr Arg Thr Ala Thr Leu Arg Thr Leu
1               5                   10                  15

<210>  100
<211>  15
<212>  PRT
<213>  Unkown

<400>  100

Pro Met Gly Tyr Arg Thr Ala Thr Leu Arg Thr Leu Thr Pro Val
1               5                   10                  15


<210>  101
<211>  15
<212>  PRT
<213>  Unknown

<220>
<223>  Peptide

<400>  101

Tyr Arg Thr Ala Thr Leu Arg Thr Leu Thr Pro Val Pro Asn Arg
1               5                   10                  15


<210>  102
<211>  15
<212>  PRT
<213>  Unknown

<220>
<223>  Peptide

<400>  102

Ala Arg Glu Ala Glu Val Arg Phe Leu Arg Gly Lys Trp Gln Arg
1               5                   10                  15


<210>  103
<211>  15
<212>  PRT
<213>  Unknown

<220>
<223>  Peptide

<400>  103

His His Ile Trp Gln Asn Leu Leu Gln Thr Glu Glu Asn Leu Leu
1               5                   10                  15


<210>  104
<211>  15
<212>  PRT
<213>  Unknown

<220>
<223>  Peptide

<400>  104

Leu Leu Gln Thr Glu Glu Asn Leu Leu Asp Phe Val Arg Phe Met
1               5                   10                  15


<210>  105
<211>  15
<212>  PRT
<213>  Unknown

<220>
<223>  Peptide

<400>  105

Thr Glu Glu Asn Leu Leu Asp Phe Val Arg Phe Met Gly Val Met
1               5                   10                  15


<210>  106
<211>  15
<212>  PRT
<213>  Unknown

<220>
<223>  Peptide

<400>  106

Asn Leu Leu Asp Phe Val Arg Phe Met Gly Val Met Ser Ser Cys
1               5                   10                  15


<210>  107
<211>  15
<212>  PRT
<213>  Unknown

<220>
<223>  Peptide

<400>  107

Asp Phe Val Arg Phe Met Gly Val Met Ser Ser Cys Asn Asn Pro
1               5                   10                  15


<210>  108
<211>  15
<212>  PRT
<213>  Unknown

<220>
<223>  Peptide

<400>  108

Ser Ser Cys Asn Asn Pro Ala Val Asn Tyr Trp Phe His Lys Thr
1               5                   10                  15


<210>  109
<211>  15
<212>  PRT

<213>  Unknown

<220>
<223>  Peptide

<400>  109

Ala Val Asn Tyr Trp Phe His Lys Thr Ile Gly Asn Phe Lys Pro
1               5                   10                  15


<210>  110
<211>  15
<212>  PRT
<213>  Unknown

<220>
<223>  Peptide

<400>  110

Tyr Trp Phe His Lys Thr Ile Gly Asn Phe Lys Pro Tyr Tyr Pro
1               5                   10                  15


<210>  111
<211>  15
<212>  PRT
<213>  Unknown

<220>
<223>  Peptide

<400>  111

His Lys Thr Ile Gly Asn Phe Lys Pro Tyr Tyr Pro Trp Asn Ala
1               5                   10                  15


<210>  112
<211>  15
<212>  PRT
<213>  Unknown

<220>
<223>  Peptide

<400>  112

Phe Lys Pro Tyr Tyr Pro Trp Asn Ala Pro Pro Asn Glu Asn Pro
1               5                   10                  15


<210>  113
<211>  15
<212>  PRT
<213>  Unknown

<220>
<223>  Peptide

<400>  113

Arg Arg Gly Ile Lys Glu His Val Ile Gln Asn Ala Phe Arg Lys
1               5                   10                  15


<210>  114
<211>  15
<212>  PRT
<213>  Unknown

<220>
<223>  Peptide

<400>  114

Ile Lys Glu His Val Ile Gln Asn Ala Phe Arg Lys Ala Gln Ile
1               5                   10                  15


<210>  115
<211>  15
<212>  PRT
<213>  Unknown

<220>
<223>  Peptide

<400>  115

Gln Asn Ala Phe Arg Lys Ala Gln Ile Gln Gly Leu Ser Met Leu
1               5                   10                  15


<210>  116
<211>  15
<212>  PRT
<213>  Unknown

<220>
<223>  Peptide

<400>  116

Ser Ser Val Ser Gln Pro Asn Lys Pro His Arg Lys His Gln Asp
1               5                   10                  15


<210>  117
<211>  15
<212>  PRT
<213>  Unknown

<220>
<223>  Peptide

<400>  117

Gly Pro His Ile Val Thr Pro Pro Ser Ala Arg Pro Arg Ile Met
1               5                   10                  15


<210>  118
<211>  15
<212>  PRT

<213>  Unknown

<220>
<223>  Peptide

<400>  118

Ala Pro Pro Val Val Arg Met Phe Met Arg Glu Arg Gln Leu Pro
1               5                   10                  15


<210>  119
<211>  15
<212>  PRT
<213>  Unknown

<220>
<223>  Peptide

<400>  119

Met Phe Met Arg Glu Arg Gln Leu Pro Gln Ser Thr Gly Arg Lys
1               5                   10                  15


<210>  120
<211>  15
<212>  PRT
<213>  Unknown

<220>
<223>  Peptide

<400>  120

Phe Trp Glu Met Arg Ala Gly Arg Glu Ile Thr Gln Met Gln Gln
1               5                   10                  15


<210>  121
<211>  15
<212>  PRT
<213>  Unknown

<220>
<223>  Peptide

<400>  121

Pro Gly Tyr Ala Gly Pro Trp Thr Pro Arg Ser Gln His Pro Cys
1               5                   10                  15


<210>  122
<211>  15
<212>  PRT
<213>  Unknown

<220>
<223>  Peptide

<400>  122

Trp Thr Pro Arg Ser Gln His Pro Cys Tyr Arg His Pro Trp Ala
1               5                   10                  15


<210>  123
<211>  15
<212>  PRT
<213>  Unknown

<220>
<223>  Peptide

<400>  123

Arg Ser Gln His Pro Cys Tyr Arg His Pro Trp Ala Pro Trp Ser
1               5                   10                  15


<210>  124
<211>  15
<212>  PRT
<213>  Unknown

<220>
<223>  Peptide

<400>  124

Phe Pro His Leu Gln Ser Glu Thr Gly Pro Pro Arg Leu Gln Leu
1               5                   10                  15


<210>  125
<211>  15
<212>  PRT
<213>  Unknown

<220>
<223>  Peptide

<400>  125

Glu Thr Gly Pro Pro Arg Leu Gln Leu Ser Leu Val Pro Leu Val
1               5                   10                  15


<210>  126
<211>  15
<212>  PRT
<213>  Unknown

<220>
<223>  Peptide

<400>  126

Arg Ala Pro Ile Arg Pro Ile Pro Thr Arg Phe Pro Pro Pro Pro
1               5                   10                  15


<210>  127
<211>  15
<212>  PRT

<213>  Unknown

<220>
<223>  Peptide

<400>  127

Leu Leu Leu Leu Ala Leu Leu Phe Trp Leu Tyr Ile Val Met Ser
1               5                   10                  15


<210>  128
<211>  15
<212>  PRT
<213>  Unknown

<220>
<223>  Peptide

<400>  128

Leu Glu Met Leu Trp Arg Leu Gly Ala Thr Ile Trp Gln Leu Leu
1               5                   10                  15


<210>  129
<211>  15
<212>  PRT
<213>  Unknown

<220>
<223>  Peptide

<400>  129

Leu Ala Ile Leu Ile Trp Met Tyr Tyr His Gly Gln Arg His Ser
1               5                   10                  15


<210>  130
<211>  15
<212>  PRT
<213>  Unknown

<220>
<223>  Peptide

<400>  130

Leu Ile Trp Met Tyr Tyr His Gly Gln Arg His Ser Asp Glu His
1               5                   10                  15


<210>  131
<211>  15
<212>  PRT
<213>  Unknown

<220>
<223>  Peptide

<400>  131

Met Tyr Tyr His Gly Gln Arg His Ser Asp Glu His His His Asp
1               5                   10                  15

<210>  132
<211>  15
<212>  PRT
<213>  Unknown

<220>
<223>  Peptide

<400>  132

Met Ala Arg Arg Leu Pro Lys Pro Thr Leu Gln Gly Arg Leu Glu
1               5                   10                  15

<210>  133
<211>  15
<212>  PRT
<213>  Unknown

<220>
<223>  Peptide

<400>  133

Phe Cys Tyr Glu Glu Tyr Val Gln Arg Thr Phe Gly Val Pro Arg
1               5                   10                  15

<210>  134
<211>  15
<212>  PRT
<213>  Unknown

<220>
<223>  Peptide

<400>  134

Thr Phe Gly Val Pro Arg Arg Gln Arg Ala Ile Asp Lys Arg Gln
1               5                   10                  15

<210>  135
<211>  15
<212>  PRT
<213>  Unknown

<220>
<223>  Peptide

<400>  135

Arg Ala Ser Val Ala Gly Ala Gly Ala His Ala His Leu Gly Gly
1               5                   10                  15

## Description

### FIELD OF THE INVENTION

The present invention relates to the development of an in vitro method for diagnosing Chronic Fatigue Syndrome (CFS) by determining the presence, absence or the amount of at least one specific marker characteristic of an Epstein-Barr virus (EBV) infection in a sample obtained from the body of an individual. In addition, the invention relates to a device for the diagnosis of Chronic Fatigue Syndrome using at least one specific marker characteristic of Epstein-Barr virus.

### BACKGROUND

Chronic Fatigue Syndrome (CFS) is a complex disease characterized by overwhelming fatigue accompanied by physical symptoms resembling a severe flu-like illness, including muscle pain, impaired memory or mental concentration, insomnia, and post-exertion malaise. CFS can persist for years and is difficult to diagnose since no diagnostic tests are available. At present, CFS is diagnosed mainly according to the Fukuda-Criteria which were established in 1994. These guidelines are based on the fulfillment of two major criteria: chronic fatigue causing substantial reduction of occupational, personal and social activities, lasting more than 6 months and the exclusion of associated medical and psychiatric conditions (Fukuda et al. Ann. Intern. Med. 1994, 121: 953-959). In 2003, the Center of Disease Control published recommendations for use of the definition, standardization of classification instruments and study design to improve the identification of CFS in patients. Even though CFS has an estimated prevalence of 0,3 % in the general population and is relatively frequent, it is often not diagnosed correctly. So far, CFS is a purely clinical diagnosis, which can only be provided by experienced physicians. The distinction between CFS and other physical disorders, such as depression or burn-out, is often difficult. At present, there is neither a diagnostic marker nor approved drug to treat CFS.

Numerous viral or microbial pathogens have been considered as possible causes of CFS, since many patients report an acute onset of symptoms with an infectious disease (Prins, et al., 2006, Lancet, 367(9507):346-355). Several human viruses (e.g. Epstein Barr Virus (EBV), Human Herpes Virus (HHV), Xenotropic murine leukemia virus-related virus (XMRV), Enteroviruses) have been associated with CFS, but their pathogenic relationship to the syndrome has not been demonstrated conclusively (Frémont M et al., 2009, In Vivo, 23(2):209-13; Wallace HL, 1999, Clin. Diagn. Lab. Immunol., 6(2):216-23; Lombardi et al., 2009, Science, 326(5952):585-589; van Kuppeveld, 2010, BMJ, 25:340). In this regard, various attempts have been made to diagnose CFS using the connection to pathogens such as Herpes Viruses, however none of these have so far led to a satisfactory result.

In US patent 6,894,056 B2, it is disclosed that CFS may be detected by combining the steps of monitoring the patients for T-wave abnormalities with an electrocardiograph to document the cardiac pathology considered the basis for CFS and evaluating the patient for serologic evidence of EBV and HCMV infection by measuring antibodies to these viruses. For the EBV, the capsid antigen (VCA) IgM and total early antigens (EA, EA-D) were measured, to see if an active or persistent multiplication of this virus could be determined, which was possible in a subset of patients having cardiac pathology indicative of CFS. However, a direct relationship between EBV and CFS could not be observed as healthy control individuals also tested positive for these EBV-specific antibodies. Rather, the serologic evidence provided a basis for the differential treatment of CFS patients with antiviral medications.

Based on these studies, the same inventor continued the research, which was published as WO 2009/054957 A2. Again, this document relates to possible methods for diagnosing and treating chronic fatigue syndrome in different subclasses of patients. Here, patients were tested for the presence of serum antibodies to herpes viruses such as e.g. EBV (VCA, EA-D, IgM-antigen-viral capsid p18), human cytomegalovirus (HCMV) and human herpes virus 6 (HHV6). Patients being only seropositive for EBV represented a subclass of CFS patients, since other subgroups of patients were tested seropositive for only HCMV or HHV6. However, CFS patients were not compared to control groups, but rather subclassified to allow differential pharmacological treatment of CFS, so that while this publication indicates a possible connection between CFS and EBV, HHV6 or HCMV, it does not provide a method for diagnosing CFS. In fact, these studies imply that EBV may only be involved in a subset of CFS patients.

Thus despite this research into the possible causes of CFS and possible treatment approaches, there remains a need for a diagnostic marker or test to differentiate CFS from other physical disorders with similar symptoms. So far there is no diagnostic marker available that can distinguish between a healthy individual and an individual affected by CFS.

### SUMMARY OF THE INVENTION

The present invention relies on the comparison of samples from healthy individuals to samples from individuals suffering from CFS as diagnosed by experienced physicians. The inventors surprisingly found that in patients suffering from CFS, the immune response to several markers characteristic of EBV is different from that found in healthy individuals. Based on these differences, the present invention provides an *in vitro* method for diagnosing Chronic Fatigue Syndrome. The method of the invention comprises determining the presence, absence or the amount of at least one marker characteristic of Epstein-Barr virus (EBV) infection in a sample obtained from the body of an individual, wherein the presence of Chronic Fatigue Syndrome can be concluded from the presence, absence or the amount of the marker in the sample.

In the most preferred embodiment, the marker is selected from the group consisting of EBNA1 EBNA3, EBNA4, EBNA6, BZLF1, LMP1 and VP26. In one embodiment of the invention, the absence of one, two or all of the markers EBNA1, EBNA3, EBNA4 and EBNA6 in the sample indicates the presence of Chronic Fatigue Syndrome. In another embodiment, the presence of one or both markers BZLF1 and VP26 indicates the presence of Chronic Fatigue Syndrome.

The method of the invention also allows for a distinction between Chronic Fatigue Syndrome and a different physical disorder, such as depression or burnout syndrome, by determining the presence, absence or amount of at least one of the markers EBNA3, EBNA4, and EBNA6.

In the methods of the present invention, the presence, absence of the amount of the marker can be determined directly or indirectly by means of a suitable assay. Specifically, the assay can determine the presence, absence or amount of each marker directly by measuring the presence, absence or amount of the corresponding protein or a fragment thereof or the corresponding nucleotide encoding the protein, or indirectly by measuring the presence, absence of amount of an immune response to the corresponding protein or a fragment thereof.

In a preferred embodiment of the invention, the immune response is determined by an immunoassay. Specifically, the immunoassay can comprise at least one of the following protein fragments. The protein fragments can be between 4 and 70 amino acids in length from the specified regions of the following sequences: 80 - 105 of SEQ ID NO.: 1; amino acid 158 - 245 of SEO ID NO.: 1; amino acid 80 - 105 of SEO ID NO.: 2; amino acid 158 - 245 of SEQ ID NO.: 2; amino acid 80 - 105 of SEQ ID NO.: 3; amino acid 158 - 245 of SEQ ID NO.: 3; amino acid 479 - 555 of SEQ ID NO.: 4; amino acid 479 - 555 of SEQ ID NO.: 5; amino acid 479 - 555 of SEQ ID NO.: 6; amino acid 1 - 30 of SEQ ID NO.: 7; amino acid 76 - 110 of SEQ ID NO.: 7; amino acid 154 - 431 of SEQ ID NO.: 7; amino acid 489 - 548 of SEQ ID NO.: 7; amino acid 784 - 828 of SEQ ID NO.: 7; amino acid 1 - 30 of SEQ ID NO.: 8; amino acid 77 - 111 of SEQ ID NO.: 8; amino acid 155 - 432 of SEQ ID NO.: 8; amino acid 503 - 567 of SEQ ID NO.: 8; amino acid 803 - 828 of SEQ ID NO.: 8; amino acid 1 - 30 of SEQ ID NO.: 9; amino acid 77 - 111 of SEQ ID NO.: 9; amino acid 155 - 432 of SEQ ID NO.: 9; amino acid 503 - 567 of SEQ ID NO.: 9; amino acid 794 - 819 of SEQ ID NO.: 9; amino acid 161 - 204 of SEQ ID NO.: 10; amino acid 230 - 259 of SEQ ID NO.: 10; amino acid 290 - 351 of SEQ ID NO.: 10; amino acid 383 - 450 of SEQ ID NO.: 10; amino acid 524 - 549 of SEQ ID NO.: 10; amino acid 614 - 719 of SEQ ID NO.: 10; amino acid 790 - 815 of SEQ ID NO.: 10; amino acid 922 - 946 of SEQ ID NO.: 10; amino acid 161 - 204 of SEQ ID NO.: 11; amino acid 230 - 259 of SEQ ID NO.: 11; amino acid 290 - 351 of SEQ ID NO.: 11; amino acid 383 - 450 of SEQ ID NO.: 11; amino acid 524 - 549 of SEQ ID NO.: 11; amino acid 614 - 711 of SEQ ID NO.: 11; amino acid 782 - 807 of SEQ ID NO.: 11; amino acid 914 - 938 of SEQ ID NO.: 11; amino acid 161 - 204 of SEQ ID NO.: 12; amino acid 230 - 259 of SEQ ID NO.: 12; amino acid 290 - 351 of SEQ ID NO.: 12; amino acid 383 - 450 of SEQ ID NO.: 12; amino acid 524 - 549 of SEQ ID NO.: 12; amino acid 614 - 711 of SEQ ID NO.: 12; amino acid 782 - 807 of SEQ ID NO.: 12; amino acid 914 - 938 of SEQ ID NO.: 12; amino acid 53 - 204 of SEQ ID NO.: 13; amino acid 236 - 306 of SEQ ID NO.: 13; amino acid 519 - 575 of SEQ ID NO.: 13; amino acid 632 - 696 of SEQ ID NO.: 13; amino acid 855 - 980 of SEQ ID NO.: 13; amino acid 53 - 204 of SEQ ID NO.: 14; amino acid 236 - 306 of SEQ ID NO.: 14; amino acid 518 - 543 of SEQ ID NO.: 14; amino acid 605 - 669 of SEQ ID NO.: 14; amino acid 785 - 903 of SEQ ID NO.: 14; amino acid 53 - 204 of SEQ ID NO.: 15; amino acid 236 - 306 of SEQ ID NO.: 15; amino acid 518 - 543 of SEQ ID NO.: 15; amino acid 570 - 634 of SEQ ID NO.: 15; amino acid 802 - 920 of SEQ ID NO.: 15; amino acid 26 - 51 of SEQ ID NO.: 16; amino acid 122 - 147 of SEQ ID NO.: 16; amino acid 173 - 204 of SEQ ID NO.: 16; amino acid 26 - 51 of SEQ ID NO.: 17; amino acid 122 - 147 of SEQ ID NO.: 17; amino acid 173 - 204 of SEQ ID NO.: 17; amino acid 26 - 51 of SEQ ID NO.: 18; amino acid 122 - 147 of SEQ ID NO.: 18; amino acid 173 - 204 of SEQ ID NO.: 18; amino acid 26 - 51 of SEQ ID NO.: 19; amino acid 122 - 147 of SEQ ID NO.: 19; amino acid 173 - 204 of SEQ ID NO.: 19; amino acid 26 - 51 of SEQ ID NO.: 20; amino acid 122 - 147 of SEQ ID NO.: 20; amino acid 173 - 204 of SEQ ID NO.: 20; amino acid 1 - 21 of SEQ ID NO.: 21; or amino acid 50 - 99 of SEQ ID NO.: 21., or any one of SEQ ID NOs.:22-135.. In a preferred embodiment, the fragments have a length of 15 amino acids. In a further preferred embodiment, the fragments correspond to one or more of SEQ ID NOs.: 22-135. The sequences referred to above are summarized in Table 1 of the detailed description.

In one embodiment of the invention, an immunoassay is used to determine the presence, absence or amount of at least one marker, and the protein fragments are immobilized on a solid phase and brought into contact with the sample obtained from the body of the individual, to determine the immune response (antibody response) of the individual. In a preferred embodiment, the immune response to at least one marker which are selected from the group consisting of EBNA1, EBNA3, EBNA4, EBNA6, BZLF1, LMP1 and VP26 is determined.

In some embodiments of the method of the invention, the method further comprises determining the number of memory B cells of the individual. In this embodiment, the total number of memory B cells and the number of EBV-specific memory B cells can be determined, wherein a reduction in the number of EBV-specific memory B cells relative to the number of EBV specific memory B-cells from an EBV seropositive control group of healthy individuals indicates the presence of Chronic Fatigue Syndrome.

In another embodiment of the invention, the method further comprises determining the response of TNF-alpha or interferon gamma-producing memory T cells specific to EBV. In this embodiment a reduction of TNF-alpha or interferon gamma-producing memory T cells relative to the number of TNF-alpha or interferon gamma-producing memory T cells of an EBV seropositive control group of healthy individuals indicates the presence of Chronic Fatigue Syndrome.

In the methods of the invention, the sample obtained from the body of an individual is selected from the group consisting of a blood sample, a saliva sample, a urine sample, and a sample from a pharyngeal wash.

The present invention also encompasses a device for the diagnosis of Chronic Fatigue Syndrome, wherein the device comprises a solid phase having immobilized thereon a protein comprising or consisting of at least one marker characteristic of a Epstein-Barr virus (EBV) infection or a protein fragment thereof. In a preferred embodiment, the marker is selected from the group consisting of EBNA1, EBNA3, EBNA4, EBNA6, BZLF1, LMP1 and VP26.

Finally, the invention relates to a protein comprising or consisting at least one marker or protein fragment thereof for use in the diagnosis of Chronic Fatigue Syndrome, wherein the marker is selected from the group consisting of EBNA1 EBNA3, EBNA4, EBNA6, BZLF1, LMP1 and VP26.

In one embodiment of the invention, the preferred marker is EBNA3, EBNA4 or EBNA6 or a fragment thereof.

### BRIEF DESCRIPTION OF THE DRAWINGS

The invention will be better understood with reference to the detailed description when considered in conjunction with the non-limiting examples and the accompanying drawings, which are:

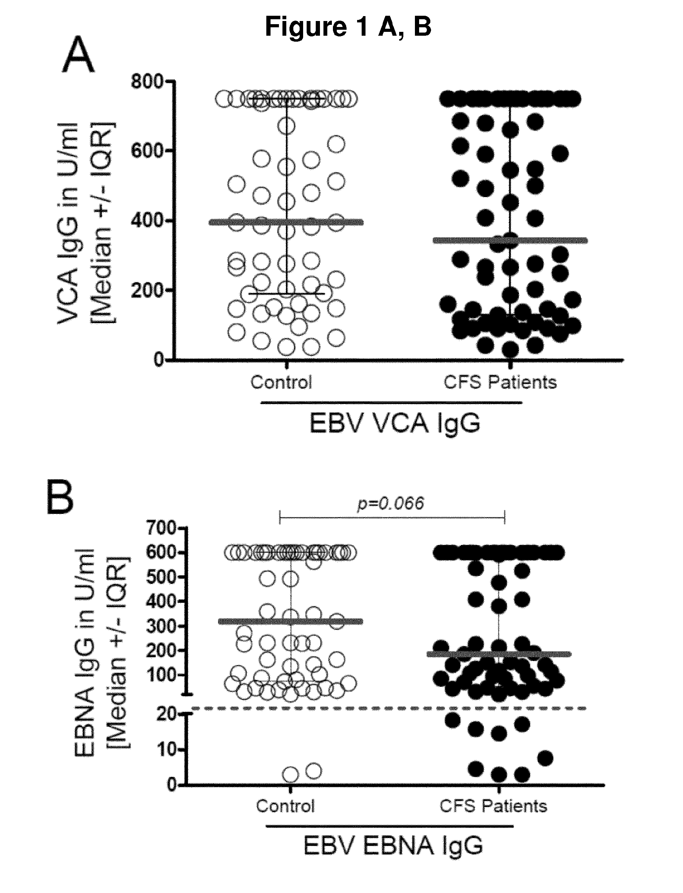

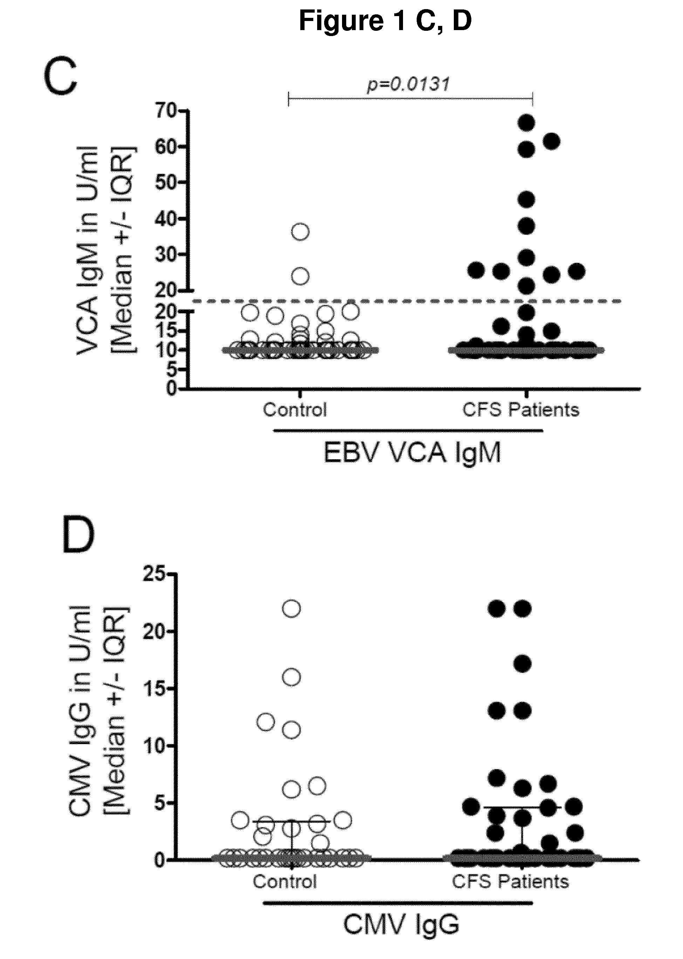

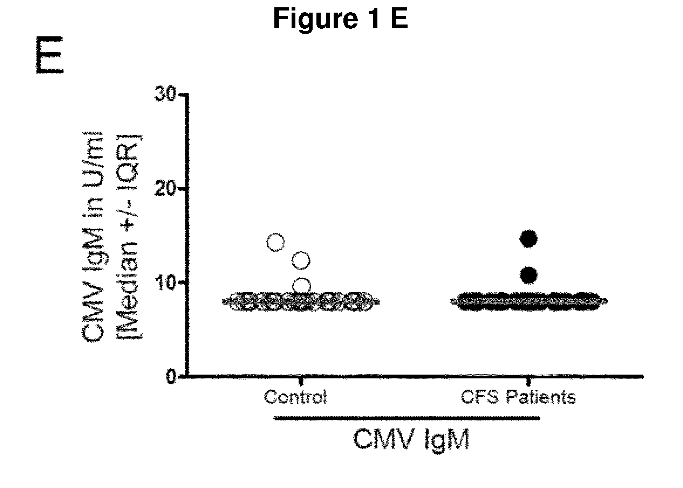

- **Figure 1:**: (A) Serum IgG titer were assessed for healthy donors (Control) and CFS patients by ELISA for EBV-VCA IgG (control n=57, CFS n=63),
(B) EBNA IgG (control n=57, CFS n=63),
(C) EBV-VCA IgM (control n=57, CFS n=63) and (D) CMV IgG (control n=32, CFS n=41) and (E) CMV IgM (control n=32, CFS n=41). Statistical analysis was performed using the two-tailed Mann-Whitney-U test and for EBNA IgG and EBV-VCA IgM Fisher's exact one-tailed test for association analysis with * p<0.05
- **Figure 2:**: (A) Serum IgG titer were assessed for CFS patients by ELISA for EBV-IgG and EBV-EBNA-1 IgG (n=397), (B) EBNA-1 negative (neg, n=7) and positive (pos, n=8) CFS patients from the cohort of A were compared for total IgG, and (C) the frequencies of CD19⁺ B-cells, (D) IgD⁺ IgM⁺ CD27⁺ marginal zone B-cells, and (D) IgD⁻ CD27⁺ class-switched memory B-cells. Statistical analysis was performed using the two-tailed Mann-Whitney-U test with *** p<0.001 and n.s. - not significant.

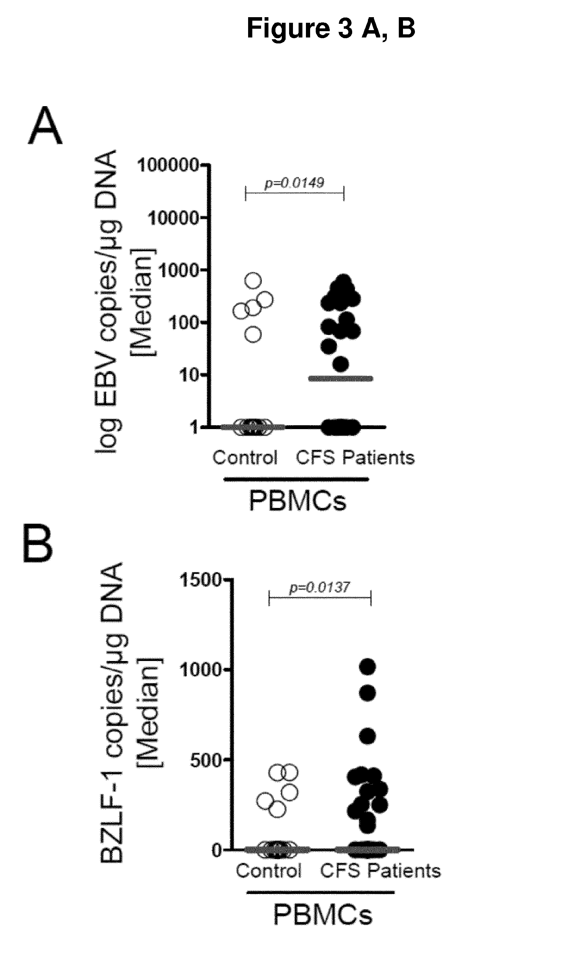

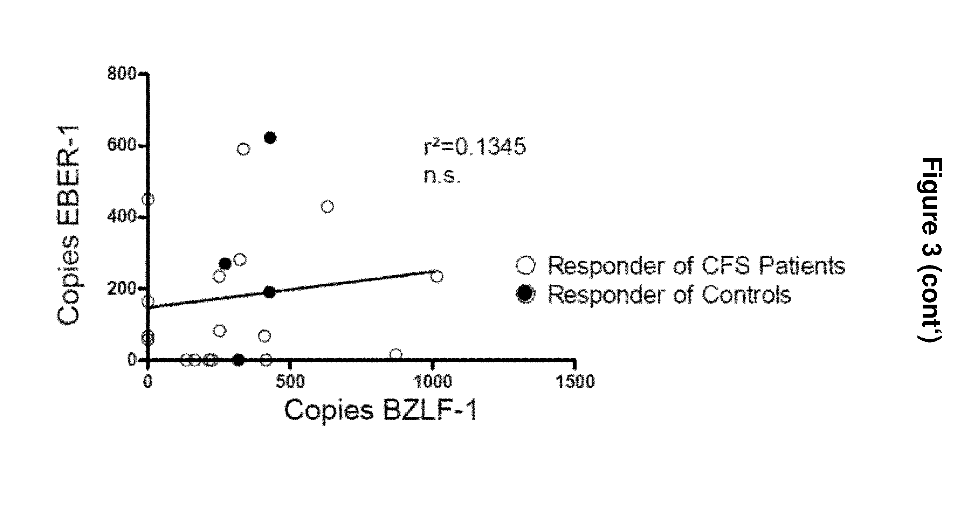

- **Figure 3:**: Replicating EBV can be detected in CFS patients. (A) EBV DNA was analyzed via nested real-time PCR in total PBMCs of healthy donors (Controls, n=29) and CFS patients (n=26) for EBER-1. (B) The same analysis as described in A was performed for EBV-BZLF-1 in PBMCs of CFS patients and healthy controls (n=27). Correlation of BZLF-1 and EBER-1 is displayed for responders of CFS patients (white circles) and responders of healthy controls (black circles) that showed detectable copy numbers of either BZLF-1 or EBER-1. EBER-1 and BZLF-1 copies were calculated in accordance to Namalwa standard. Statistical analysis was performed using the one-tailed Mann-Whitney-U test with * p<0.05. Correlation is displayed as linear regression analysis with n.s. - not significant.

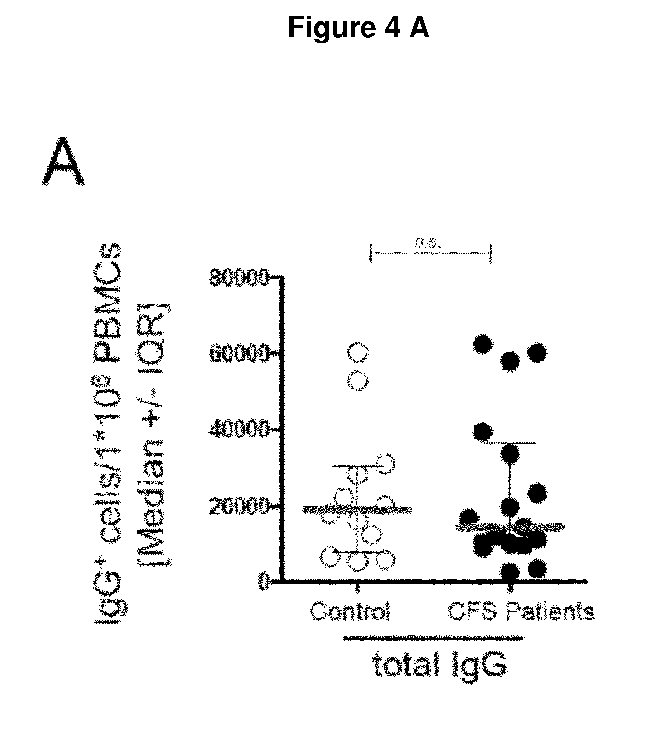

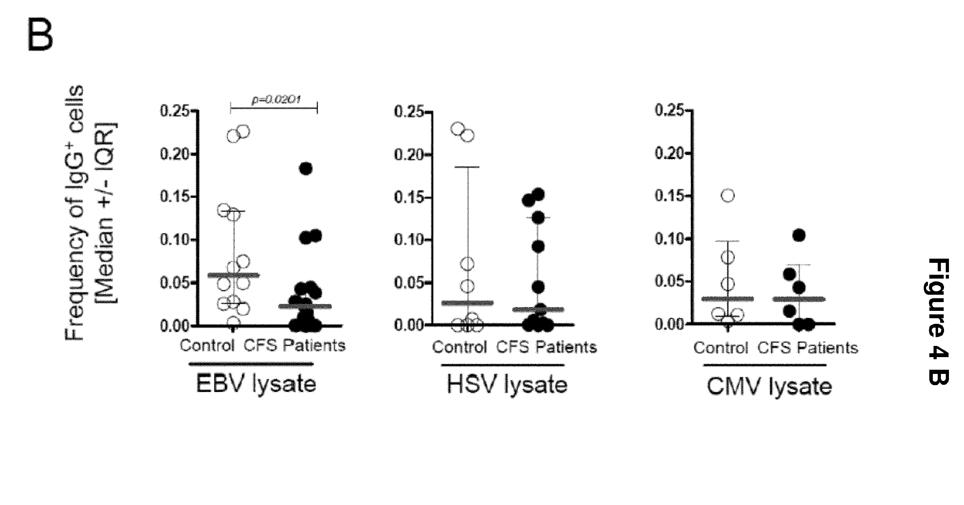

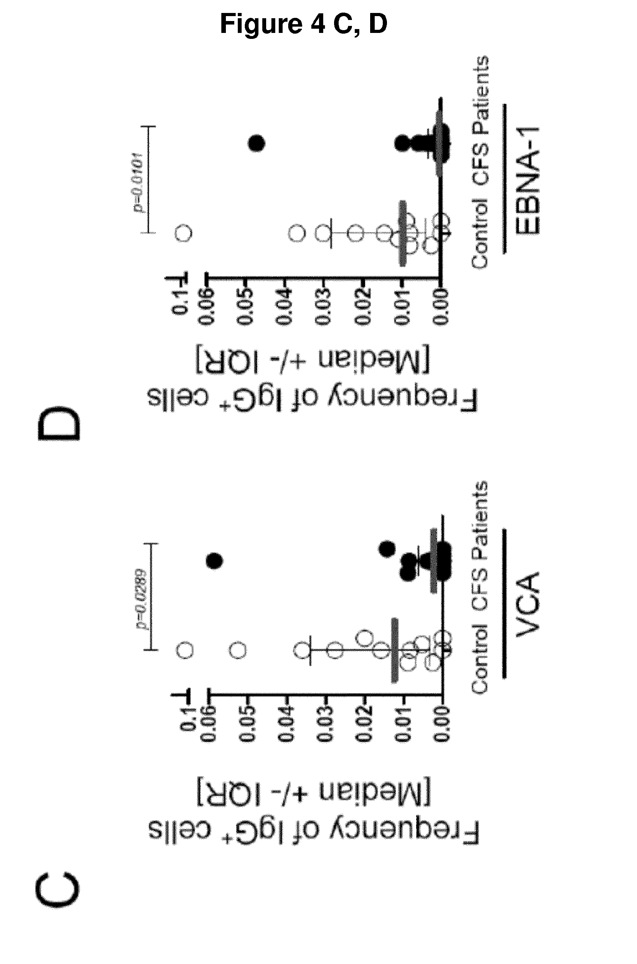

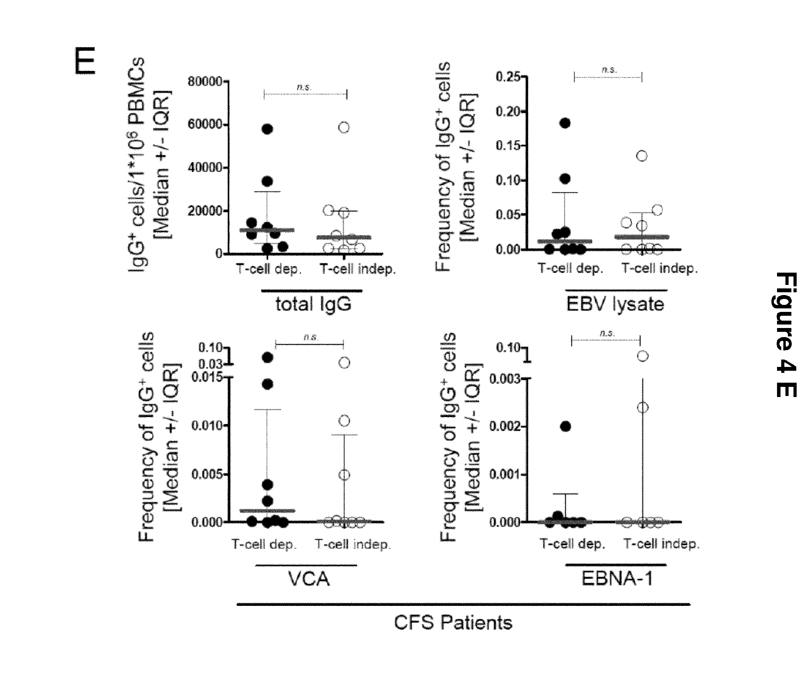

- **Figure 4:**: EBV specific memory B-cells are reduced in CFS patients. (A) Compared are the frequencies of EBV-specific memory B-cells between healthy donors (Controls) and CFS patients after 7 days of polyclonal stimulation. Secreted total IgG was assessed with the ELISpot assay after 6 h of incubation on a 96 well plate coated with anti-IgG (n=10), (B) EBV (n=10), HSV and CMV lysate (control n=6, CFS n=4), (C) VCA protein (n=10), and (D) EBNA-1 protein (n=10). IgG secreting B-cells are shown as frequencies from 1*10⁶ seeded cells. Statistical analysis was performed using the two-tailed Mann-Whitney-U test with * p<0.05, ** p<0.001 and n.s. - not significant.
- **Figure 5:**: CFS patients show diminished cytokine response against EBV. Whole blood of healthy donors (Control) and CFS patients was analyzed by CBA for (A) IFN-γ production after stimulation with either EBV lysate (control n=29, CFS n=22), EBNA-1 peptide (control n=24, CFS n=11) and SEB (control n=21, CFS n=11) in cell supernatants and (B) after EBV lysate stimulation for TNF-α (control n=29, CFS n=22), IL-2 (control n=29, CFS n=22) and IL-10 (control n=25, CFS n=13). Statistical analysis was performed using the two-tailed Mann-Whitney-U test with * p<0.05 and ** p<0.01.
- **Figure 6**: CFS patients show reduced EBV specific memory T-cell response. Comparison of cytokine producing CD8⁺ (upper panels) and CD4⁺ T-cells (lower panels) of CFS patients (n=15) and healthy donors (Controls, n=9)) after 10 days of antigen specific stimulation with EBNA-1. Cytokines were assessed by intracellular staining of isolated PBMCs after incubation with Brefeldin A for 16 h. Boolean gating strategy was applied to analyze (A) IFN-γ, TNF-α and IL-2 single, (B) IFN-γ/ TNF-α double and (C) IFN-γ/ TNF-α/ IL-2 triple cytokine producing T-cells. Statistical analysis was performed using the two-tailed Mann-Whitney-U test with * p<0.05 and n.s. - not significant.
- **Figure 7:**: Heatmap of BLRF2 in Normal, Depression and CFS patients. The x-axis represents the samples used in the screening experiment, the y axis shows peptides representing a scan through the respective protein with a peptide length of 15 amino acids and an overlap of 11 amino acids (N-terminus - bottom, C-terminus - top).
- **Figure 8:**: Heatmap of BZLF1 in Normal, Depression and CFS patients
- **Figure 9**: Heatmap of EBNA1 in Normal, Depression and CFS patients
- **Figure 10:**: Heatmap of EBNA3 in Normal, Depression and CFS patients
- **Figure 11:**: Heatmap of EBNA4 in Normal, Depression and CFS patients
- **Figure 12:**: Heatmap of EBNA6 in Normal, Depression and CFS patients
- **Figure 13:**: Heatmap of LMP1 in Normal, Depression and CFS patients
- **Figure 14:**: Heatmap of VP26 in Normal, Depression and CFS patients
- **Figure 15:**: Heatmap of signal intensities for selected peptides for all patients Selection criterion is a p-value < 0.01 for the signal intensities of CFS-patients vs. controls and CFS-patients vs. patients diagnosed with depression.
- **Figure 16A-16I:**: Boxplots of signal intensities for individual peptides fragments with a p-value < 0.01: CFS patients vs. Depression patients

### DETAILED DESCRIPTION OF THE INVENTION

As already mentioned above, the present invention relies on the comparison of samples from healthy individuals to samples from individuals suffering from CFS as diagnosed by experienced physicians. While former studies, such as those cited above, indicated a possible involvement of EBV, HHV6 and CMV in CFS and allowed for the classification of subgroups of CFS patients for differential treatment with antivirals, no significant differences between the immune response to specific antigens of these viruses (for EBV: VCA, EA-D, IgM-antigen-viral capsid p18) was found between CFS and healthy individuals.

Thus the technical problem of the present invention is to develop an *in vitro* method for diagnosing CFS in a sample obtained from the body of an individual. Surprisingly, contrary to previous findings, it was found that in patients suffering from CFS, the immune response to several markers characteristic of EBV is different from that found in healthy individuals and in individuals with other physical disorders such as depression. In the present application it was found that the analysis of specific antibodies and of cellular defects is suitable for the diagnosis of CFS. Especially, microarrays using protein fragments are excellently suitable for the diagnosis of CFS. Below, the experiments performed to arrive at the invention are summarized briefly after which each of the methods for diagnosing CFS of the present invention will be described in more detail. In this experiment, it was found that a subset of CFS patients shows abnormal EBV serology.

In a preliminary study, EBV-specific antibody titers (EBNA-1 IgG, VCA IgG and IgM), were determined and analysed via ELISA **(Example 1).** The most prominent finding was the deficiency of EBNA1-IgG antibodies (≤ 20 U/ml ) in 12,7 % of EBV-seropositive CFS patients, in contrast to 3,5% of healthy EBV-seropositive controls (p<0.01). In addition, it was found that while titers of VCA IgG were similar in CFS patients and controls, 17,5% of the CFS patients but only 3,5% of the controls had elevated VCA IgM levels. Thus Elevated EBV-VCA IgM and lack of EBNA IgG were detected in different patient subsets accounting for 30% of CFS patients with an abnormal EBV serology. As a comparison, HSV and CMV IgG were measured and were detected in all groups, revealing no difference between CFS patients and healthy controls. This finding led the inventors to focus on EBV. In order to determine if CFS-patients bear a higher EBV load than healthy subjects. In a further preliminary study, real-time PCR **(Example 2)** was used to detect viral transcripts in samples obtained from the body of individuals. However, it was found that this method did not allow the unambiguous differentiation of CFS patients from healthy individuals.

As mentioned above, EBV infects B-cells and remains latent in these cells after the acute infection has passed, so that EBV-specific memory B-cells are present in the host after the infection. This allowed for the investigation of EBV-specific memory B-cells after polyclonal stimulation in CFS-patients and healthy controls **(Example 3),** and the ELISASpot assay was used to enumerate IgG-producing cells. US patent application 2010/0267009 A1 describes an "ELISASpot" assay for differentiation between acute infections and latent or overcome infections, which can be EBV, that comprises incubating eukaryotic cells with an antigen and testing for cells secreting antigen-specific antibodies. In the Experiments described in Example 3, was found that EBV specific memory B-cells are low in number or absent in most CFS patients. No difference between CFS patients and healthy controls was detected for total IgG producing B-cells quantified by ELISpot assay. Remarkably, the in vitro differentiation of memory B cells into both EBNA-1 and VCA-antibody secreting plasma cells was strongly diminished in patients with CFS.

After this, Antigen-specific T-cell responses were measured in a subset of patients by cytokine production in culture supernatants **(Examples 4 and 5).** Here it was found that peripheral blood mononuclear cells (PBMCs) of patients with CFS produced significantly lower levels of TNF-α and IFN-γ in response to EBV lysate and certain EBV peptides. In vitro expanded EBNA-specific memory T cells had a significantly diminished fraction of multifunctional CD4 and CD8 T cells. US patent 7,897,357 B2 and US patent application 2010/0035282 A1 describe an assay for the detection of chemokines having activity that is upregulated by Th-1 cytokines (such IFN- γ) and chemokines that upregulate the activity of Th-1 cytokines (such as IFN-γ). The detection of the chemokine monokine induced by gamma interferon (MIG) provides a measure of the biological effect of IFN-γ rather than direct quantitation of IFN-γ or IFN-γ secreting cells per se.

As already mentioned above, both the ELISPOT described in US 2010/0267009 A1 as well as the EBV-T-cell assay (US patent 7,897,357 B2; and US patent application 2010/0035282 A1) are standard methods, which were developed for the detection of an infection-specific immune response. Other protein/peptide based techniques such as Luminex technology to detect antibodies or flow cytometry using biotin-labeled proteins or peptides to detect antibody-bearing memory B cells could also be used in this context. Taken together, the Experiments described above and in Examples 1-5 provide evidence for an exhausted B and T cell memory response against EBV in patients with CFS. This suggests a chronic low level of EBV replication in patients with CFS.

These findings prompted the inventors to test the samples from CFS patients, healthy volunteers and depression patients for an immune response to several specific EBV-antigens known to be characteristic for latent EBV infection, as well as several antigens characteristic for acute EBV infection by means of microarrays in order to identify specific markers for CFS **(Example 6).** The strongest markers for CFS identified are EBNA3, EBNA4 and EBNA6, though differences were also found in the immune response to BZLF1, EBNA1, LMP1 and VP26.

Based on these differences, the present invention provides an *in vitro* method for diagnosing Chronic Fatigue Syndrome. Specifically, three different tests for diagnosing CFS are provided. As summarized above, the primary method for diagnosing CFS involves determining the presence, absence or amount of at least one marker for EBV selected from the group of EBNA1, EBNA3, EBNA4, EBNA6, BZLF1, LMP1 and VP26. Of course this method also relates to determining the presence, absence or amount of two, three, four, five, six or all of these markers. After this, the presence, absence or amount of further markers for EBV can also be determined.

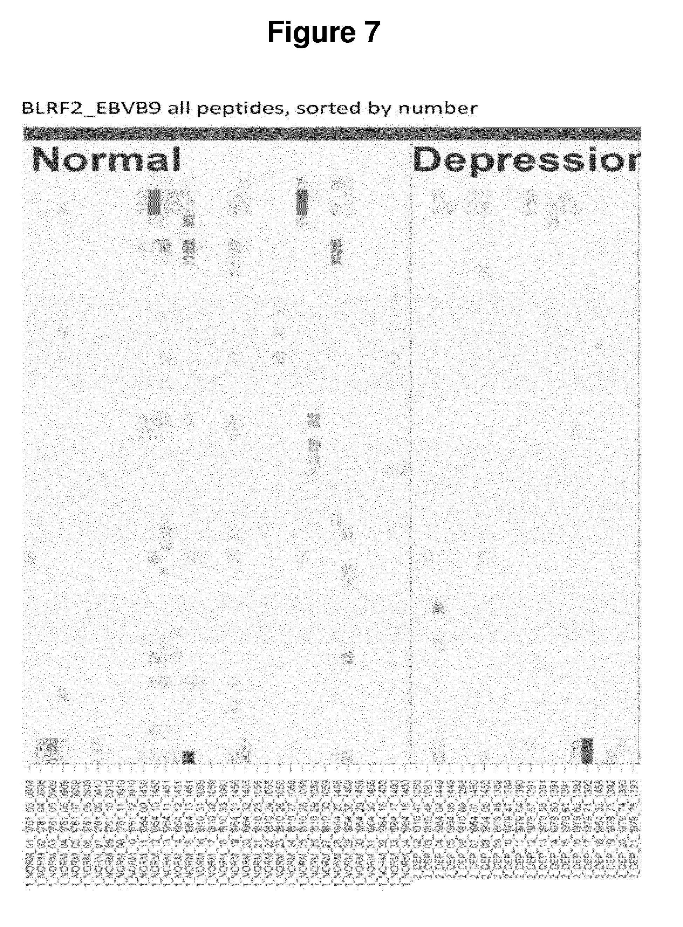

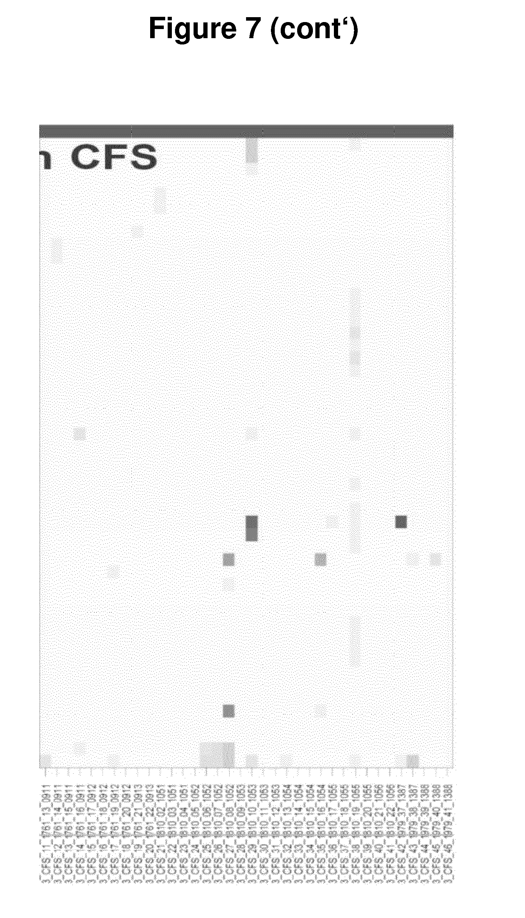

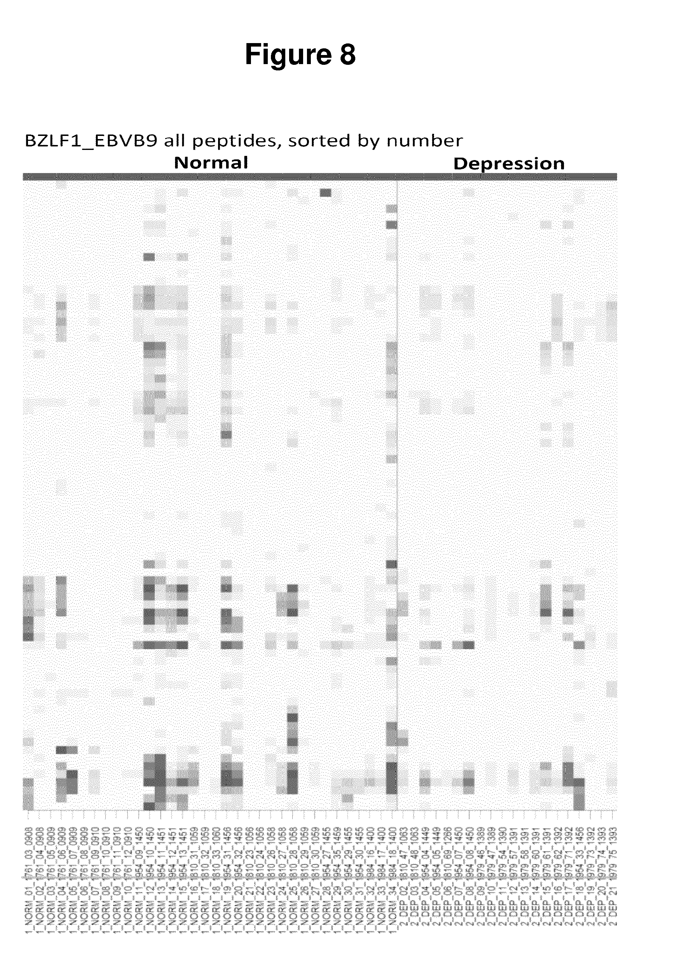

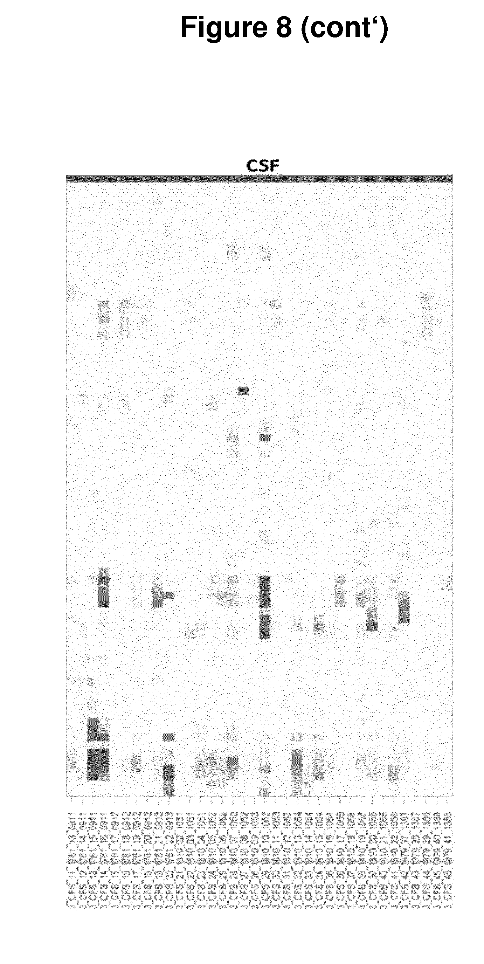

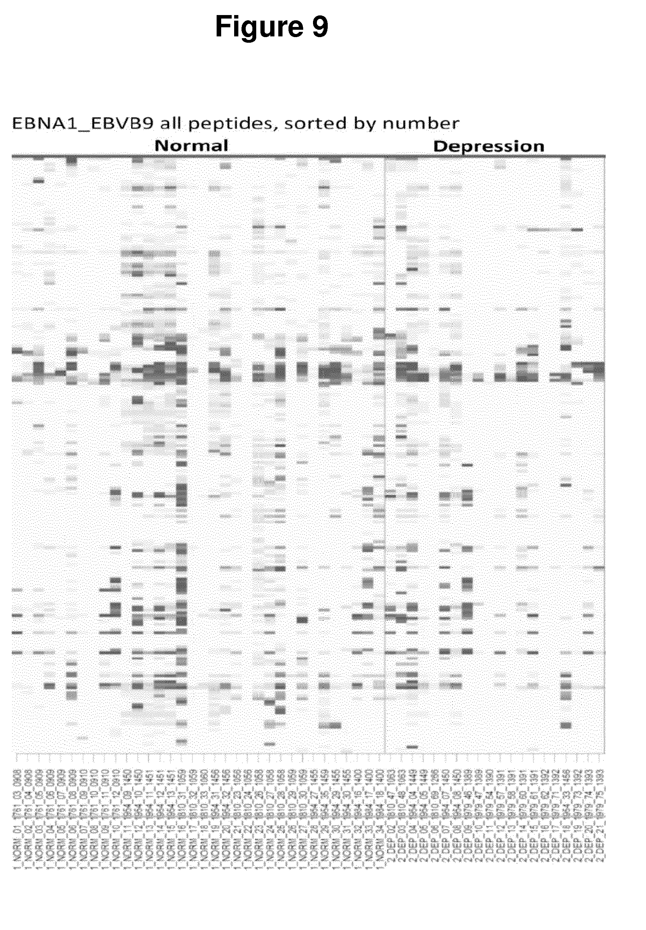

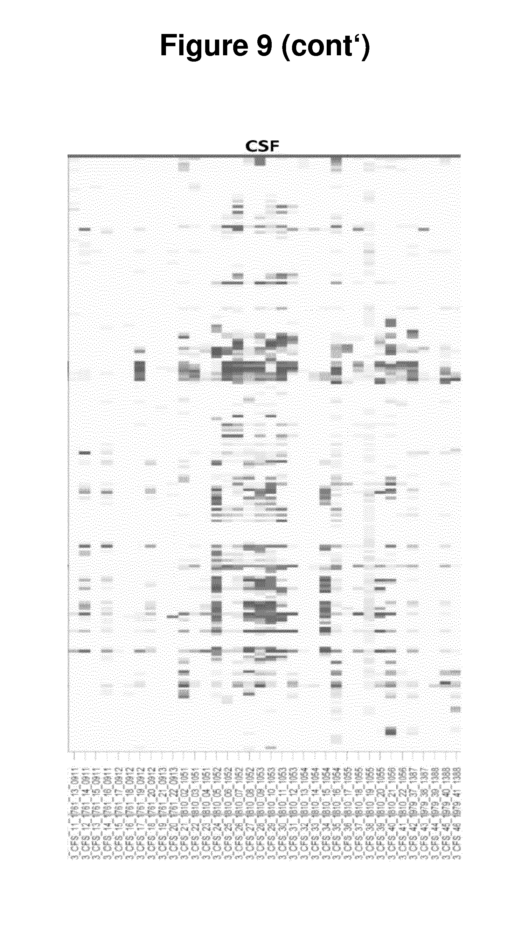

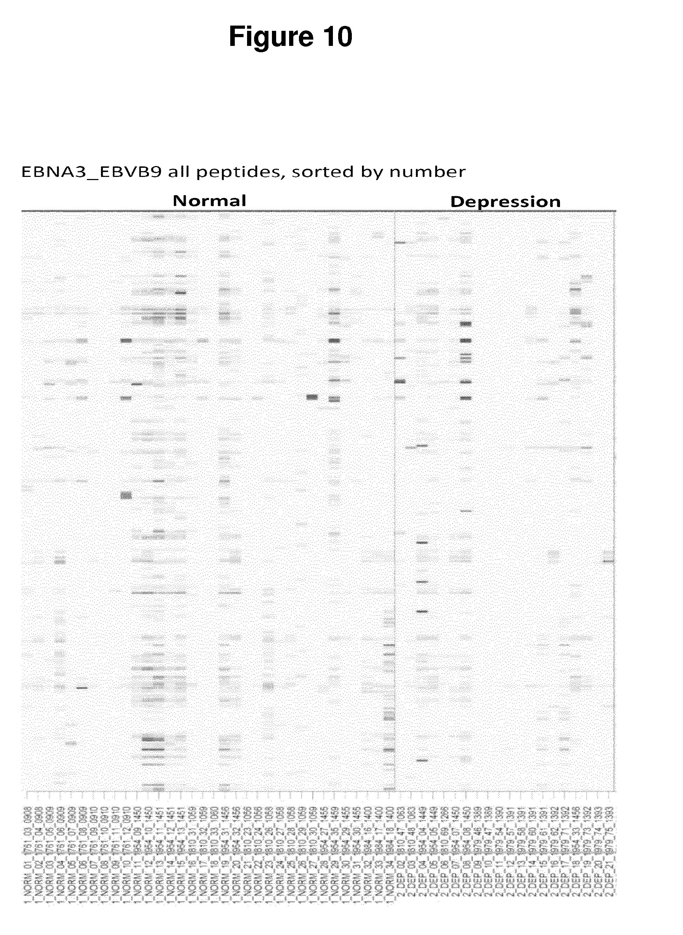

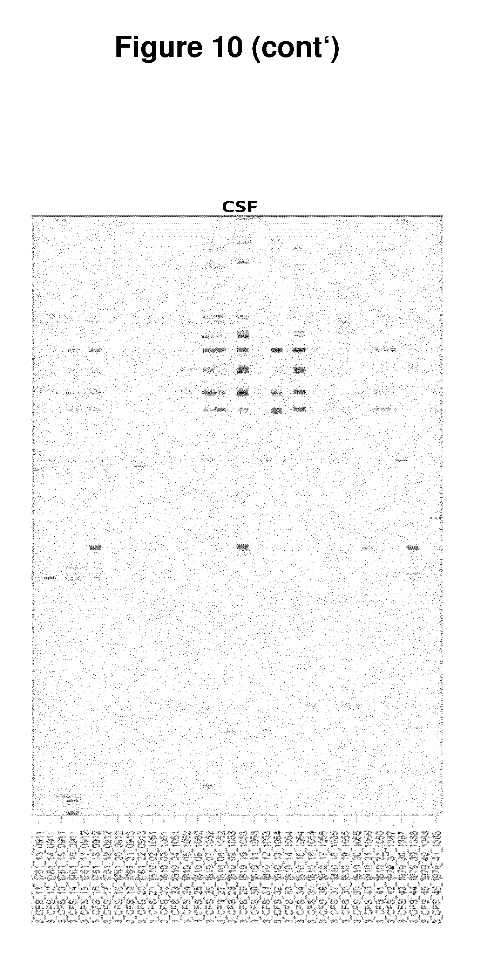

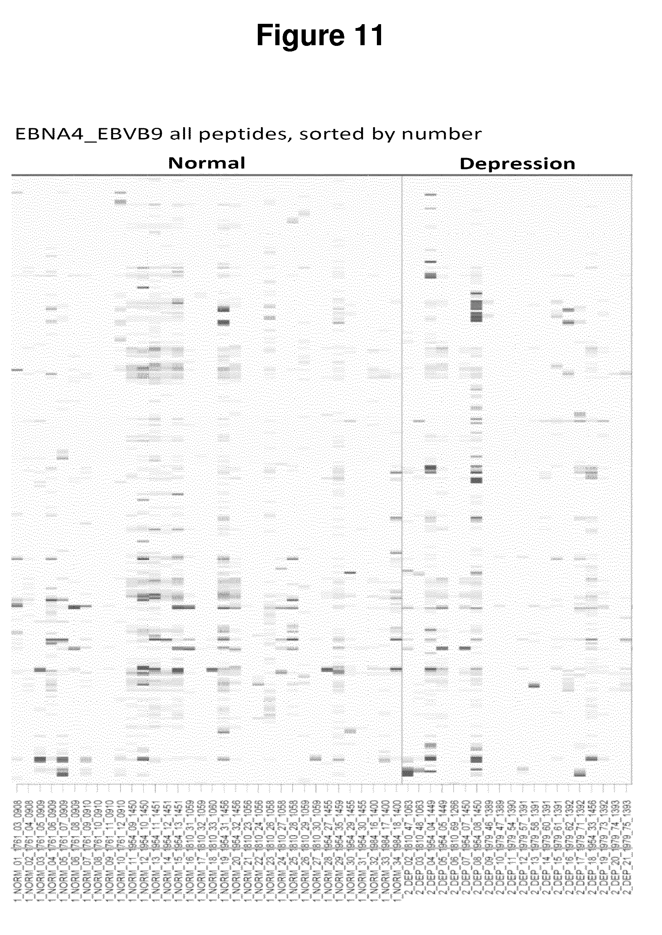

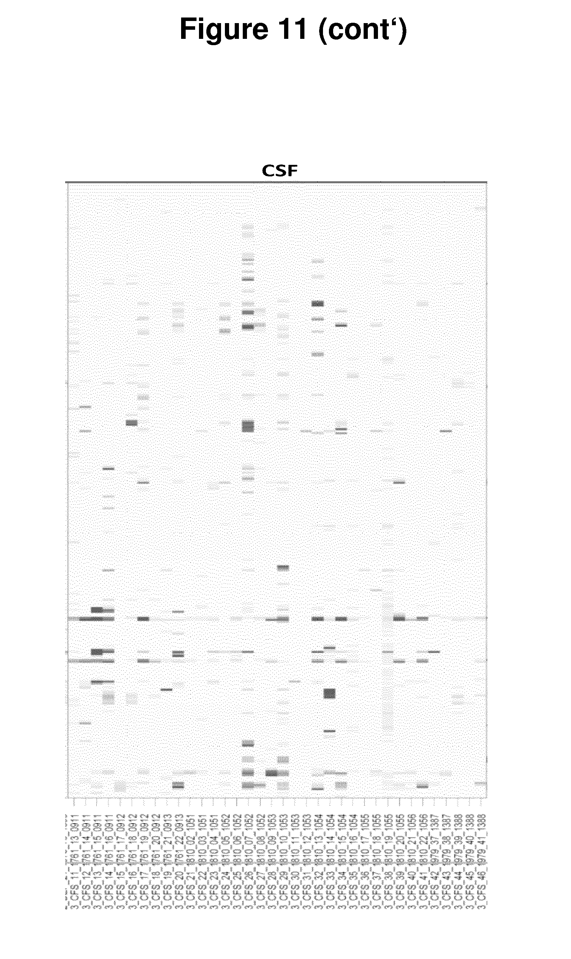

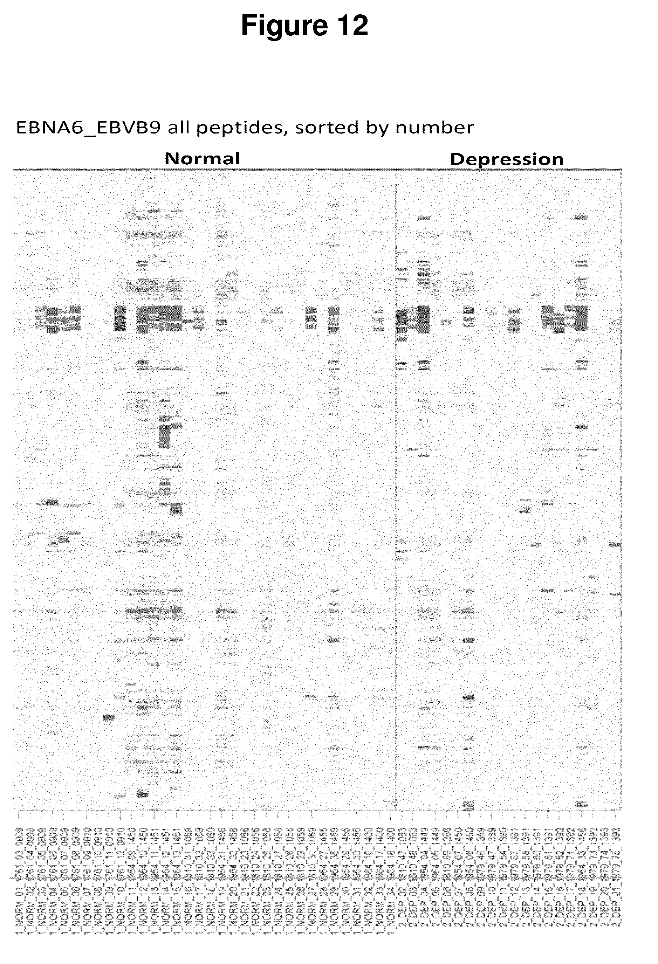

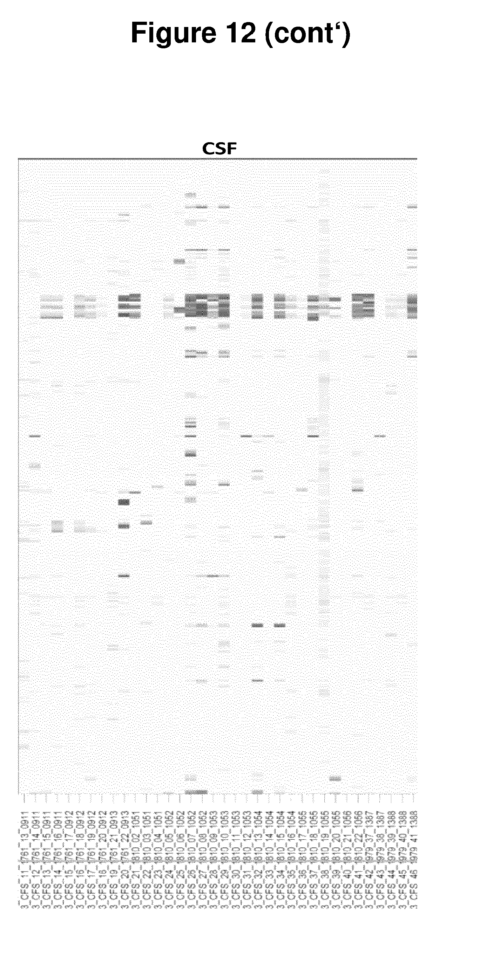

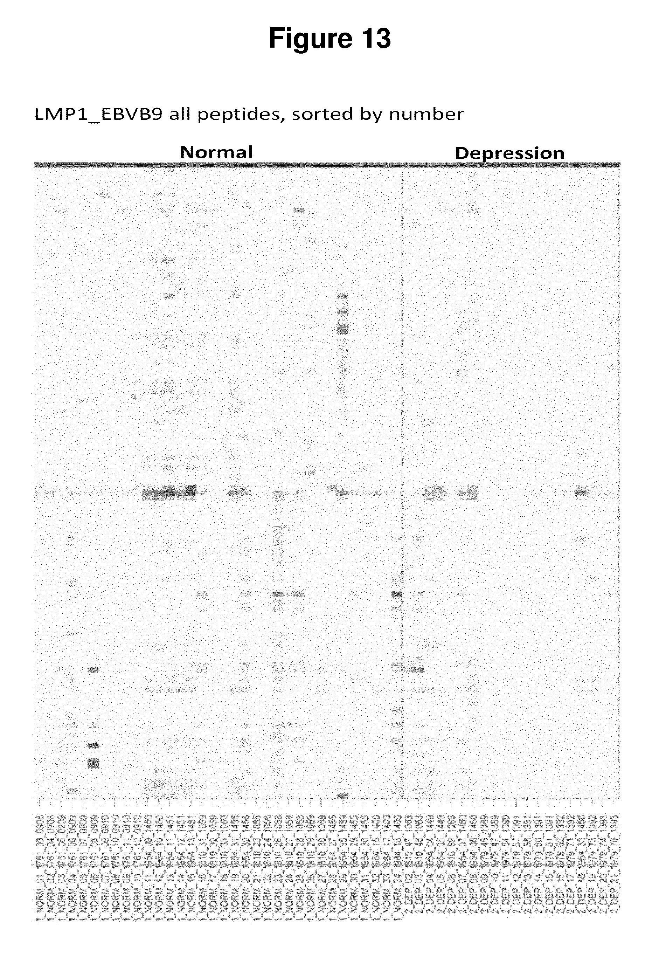

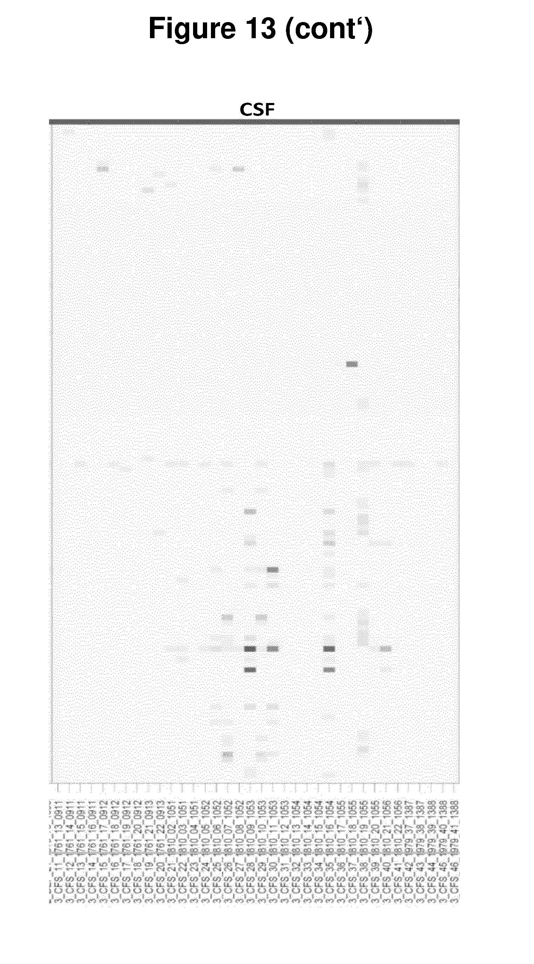

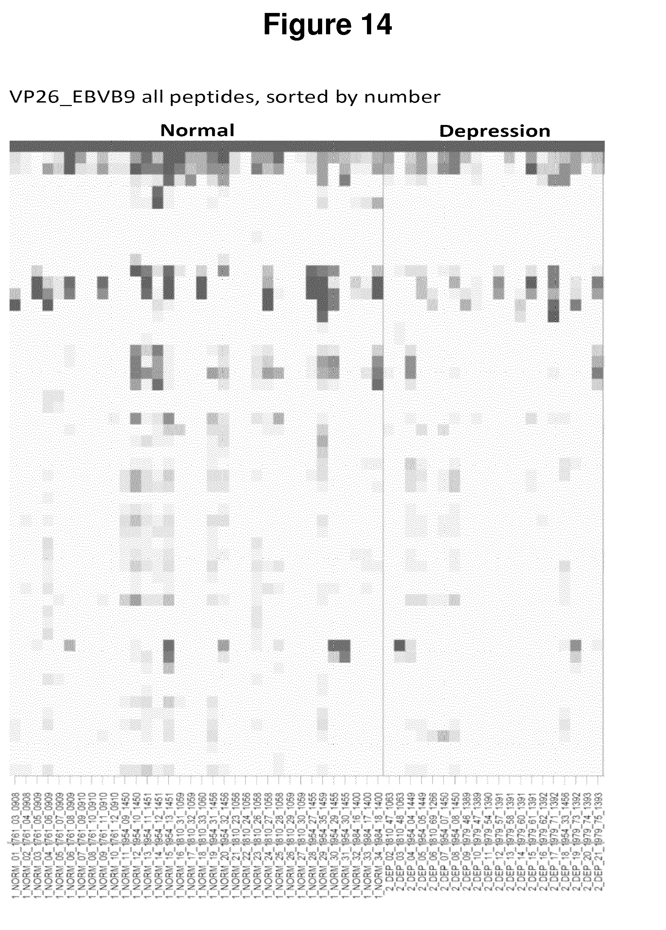

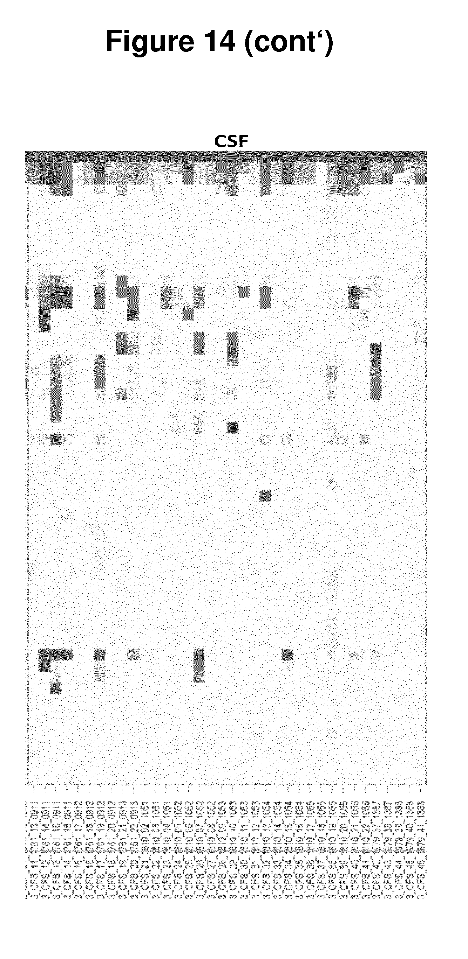

In this regard it should be noted that the presence, absence or amount of the marker can be determined by any assay suitable to detect the presence or absence of the marker directly or indirectly. The marker is measured "directly" by measuring the presence, absence or amount of the corresponding protein or a fragment thereof or the corresponding nucleotide encoding the protein in the sample. The marker can also be measured "indirectly" by measuring the presence, absence of amount of an immune response to the corresponding protein or a fragment thereof. In the methods described in **Example 6,** the absence of the markers of interest in CFS patients was determined indirectly using an immunoassay with fragments of the proteins of interest bound to microarrays. The microarray libraries were then incubated with the samples from patients with CFS, patients with depression and healthy volunteers as described in **Example 6.** The heatmaps of **Figures 7 to 15** show the distinct immune response (seroreactivity) of individuals with CFS as opposed to the healthy controls and the patients suffering from depression. This shows that the specific markers of the invention also allow for the differentiation between CFS and other physical disorders such as depression.

Specifically, in patients suffering from CFS, the immune response to at least one of EBNA1, EBNA3, EBNA4 or EBNA6 was absent or reduced. In some cases, the immune response to 2, 3 or all of these markers was absent or reduced. Thus the absence of an immune response to the full length protein or fragments of EBNA1, EBNA3, EBNA4 or EBNA6 (or to two, three or all of these) indicates that the patient is suffering from CFS. Likewise, the presence of an immune response to the full length protein or fragments of BZLF1 or VP26 is indicative of CFS.

Another sensitive approach to determine the B cell response against EBV is by analysing the number of EBV-specific B cells, which are severely reduced or absent in most patients with CFS. In a similar manner the memory T cell response against EBV is reduced in patients with CFS as outlined in detail below.

After this first step, the total number of memory B cells and the number of EBV-specific memory B cells is determined and compared to the number of EBV-specific memory B cells from a seropositive control group of healthy individuals. A reduction in the number of EBV-specific memory B cells is indicative of CFS.

Specifically, the ELISpot assay was used as a readout to analyze antigen-specific memory B-cells. It was demonstrated that CFS - patients show significantly reduced frequencies of EBV-specific memory B-cells after polyclonal stimulation. For two EBV-specific antigens used in these experiments (EBV-VCA protein and EBV-EBNA-1 protein) and EBV-lysate patients displayed a reduced response compared to healthy controls. Also the total amount of IgG secreting cell is similar in healthy controls and CFS-patients. This strongly suggests that overall humoral immune response in CFS-patients is not altered.

To assess whether the reduction of EBV-specific memory B-cells is due to T-cells with cytotoxic activity that recognize the MHC-EBV-peptide complex on the B-cells surface enriched B-cells were cultivated without T-cells. Frequencies of EBV-specific memory B-cells resulting from this stimulation are comparable to the results obtained with polyclonal stimulation. Even though frequencies detected after stimulation were higher, it has to be considered that enriched B-cells were used for this protocol. Overall same frequencies were found for EBV-lysate specific B-cell as for the polyclonal stimulation. These findings suggest that disruption of memory B-cells by T-cells in the course of differentiation is unlikely. Higher frequencies would have been expected for the stimulation with soluble CD40 ligand, since this stimulation is independent of donor-derived T-cells.

A further method for diagnosing EBV involves determining the response of TNF-alpha or interferon gamma-producing memory T cells, wherein the reduction of TNF-alpha or interferon gamma-producing memory T cells of an EBV seropositive control group relative to the number of TNF-alpha or interferon gamma-producing memory T cells of healthy individuals indicates the presence of Chronic Fatigue Syndrome. This method can be performed as described in the prior art mentioned above or according to Example 4. In this regard it was found that the PBMCs of patients with CFS produced significantly lower levels of TNFα and IFNγ in response to EBV lysate and certain EBV peptides. In vitro expanded EBNA-specific memory T cells had a significantly diminished fraction of multifunctional CD4 and CD8 T cells.

This "exhausted" T-cell response is often observed in HIV and HCV infection (Rajziewicz et al., 2007, J. Virol. 81: 2545-2553). It has been studied thoroughly in LCMV- infection in mice and mechanisms for the underlying causes have been proposed. T-cell dysfunction is controlled by cytokines and negative regulatory receptors. It is proposed that persistence and continuous exposure to antigen hyperimmune activation together with several other factors (i.e. presence of IL-10 and upregulation of immune-suppressor molecules) gradually drives T cells into exhaustion (EI-Far et al., 2008, Curr. HIV/AIDS Rep. 5: 13-19). Cellular exhaustion as a consequence of persistent viral stimulus in CFS-patients has been described in the past though only IFNγ was assessed (Klimas et al., 1990, J Clin Microbiol 28: 1403-1410; Visser et al., 1998, J. Infect. Dis., 177: 451-454).

In a further embodiment, the invention relates to a device for the diagnosis of CFS comprising a solid phase comprising at least one marker for EBV or a fragment thereof immobilized on it. In a preferred embodiment, the marker for EBV selected from the group of EBNA1, EBNA3, EBNA4, EBNA6, BZLF1, LMP1 and VP26. In the most preferred embodiment, at least one of the markers is EBNA3, EBNA4 or EBNA6 or a fragment thereof. Suitable fragments are described below, most preferable are those provided in SEQ ID NOs.:22-135. The device of the invention can be any solid phase test device such as a microarray, an immunoassay such as an ELISA plate, or a polymer bead, to which the fragments can be attached by methods known in the art, for example following the instructions published elsewhere (Barouch et al., (2013) J Infect Dis 207: 248-256).

These embodiments are characterized and described herein, illustrated in the Examples, and reflected in the claims. In the following section, the terms used in the claims and in the description are defined.

In the context of the present invention, the term *"in vitro* method" refers to a method that is performed outside of the human body, in contrast to an in vivo method. The method of the present invention is a diagnostic assay, so that the word "method" could be replaced with "test" or "assay".

The aim of the *in vitro* method of the invention is to diagnose Chronic Fatigue Syndrome. The expression "diagnose" means identifying CFS in a patient based on the methods of the invention.

As already mentioned above, the term "Chronic Fatigue Syndrome" (CFS) refers to a clinical condition of uncertain cause that is characterized by overwhelming fatigue, as diagnosed mainly according to the Fukuda-Criteria which were established in 1994. These guidelines are based on the fulfillment of two major criteria: chronic fatigue causing substantial reduction of occupational, personal and social activities, lasting more than 6 months and the exclusion of associated medical and psychiatric conditions (Fukuda et al. Ann. Intern. Med. 1994, 121: 953-959).

The term "determining the presence, absence or amount" reflects the use of the method to investigate the presence or absence of the marker and, if present, determining the amount thereof. The presence, absence or amount of the marker can be determined by an assay suitable to detect the presence or absence of the marker directly or indirectly, such as a peptide microarray, ELISA, Luminex or Luminex-like platforms, ELISPOT assay for antibody and B cell responses and specific cytokine release in response to the marker assessed by ELISA or flow cytometry for T cell responses both ex vivo or after in vitro expansion.

The marker is measured "directly" by measuring the presence, absence or amount of the corresponding protein or a fragment thereof or the corresponding nucleotide encoding the protein in the sample. The marker can also be measured indirectly by measuring the presence, absence of amount of an immune response to the corresponding protein or a fragment thereof. Specific fragments are provided in SEQ ID Nos.: 22-135 and other suitable fragments of SEQ ID Nos.: 1-21 are defined below. Any other protein/peptide based technique, such as Luminex technology, or flow cytometry using biotin-labeled proteins or peptides to detect antibody-bearing memory B cells can also be used to detect antibodies. Likewise the absence of a marker can also refer to the absence of an indirect measurement to the marker, such as the absence of an immune response to a fragment of the marker.

In the context of the present invention, the term "marker" refers to a protein found in a sample from the body of an individual that is characteristic of a certain condition or an immune response directed towards this protein or a fragment thereof. Thus a "marker characteristic of Epstein-Barr virus infection" is a protein that is known to be expressed in either acute or latent Epstein-Barr syndrome and that can therefore be identified in a sample from the body of an individual suffering from Epstein-Barr syndrome. The markers of particular interest of the present invention are EBNA3, EBNA4, EBNA6, EBNA1, LMP-1, BZLF1 and VP26, having the respective protein sequences of SEQ ID NOs.:1-21. Most preferably, the markers of the present invention are selected from EBNA1, EBNA4 and EBNA6. These markers can have an amino acid sequence corresponding to any one of SEQ ID NOs.:4, 5, 6, 10, 11, 12, 13, 14 or 15. The identity of these sequences is summarized in Table 1 below.

In the context of the invention, a "protein fragment" is a fragment of one of the markers of the invention mentioned above. The term "protein fragment" could be replaced with the term "peptide" or "polypeptide fragment". A protein fragment according to the invention can be any fragment of any one of SEQ ID NOs.: 1-21. In a preferred embodiment, the protein fragment is between 4 and 70 amino acids, between 4 and 25 amino acids or between 4 and 15 amino acids in length from the specified regions of the following sequences: amino acid 80 - 105 of SEQ ID NO.: 1; amino acid 158 - 245 of SEQ ID NO.: 1; amino acid 80 - 105 of SEQ ID NO.: 2; amino acid 158 - 245 of SEQ ID NO.: 2; amino acid 80 - 105 of SEQ ID NO.: 3; amino acid 158 - 245 of SEQ ID NO.: 3; amino acid 479 - 555 of SEQ ID NO.: 4; amino acid 479 - 555 of SEQ ID NO.: 5; amino acid 479 - 555 of SEQ ID NO.: 6; amino acid 1 - 30 of SEQ ID NO.: 7; amino acid 76 - 110 of SEQ ID NO.: 7; amino acid 154 - 431 of SEQ ID NO.: 7; amino acid 489 - 548 of SEQ ID NO.: 7; amino acid 784 - 828 of SEQ ID NO.: 7; amino acid 1 - 30 of SEQ ID NO.: 8; amino acid 77 - 111 of SEQ ID NO.: 8; amino acid 155 - 432 of SEQ ID NO.: 8; amino acid 503 - 567 of SEQ ID NO.: 8; amino acid 803 - 828 of SEQ ID NO.: 8; amino acid 1 - 30 of SEQ ID NO.: 9; amino acid 77 - 111 of SEQ ID NO.: 9; amino acid 155 - 432 of SEQ ID NO.: 9; amino acid 503 - 567 of SEQ ID NO.: 9; amino acid 794 - 819 of SEQ ID NO.: 9; amino acid 161 - 204 of SEQ ID NO.: 10; amino acid 230 - 259 of SEQ ID NO.: 10; amino acid 290 - 351 of SEQ ID NO.: 10; amino acid 383 - 450 of SEQ ID NO.: 10; amino acid 524 - 549 of SEQ ID NO.: 10; amino acid 614 - 719 of SEQ ID NO.: 10; amino acid 790 - 815 of SEQ ID NO.: 10; amino acid 922 - 946 of SEQ ID NO.: 10; amino acid 161 - 204 of SEQ ID NO.: 11; amino acid 230 - 259 of SEQ ID NO.: 11; amino acid 290 - 351 of SEQ ID NO.: 11; amino acid 383 - 450 of SEQ ID NO.: 11; amino acid 524 - 549 of SEQ ID NO.: 11; amino acid 614 - 711 of SEQ ID NO.: 11; amino acid 782 - 807 of SEQ ID NO.: 11; amino acid 914 - 938 of SEQ ID NO.: 11; amino acid 161 - 204 of SEQ ID NO.: 12; amino acid 230 - 259 of SEQ ID NO.: 12; amino acid 290 - 351 of SEQ ID NO.: 12; amino acid 383 - 450 of SEQ ID NO.: 12; amino acid 524 - 549 of SEQ ID NO.: 12; amino acid 614 - 711 of SEQ ID NO.: 12; amino acid 782 - 807 of SEQ ID NO.: 12; amino acid 914 - 938 of SEQ ID NO.: 12; amino acid 53 - 204 of SEQ ID NO.: 13; amino acid 236 - 306 of SEQ ID NO.: 13; amino acid 519 - 575 of SEQ ID NO.: 13; amino acid 632 - 696 of SEQ ID NO.: 13; amino acid 855 - 980 of SEQ ID NO.: 13; amino acid 53 - 204 of SEQ ID NO.: 14; amino acid 236 - 306 of SEQ ID NO.: 14; amino acid 518 - 543 of SEQ ID NO.: 14; amino acid 605 - 669 of SEQ ID NO.: 14; amino acid 785 - 903 of SEQ ID NO.: 14; amino acid 53 - 204 of SEQ ID NO.: 15; amino acid 236 - 306 of SEQ ID NO.: 15; amino acid 518 - 543 of SEQ ID NO.: 15; amino acid 570 - 634 of SEQ ID NO.: 15; amino acid 802 - 920 of SEQ ID NO.: 15; amino acid 26 - 51 of SEQ ID NO.: 16; amino acid 122 - 147 of SEQ ID NO.: 16; amino acid 173 - 204 of SEQ ID NO.: 16; amino acid 26 - 51 of SEQ ID NO.: 17; amino acid 122 - 147 of SEQ ID NO.: 17; amino acid 173 - 204 of SEQ ID NO.: 17; amino acid 26 - 51 of SEQ ID NO.: 18; amino acid 122 - 147 of SEQ ID NO.: 18; amino acid 173 - 204 of SEQ ID NO.: 18; amino acid 26 - 51 of SEQ ID NO.: 19; amino acid 122 - 147 of SEQ ID NO.: 19; amino acid 173 - 204 of SEQ ID NO.: 19; amino acid 26 - 51 of SEQ ID NO.: 20; amino acid 122 - 147 of SEQ ID NO.: 20; amino acid 173 - 204 of SEQ ID NO.: 20; amino acid 1 - 21 of SEQ ID NO.: 21; or amino acid 50 - 99 of SEQ ID NO.: 21.

In one embodiment, the fragments can be 5, 6, 7, 8, 9, 10, 11, 12, 13, 14, 15, 16, 17, 18, 19, 20, 21, 22, 23, 24, or 25 amino acids in length. In a preferred embodiment, the fragments are 15 amino acids. In a further preferred embodiment, the fragments correspond to one or more of SEQ ID NOs.: 22-135. The above fragments can of course be part of a larger construct or vector and may have various additional modifications known in the art such as linkers or labels. The sequences referred to above are summarized in Table 1 below.

| | |
|---|---|
| SEQ ID NO: 1 | BZLF1 Strain AG876 |
| SEQ ID NO: 2 | BZLF1 Strain B95-8 |
| SEQ ID NO: 3 | BZLF1 Strain GD1 |
| SEQ ID NO: 4 | EBNA1 Strain AG876 |
| SEQ ID NO: 5 | EBNA1 Strain B95-8 |
| SEQ ID NO: 6 | EBNA1 Strain GD1 |
| SEQ ID NO: 7 | EBNA3 Strain AG876 |
| SEQ ID NO: 8 | EBNA3 Strain B95-8 |
| SEQ ID NO: 9 | EBNA3 Strain GD1 |
| SEQ ID NO: 10 | EBNA4 Strain AG876 |
| SEQ ID NO: 11 | EBNA4 Strain B95-8 |
| SEQ ID NO: 12 | EBNA4 Strain GD1 |
| SEQ ID NO: 13 | EBNA6 Strain AG876 |
| SEQ ID NO: 14 | EBNA6 Strain B95-8 |
| SEQ ID NO: 15 | EBNA6 Strain GD1 |
| SEQ ID NO: 16 | LMP1 Strain AG876 |
| SEQ ID NO: 17 | LMP1 Strain B95-8 |
| SEQ ID NO: 18 | LMP1 Strain Cao |
| SEQ ID NO: 19 | LMP1 Strain GD1 |
| SEQ ID NO: 20 | LMP1 Strain Raji |
| SEQ ID NO: 21 | VP26 Strain B95-8 |
| SEQ ID NO: 22 | BZLF1_EBVB9_0085 AYQAYAAPQLFPVSD |
| SEQ ID NO: 23 | BZLF1_EBVB9_0163 QQPESLEECDSELEI |
| SEQ ID NO: 24 | BZLF1_EBVB9_0169 EECDSELEIKRYKNR |
| SEQ ID NO: 25 | BZLF1_EBVB9_0172 DSELEIKRYKNRVAS |
| SEQ ID NO: 26 | BZLF1_EBVB9_0193 FKQLLQHYREVAAAK |
| SEQ ID NO: 27 | BZLF1_EBVB9_0196 LLQHYREVAAAKSSE |
| SEQ ID NO: 28 | BZLF1_EBVB9_0223 PSLDVDSIIPRTPDV |
| SEQ ID NO: 29 | BZLF1_EBVB9_0226 DVDSIIPRTPDVLHE |
| SEQ ID NO: 30 | EBNA1_EBVB9_0484 GLRALLARSHVERTT |
| SEQ ID NO: 31 | EBNA1_EBVB9_0514 KTSLYNLRRGTALAI |
| SEQ ID NO: 32 | EBNA1_EBVB9_0517 LYNLRRGTALAIPQC |
| SEQ ID NO: 33 | EBNA1_EBVB9_0523 GTALAIPQCRLTPLS |
| SEQ ID NO: 34 | EBNA1_EBVB9_0526 LAIPQCRLTPLSRLP |
| SEQ ID NO: 35 | EBNA1_EBVB9_0532 RLTPLSRLPFGMAPG |
| SEQ ID NO: 36 | EBNA1_EBVB9_0535 PLSRLPFGMAPGPGP |
| SEQ ID NO: 37 | EBNA3_EBVB9_0004 DRPGPPALDDNMEEE |
| SEQ ID NO: 38 | EBNA3_EBVB9_0010 ALDDNMEEEVPSTSV |
| SEQ ID NO: 39 | EBNA3_EBVB9_0082 PPQPDLPGREAILRR |
| SEQ ID NO: 40 | EBNA3_EBVB9_0088 PGREAILRRFPLDLR |
| SEQ ID NO: 41 | EBNA3_EBVB9_0091 EAILRRFPLDLRTLL |
| SEQ ID NO: 42 | EBNA3_EBVB9_0160 KWRLQTLAAGWPMGY |
| SEQ ID NO: 43 | EBNA3_EBVB9_0163 LQTLAAGWPMGYQAY |
| SEQ ID NO: 44 | EBNA3_EBVB9_0184 YTDHQTTPTFVHLQA |
| SEQ ID NO: 45 | EBNA3_EBVB9_0196 LQATLGCTGGRRCHV |
| SEQ ID NO: 46 | EBNA3_EBVB9_0199 TLGCTGGRRCHVTFS |
| SEQ ID NO: 47 | EBNA3_EBVB9_0211 TFSAGTFKLPRCTPG |
| SEQ ID NO: 48 | EBNA3_EBVB9_0220 PRCTPGDRQWLYVQS |
| SEQ ID NO: 49 | EBNA3_EBVB9_0244 NPRYSIFFDYMAIHR |
| SEQ ID NO: 50 | EBNA3_EBVB9_0247 YSIFFDYMAIHRSLT |
| SEQ ID NO: 51 | EBNA3_EBVB9_0250 FFDYMAIHRSLTKIW |
| SEQ ID NO: 52 | EBNA3_EBVB9_0280 LGFLQRTDLSYIKSF |
| SEQ ID NO: 53 | EBNA3_EBVB9_0283 LQRTDLSYIKSFVSD |
| SEQ ID NO: 54 | EBNA3_EBVB9_0319 QAWNAGFLRGRAYGI |
| SEQ ID NO: 55 | EBNA3_EBVB9_0325 FLRGRAYGIDLLRTE |
| SEQ ID NO: 56 | EBNA3_EBVB9_0331 YGIDLLRTEGEHVEG |
| SEQ ID NO: 57 | EBNA3_EBVB9_0370 VSRGGPKVKRPPIFI |
| SEQ ID NO: 58 | EBNA3_EBVB9_0373 GGPKVKRPPIFIRRL |
| SEQ ID NO: 59 | EBNA3_EBVB9_0412 STYGTPRPPVPKPRP |
| SEQ ID NO: 60 | EBNA3_EBVB9_0508 PIVRPWEPSLTQAAG |
| SEQ ID NO: 61 | EBNA3_EBVB9_0544 ALERPVYPKPVRPAP |
| SEQ ID NO: 62 | EBNA3_EBVB9_0547 RPVYPKPVRPAPPKI |
| SEQ ID NO: 63 | EBNA3_EBVB9_0808 LGYTLHGLNHPGVPV |
| SEQ ID NO: 64 | EBNA4_EBVB9_0166 RWKLLSSCRSWRMGY |
| SEQ ID NO: 65 | EBNA4_EBVB9_0169 LLSSCRSWRMGYRTH |
| SEQ ID NO: 66 | EBNA4_EBVB9_0178 MGYRTHNLKVNSFES |
| SEQ ID NO: 67 | EBNA4_EBVB9_0184 NLKVNSFESGGDNVH |
| SEQ ID NO: 68 | EBNA4_EBVB9_0235 IETAFLMARRARSLS |
| SEQ ID NO: 69 | EBNA4_EBVB9_0238 AFLMARRARSLSAER |
| SEQ ID NO: 70 | EBNA4_EBVB9_0295 HIREWFRQCTGRPKA |
| SEQ ID NO: 71 | EBNA4_EBVB9_0307 PKAAKPWLRAHPVAI |
| SEQ ID NO: 72 | EBNA4_EBVB9_0310 AKPWLRAHPVAIPYD |
| SEQ ID NO: 73 | EBNA4_EBVB9_0331 EIDLAYARGQAMNIE |
| SEQ ID NO: 74 | EBNA4_EBVB9_0388 LPYNPTVYGRPAVFD |
| SEQ ID NO: 75 | EBNA4_EBVB9_0394 VYGRPAVFDRKSDAK |
| SEQ ID NO: 76 | EBNA4_EBVB9_0430 HRKKKAARTEQPRAT |
| SEQ ID NO: 77 | EBNA4_EBVB9_0529 PQQPRAGRRGPCVFT |
| SEQ ID NO: 78 | EBNA4_EBVB9_0619 AAPRQWPMPLRPIPM |
| SEQ ID NO: 79 | EBNA4_EBVB9_0622 RQWPMPLRPIPMRPL |
| SEQ ID NO: 80 | EBNA4_EBVB9_0628 LRPIPMRPLRMQPIP |
| SEQ ID NO: 81 | EBNA4_EBVB9_0631 IPMRPLRMQPIPFNH |
| SEQ ID NO: 82 | EBNA4_EBVB9_0634 RPLRMQPIPFNHPVG |
| SEQ ID NO: 83 | EBNA4_EBVB9_0664 PTWAQIGHIPYQPTP |
| SEQ ID NO: 84 | EBNA4_EBVB9_0691 PATMQTPPRAPTPMS |
| SEQ ID NO: 85 | EBNA4_EBVB9_0787 LKLQAALERQAAAGW |
| SEQ ID NO: 86 | EBNA4_EBVB9_0919 LGLGDIAVSSPSSSE |
| SEQ ID NO: 87 | EBNA6_EBVB9_0058 WGQSRGDENRGWMQR |
| SEQ ID NO: 88 | EBNA6_EBVB9_0064 DENRGWMQRIRRRRR |
| SEQ ID NO: 89 | EBNA6_EBVB9_00731RRRRRRRAALSGHL |
| SEQ ID NO: 90 | EBNA6_EBVB9_0076 RRRRRAALSGHLLDT |
| SEQ ID NO: 91 | EBNA6_EBVB9_0088 LDTEDNVPPWLPPHD |
| SEQ ID NO: 92 | EBNA6_EBVB9_0097 WLPPHDITPYTARNI |
| SEQ ID NO: 93 | EBNA6_EBVB9_0103 ITPYTARNIRDAACR |
| SEQ ID NO: 94 | EBNA6_EBVB9_0109 RNIRDAACRAVKQSH |
| SEQ ID NO: 95 | EBNA6_EBVB9_0145 VMAARQRLQDIRRGP |
| SEQ ID NO: 96 | EBNA6_EBVB9_0160 LVAEGGVGWRHWLLT |
| SEQ ID NO: 97 | EBNA6_EBVB9_0163 EGGVGWRHWLLTSPS |
| SEQ ID NO: 98 | EBNA6_EBVB9_0169 RHWLLTSPSQSWPMG |
| SEQ ID NO: 99 | EBNA6_EBVB9_0178 QSWPMGYRTATLRTL |
| SEQ ID NO: 100 | EBNA6_EBVB9_0181 PMGYRTATLRTLTPV |
| SEQ ID NO: 101 | EBNA6_EBVB9_0184 YRTATLRTLTPVPNR |
| SEQ ID NO: 102 | EBNA6_EBVB9_0241 AREAEVRFLRGKWQR |
| SEQ ID NO: 103 | EBNA6_EBVB9_0271 HHIWQNLLQTEENLL |
| SEQ ID NO: 104 | EBNA6_EBVB9_0277 LLQTEENLLDFVRFM |
| SEQ ID NO: 105 | EBNA6_EBVB9_0280 TEENLLDFVRFMGVM |
| SEQ ID NO: 106 | EBNA6_EBVB9_0283 NLLDFVRFMGVMSSC |
| SEQ ID NO: 107 | EBNA6_EBVB9_0286 DFVRFMGVMSSCNNP |
| SEQ ID NO: 108 | EBNA6_EBVB9_0295 SSCNNPAVNYWFHKT |
| SEQ ID NO: 109 | EBNA6_EBVB9_0301 AVNYWFHKTIGNFKP |
| SEQ ID NO: 110 | EBNA6_EBVB9_0304 YWFHKTIGNFKPYYP |
| SEQ ID NO: 111 | EBNA6_EBVB9_0307 HKTIGNFKPYYPWNA |
| SEQ ID NO: 112 | EBNA6_EBVB9_0313 FKPYYPWNAPPNENP |
| SEQ ID NO: 113 | EBNA6_EBVB9_0331 RRGIKEHVIQNAFRK |
| SEQ ID NO: 114 | EBNA6_EBVB9_0334 IKEHVIQNAFRKAQI |
| SEQ ID NO: 115 | EBNA6_EBVB9_0340 QNAFRKAQIQGLSML |
| SEQ ID NO: 116 | EBNA6_EBVB9_0523 SSVSQPNKPHRKHQD |
| SEQ ID NO: 117 | EBNA6_EBVB9_0610 GPHIVTPPSARPRIM |
| SEQ ID NO: 118 | EBNA6_EBVB9_0625 APPVVRMFMRERQLP |
| SEQ ID NO: 119 | EBNA6_EBVB9_0631 MFMRERQLPQSTGRK |
| SEQ ID NO: 120 | EBNA6_EBVB9_0649 FWEMRAGREITQMQQ |
| SEQ ID NO: 121 | EBNA6_EBVB9_0790 PGYAGPWTPRSQHPC |
| SEQ ID NO: 122 | EBNA6_EBVB9_0796 WTPRSQHPCYRHPWA |
| SEQ ID NO: 123 | EBNA6_EBVB9_0799 RSQHPCYRHPWAPWS |
| SEQ ID NO: 124 | EBNA6_EBVB9_0850 FPHLQSETGPPRLQL |
| SEQ ID NO: 125 | EBNA6_EBVB9_0856 ETGPPRLQLSLVPLV |
| SEQ ID NO: 126 | EBNA6_EBVB9_0883 RAPIRPIPTRFPPPP |
| SEQ ID NO: 127 | LMP1_EBVB9_0031 LLLLALLFWLYIVMS |
| SEQ ID NO: 128 | LMP1_EBVB9_0127 LEMLWRLGATIWQLL |
| SEQ ID NO: 129 | LMP1_EBVB9_0178 LAILIWMYYHGQRHS |
| SEQ ID NO: 130 | LMP1_EBVB9_0181 LIWMYYHGQRHSDEH |
| SEQ ID NO: 131 | LMP1_EBVB9_0184 MYYHGQRHSDEHHHD |
| SEQ ID NO: 132 | VP26_EBVB9_0001 MARRLPKPTLQGRLE |
| SEQ ID NO: 133 | VP26_EBVB9_0055 FCYEEYVQRTFGVPR |
| SEQ ID NO: 134 | VP26_EBVB9_0064 TFGVPRRQRAIDKRQ |
| SEQ ID NO: 135 | VP26_EBVB9_0079 RASVAGAGAHAHLGG |

A "latent Epstein-Barr virus (EBV) infection" is an EBV infection that is no longer acute. In latency, specific genes are expressed, in EBV these include EBERs and EBNAs, (see US patent application 2005/0074751 pages 4-5 for a discussion of EBV). According to this publication, in Type I latency, only EBNA-1 is expressed, in Type II latency, LMP-1, LMP-2A and LMP-2B are expressed and in Type III latency, LMP-1, LMP-2A, LMP-2B LP, EBNA-1, EBNA-2, EBNA-3A, EBNA-3B and EBNA-3C are expressed.

In the context of the present invention, a "sample obtained from the body of an individual" can be selected from the group consisting of a blood sample, a saliva sample, a urine sample, a nasopharygeal sample, a sample from a pharyngeal wash and a sample from a gastric or colonic biopsy.

The "physical disorder" to be differentiated from CFS refers to a physical disorder with very similar symptoms to CFS, such as depression or burn-out syndrome.

In the context of the present invention, an "immune response" is an antibody response found in a sample from an individual. The term immune response could also be replaced with the term antibody response or seroreactivity. The immune response can be measured by an immunoassay, which is in turn an assay that measures the immune response. Such an assay can be based on a peptide microarray or other systems where peptides are immobilized to solid phases. An immune response further is a T or B cell response.

The term "measuring the amount of a protein, a fragment thereof or the corresponding nucleotide" refers to standard methods known in the art such as immunostaining or quantative PCR.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

As described herein, "preferred embodiment" means "preferred embodiment of the present invention". Likewise, as described herein, "various embodiments" and "another embodiment" means "various embodiments of the present invention" and "another embodiment of the present invention", respectively.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

### Examples

The following examples illustrate the invention. These examples should not be construed as to limit the scope of this invention. The examples are included for purposes of illustration and the present invention is limited only by the claims.

### Methods:

### Study population and specimen collection

Patients who were diagnosed with CFS according to Fukuda criteria without evidence of other severe medical, neurological or psychiatric disease (Fukuda 1994) were recruited from the outpatient clinic for Adult Immunodeficiencies of the Department of Medical Immunology of the Charité University Medicine Berlin between 2007 and 2013. Informed consent was obtained from all patients and healthy control donors and the study was approved by the Institutional Ethics Committee.

### Blood samples

Blood was obtained from CFS patients or healthy subjects. Peripheral blood mononuclear cells (PBMC) were isolated by density gradient centrifugation using Ficoll Hypaque and either cryopreserved for T-cell analysis or used in cell culture stimulation assay for memory B-cell analysis and B-cell separation. Serum was obtained separately from CFS patients, depression patients and healthy subjects and stored at -20 °C for serum antibody analysis.

### Pharyngeal wash samples

Patients gargle with 5ml of water for 2 minutes.

### Example 1: Enzyme-linked immunosorbent assay (ELISA)

### Methods:

EBNA IgG, VCA IgG and VCA IgM were detected using an immuno chemiluminescence assay (CLIA, DiaSorin, S.p.A., Saluggia, Italy) according to the manufacture's instructions. An Enzyme Immunoassay was used to detect EBV EBNA-1 IgG in the Labor Berlin GmbH. Seronegative subjects and subjects with recent EBV-infection were excluded from the analysis.

### Results:

In this experiment, it was found that a subset of CFS patients shows abnormal EBV serology. We compared serum EBV-VCA IgG, IgM, and EBNA1 IgG from patients (n=63) and controls (n=57) **(****Fig. 1A****).** All but 1 patient was EBV-VCA IgG positive, who was excluded from this analysis. While we did not observe a difference between VCA-IgG titers, IgG antibodies against EBNA1 were undetectable (≤ 20 U/ml) in 12.7% of CFS patients in contrast to 3.5% of healthy controls (p=0.066, **Fig 1B****).** Further, VCA IgM was more frequent in patients with 17.5% compared to 3.5% in healthy controls **(****Fig. 1C****,** p=0.013). In contrast, titers of CMV IgG and IgM revealed no difference between CFS patients and healthy controls **(****Figs. 1** **D** and **E).** Elevated EBV-VCA IgM and lack of EBNA1 IgG were detected in different patient subsets accounting for 30% of CFS patients with an abnormal EBV serology.

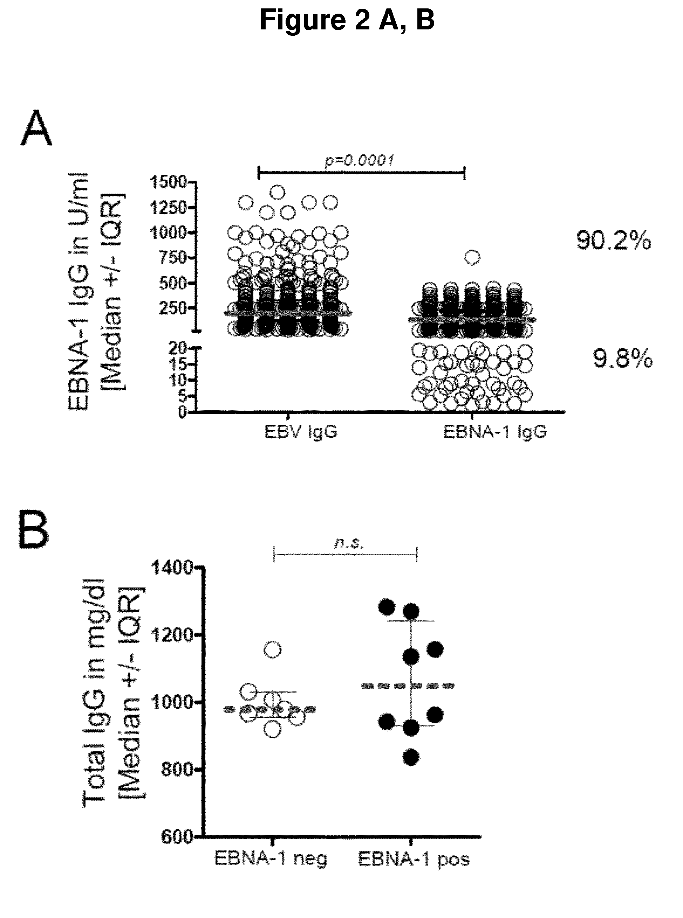

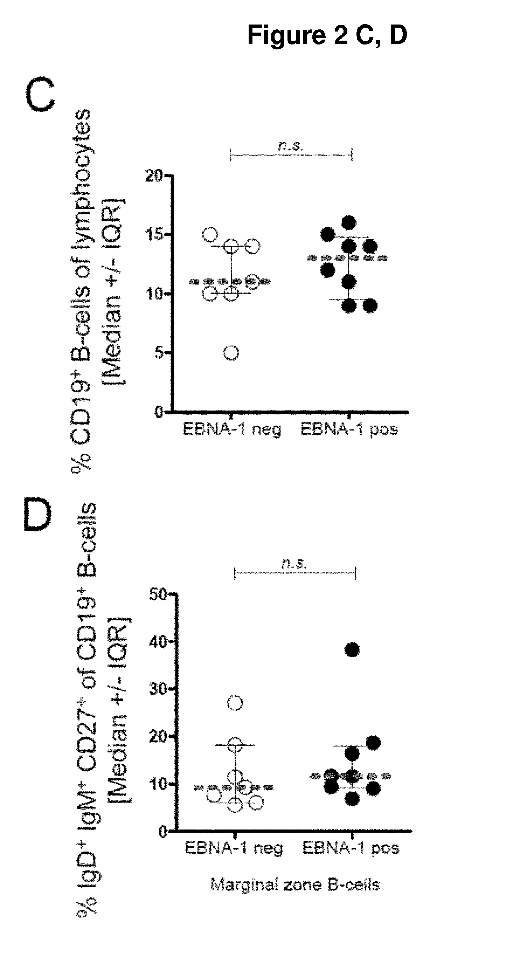

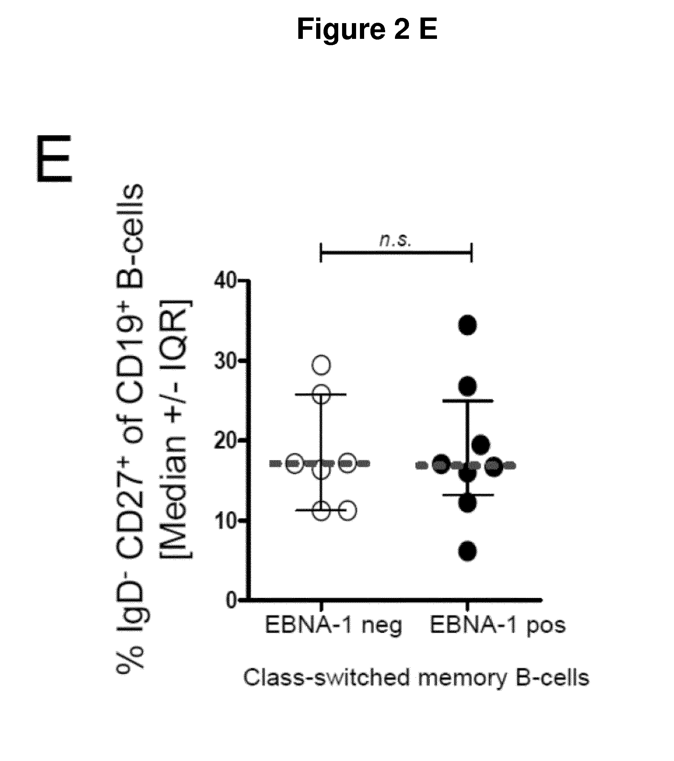

In a consecutive cohort of 397 CFS patients EBV-specific antibodies were measured with Enzyme Imunoassays determining IgG against a mixture of various EBV proteins or EBNA-1. Similarly, we observed a lack of EBNA-1 IgG in 9.8% of EBV-IgG positive patients **(****Fig. 2A****).** In a subset of EBNA-1 IgG positive and negative patients we further comparatively analyzed total IgG levels, frequencies of B-cells, and B-cell subsets. No difference in IgG as well as total and memory B-cell subset frequencies was seen in EBNA-1 IgG negative compared to EBNA-1 IgG positive CFS patients and numbers of B-cell subsets were within the normal range compared to the reference group of the Charité Immune diagnostic laboratory as indicated by the dashed line **(****Fig. 2B-E****).**

### Example 2: Quantitative real-time PCR

### Methods:

Detection of EBV DNA in PBMCs was done by nested PCR for EBER-1 with the following primers forward 5'-TCC CGG GTA CAA GTC CCG-3' and reverse 5'-TGA CCG AAG ACG GCA GAA AG-3' at 900 nM. Detection has been performed with probe FAM-5'-TGG TGA GGA CGG TGT CTG TGG TTG TGT T-3'-TAMRA (Eurofins MWG Operon, Ebersberg Germany) at 5 pM. Amplification data were analyzed by an ABI PRISM 7700 Sequence Detection System (PE Applied Biosystems, California, USA). Successful DNA isolation was verified by histone replication with the primers forward 5'-CCA GAG CGC AGC TAT CGG T-3' at 900 nM and reverse 5'-CAC GTT TGG CAT GGA TAG CAC -3' at 50 nM and the probe FAM - 5'-GCA AGT GAG GCC TAT CTG GTT GGC CTT T-3- TAMRA (Eurofins MWG Operon, Ebersberg Germany) at 5 pM. For EBNA-1 the following primers forward 5'-TACAGGACCTGGAAATGGCC-3' and reverse 5'-TCTTTGAGGTCCACTGCCG-3' at 15 pM were used. Detection has been performed with probe FAM-5'-AGGGAGACACATCTGGACCAGAAGGC-3'-TAMRA (Eurofins MWG Operon, Ebersberg Germany) at 10 pM. For BZLF-1 the following primers forward 5'-AAATTTAAGAGATCCTCGTGTAAAACATC-3' and reverse 5'-CGCCTCCTGTTGAAGCAGAT-3' at 30 pM were used. Detection has been performed with probe FAM-5'-ATAATGGAGTCAACATCCAGGCTTGGGC-3'-TAMRA (Eurofins MWG Operon, Ebersberg Germany) at 10 pM. EBV copies/µg DNA were calculated as standard EBV copies/µl DNA divided by sample DNA concentration (µg/µl). Results above 35 copies/ µg DNA were regarded as positive according to Holland et al. 1991, PNAS 88: 7276-7280).

### Results

Real-time PCR was used to determine EBV viral load by EBER-1 gene expression in blood immune cells of patients with CFS and healthy controls. EBV-EBER-1 DNA copies were detected in 50% of patients (n=13/26) and in 17.2% (n=5/29) healthy controls in peripheral blood mononuclear immune cells (PBMC) **(****Fig. 3A****).** Further, EBV DNA was compared in pharyngeal washings of 10 CFS patients and 10 healthy controls revealing a positive EBER signal in 6 samples of both cohorts. Additionally, we tested 4 EBV seronegative CFS patients and detected no EBER-1 DNA in PBMCs (data not shown). We further tried to detect EBNA-1 copies in the DNA of EBER-1 positive CFS patients but only 2 of 6 patients were positive for EBNA-1. As a third gene we determined BZLF-1 copy numbers , revealing a signal in 13 of 27 CFS patients (48%) but only in 5 of 27 healthy controls (18%) **(****Fig. 3B****).** Interestingly no correlation between EBER-1 and BZLF-1 copies was seen in the patients and controls. Altogether EBV-EBER-1 or BZLF-1 copies could be detected in PBMC in 67% (n=18/27) of patients in contrast to 26% (n=7/27) of controls. To study lytic replication we further looked for EBV RNA but did not detect copies of BZLF-1 mRNA in either patients or controls, but low level transcripts of EBER-1 mRNA in most EBER-1 DNA positive patients but also in EBER1-DNA positive controls.

### Example 3: B-cell memory analysis by ELISpot-assay

### Methods:

Analysis of memory B-cells was adapted from Crotty *et al.* (Crotty 2004, J. Immunol. Methods 286: 111-122). PBMCs were stimulated unspecifically with Pokeweed mitogen (PWM) at 10ng/ml (Sigma Aldrich, Schnelldorf, Germany), Staphylococcus aureus Cowan at 1:10000 dilution (Merck, Darmstadt, Germany) and CpG at 6µg/ml (InvivoGen, CA, USA) in RPMI 1640 (PAA Laboratories, Cölbe, Germany) supplemented with Penicillin/Streptomycin 100x (Biochrom, Berlin, Germany), L-Glutamine at 2mM (Biochrom, Berlin, Germany) and β-Mercaptoethanol at 50µM (Merck, Darmstadt, Germany) for 7 days at 37°C in 5% CO₂. For T-cell independent stimulation B-cells from CFS patients were enriched with a RosetteSep CD3 depletion kit according to the manufacturer's instructions (Stemcell Technologies, Grenoble, France). 2.5·10⁶ B-cells/ well were kept in 1ml IMDM (PAA Laboratories, Cölbe, Germany) with 10% heat-inactivated AB serum (Valley Biomedical, Winchester, VA, USA), 5µg/ml Insulin (Sigma Aldrich, Schnelldorf, Germany)/ Transferrin (Sigma Aldrich, Schnelldorf, Germany), 5ng/ml selenium (Sigma Aldrich, Schnelldorf, Germany), 1.25µg/ml CpG (Invivogen, CA, USA), 300U/ml IL-2 (Chiron-Behring, Liederbach, Germany), 12.5ng/ml IL-10 (ImmunoTools, Friesoythe) and 500ng/ml IL-21 (ImmunoTools, Friesoythe, Germany). Cells were cultured for 7 days at 37°C in 5% CO₂. After stimulation, the cells were transferred at a concentration of 1x10⁶/ 100µl into a 96-well multiscreen HTS-IP filter plate (Merck Millipore, MA, USA) coated with purified, recombinant EBV-VCA at 0.1µg/ well (tebu-bio, Le-Perray-en-Yvelines, France) and EBV-EBNA-1 at 1µg/ well (tebu-bio, Le-Perray-en-Yvelines, France) and purified EBV-lysate at 1:20 dilution (tebu-bio, Le-Perray-en-Yvelines, France). For the analysis of total IgG anti-human IgG Fc-fragment antibody (Jackson Immunoresearch, PA, USA) was coated at a concentration of 1.2 µg/ well and cells were seeded at a concentration of 1.25 x 10⁴/100µl, 6250/100µl and 3125/100µl for 6h. Secreted IgGs were detected using an anti-human IgG, F(ab')2 fragment coupled to Biotin at 1µg/ml (Biosource, Life Technologies, Darmstadt, Germany) and Horseradish Peroxidase Avidin D at 5ng/ml (Vector Laboratories, MI, USA). IgG spots were visualized by adding 3-Amino-9-ethylcarbazole (Sigma-Aldrich, Schnelldorf, Germany). Plates were scanned and spots enumerated on a CTL Immunoplate reader using Immunospot Academic software (Cellular Technology Ltd, OH, USA). Frequencies were expressed as the ratio of the mean number of antigen specific spots and mean number of total IgG spots.

### Results:

In this experiment, it was found that EBV specific memory B-cells are low or absent in most CFS patients. No difference between CFS patients and healthy controls was detected for total IgG producing B-cells quantified by ELISpot assay **(****Fig. 4A****).** However, the CFS patients had significantly reduced frequencies of B-cells producing antibodies against EBV lysate. In contrast we detected no difference in ASCs producing antibodies against HSV or CMV lysate analyzed in a subset of patients and controls **(****Fig. 4B****).** In addition using overlapping peptide pools, VCA-specific **(****Fig. 4C****)** and EBNA-1 IgG secreting B-cells were analyzed **(****Fig. 4D****)** which were low or absent in most CFS patients analyzed.

Taken together, the lack of memory B-cells despite the presence of normal antibody levels in many patients suggests a secondary immune deficiency or exhaustion of the EBV-specific B-cell response in patients with CFS.

### Example 4: T cell response by cytokine analysis

### Methods:

Antigen specific T-cell response was measured by cytokine production in culture supernatants of PBMCs stimulated with either 1 µg/ml SEB (Sigma-Aldrich, Schnelldorf, Germany), 1 µg/ml EBV total lysate or 1 µg/ml of the EBV peptide EBNA-1 (JPT, Berlin, Germany) for 48h. 2x10⁶ PBMCs were kept in 1ml serumfree RPMI (PAA Laboratories, Cölbe, Germany) with 2% Hepes buffer, 1% L-Glutamin (Biochrom, Berlin, Deutschland) and 0.5 % Gentamycin (Merck, Darmstadt, Germany). IFN-γ, IL-10, IL-2 and TNF-α were measured in cell culture supernatants with a 7-plex-Immunoassay (Merck Millipore, MA, USA) on a Luminex 200 (Luminex, TX, USA) according to manufacturer's instructions.

### Results:

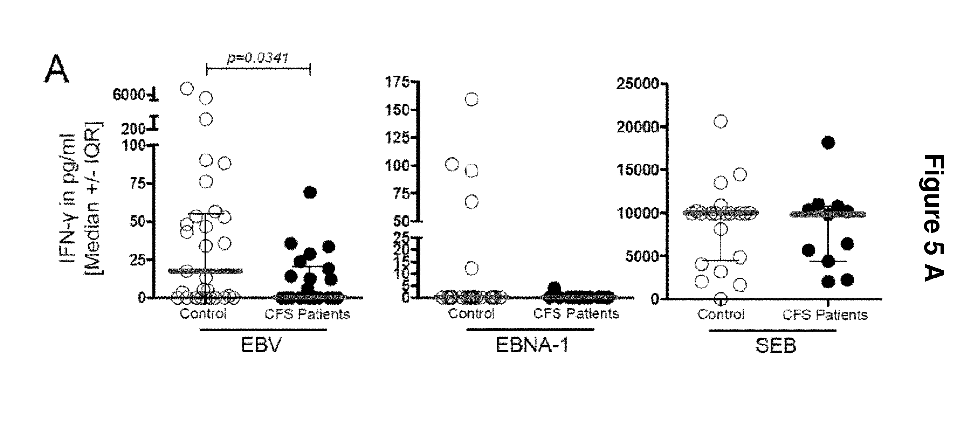

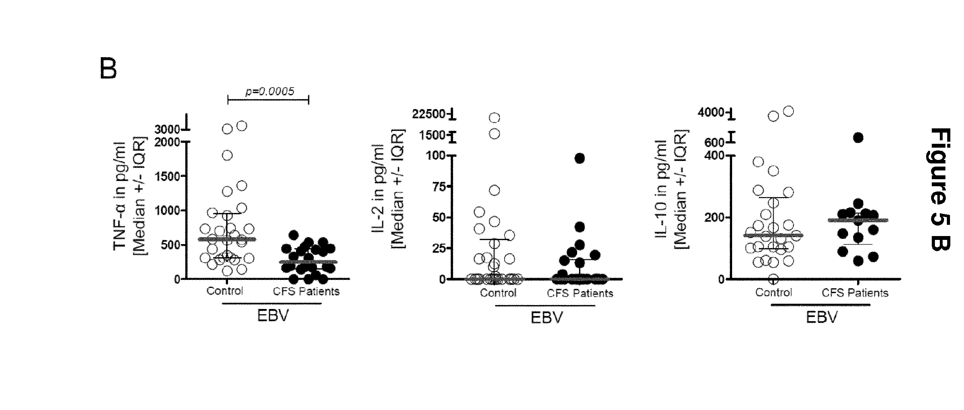

In this experiment, it was found that CFS patients show diminished T-cell cytokine response against EBV. First, EBV lysate induced production of several cytokines that were analyzed in whole blood revealed a reduced number of IFN-γ responders in the patient group with 50% (n=11/22) vs. 69% (n=20/29) in the control group **(****Fig. 5A****).** Using overlapping 15-mer peptides from various EBV proteins for stimulation only few responders could be detected in healthy controls as shown for EBNA-1 induced IFN-γ. Similar IFN-γ levels were observed in response to the T-cell super-antigen SEB in patients and controls. In addition, significantly reduced levels of TNF-α and a lower number of patients producing IL-2 were observed, while the IL-10 response was not diminished in patients in response to EBV lysate **(****Fig. 5B****).**

### Example 5: T-cell response assessed by flow cytometry after in vitro expansion

### Methods:

EBV specific memory T-cells were analyzed after stimulation with EBNA-1 peptides and expansion *in vitro* as recently described (Guerreiro 2010). After overnight incubation of PBMCs in IMDM (PAA Laboratories, Cölbe, Germany) containing 10% AB serum (Valley Biomedical, Winchester, VA, USA) at 37°C in 5% CO₂, cells were stimulated with 1µg/mL EBNA-1 peptide pool (JPT, Berlin, Germany), 50IU/mL rhIL-2 (Chiron-Behring, Liederbach, Germany) and 10ng/mL rhIL-7 (ImmunoTools, Friesoythe, Germany) in 96-well round bottom plates at a concentration of 2x10⁵ cells per well. On day 3, 5 and 7 media and IL-2 50ng/µl were renewed. IL-7 5ng/µl was added on day 7 of culture, cells were harvested, washed and stained for cytokines.

### Multiparameter flow cytometry

Intracellular and extracellular staining was applied for T-cell analysis after 10 days of expansion. 2x10⁶ PBMCs were restimulated with EBNA-1 peptide pool (JPT, Berlin, Germany) (1µg/mL) or DMSO (1µg/mL) (Sigma Aldrich, Schnelldorf, Germany) as negative control for 5h. Brefeldin A (7.5 µg/mL) (Sigma Aldrich, Schnelldorf, Germany) was added after 1h of stimulation. Live/dead cells were discriminated using an amine reactive dye (Invitrogen, Life Technologies, Darmstadt, Germany) and stained with fluorescence conjugated monoclonal antibody against CD3, CD4, CD8 and IFN-y, TNF-α and IL-2 (BD Biosciences, NJ, USA). Background events in DMSO controls were subtracted from events counted in response to EBNA-1 stimulation. Data acquisition was performed on BD LSR II (Becton Dickinson, NJ, USA) and analysis was done using FlowJo software.

### Statistical Analysis

Statistical data analyses were done using the software SPSS Statistics 19 and GraphPad Prism 5. Nonparametric statistical methods were used. Continuous variables were expressed as median and interquartile range (IQR), if not indicated otherwise. Univariate comparisons of two independent groups were done using the Mann-Whitney-U test. For association analysis Fisher's exact test was used. A p-value of <0.05 was considered statistically significant.

### Results:

In this experiment, it was found that CFS patients show reduced EBV specific multifunctional memory T-cells. Interferon (IFN)-γ⁺ Tumor necrosis factor (TNF)-α⁺ and Interleukine (IL)-2⁺ producing CD3⁺ CD8⁺ and CD3⁺ CD4⁺ T-cells were analyzed by flow cytometry. While we observed similar frequencies of single producers in CFS patients and the control group frequencies of IFN-γ⁺ TNF-α⁺ double producing CD4⁺ T-cells and of IFN-γ⁺ TNF-α⁺ IL-2⁺ triple producing so called multifunctional CD8⁺ and CD4⁺ T-cells were significantly lower in the patient group **(Fig. 6A-C).** Frequencies of IFN-γ⁺ IL-2⁺ and TNF-α⁺ IL-2⁺ double positive T-cells were too low to be reliably determined.

### Example 6 Screening of Sera from Patients with CFS, Depression and Healthy Donors

This experiment follows the observation that in CFS patients altered titers of EBV specific antibodies are observed. Peptide microarray experiments with sera from 36 CFS patients, 34 healthy volunteers and 20 depression patients show a skewed antibody repertoire against EBV-derived antigens with different responses against various peptides from EBNA1, EBNA3, EBNA4, EBNA6, BZLF1, LMP1, and VP26.

### Methods:

A library of 1465 peptides representing a 15/11 scan through EBV-antigens BLRF2, BZLF1, EBNA1, EBNA3, EBNA4, EBNA6, LMP1 and VP26 was applied to microarrays. The preparation of the peptide array (microarray) was performed as published elsewhere (Barouch et al., (2013) J Infect Dis 207: 248-256). Incubation of the microarrays was performed in an automated Hybridization Station and all steps were carried out at 30°C. The serum was diluted 1:200 in diluent buffer SuperBlock T20 (TBS, Pierce). The microarrays were blocked for 1 hour, incubated with the diluted serum for 2 hours and then incubated with Cy5-conjugated AffiniPure Mouse Anti-Human IgG secondary antibody (Jackson Immuno) for 45 minutes, with washing steps with 1xTBS+0.1%Tween20 in between. After the final wash steps microarrays were dried with nitrogen and the signal was read out with a Microarray Scanner. The signal intensity was displayed by relative fluorescence units and heatmaps of signal intensities for individual proteins were then evaluated (see **Figures 7-15****).**

### Results:

A p-value was calculated for each peptide for the null hypothesis that the distribution of signal intensities is equal between patient groups:
a) CFS-Patients vs. healthy volunteers (NORM)
b) CFS-Patients vs. Depression Patients (DEP)

It was found that 114 peptide fragments fulfill the condition of p<0.01 for the comparison of signals from the CFS group to all other groups. These specific protein fragments are disclosed as SEQ ID NOs.: 22-135 in **Table 1.** In the table below, the number of peptides from each protein that are distinct between CFS patients and the other patient groups is shown:

**Table 2**

| **Protein** | **BZLF1** | **EBNA1** | **EBNA3** | **EBNA4** | **EBNA6** | **LMP1** | **VP26** |
|---|---|---|---|---|---|---|---|
| **No. of peptides** | **8** | **7** | **27** | **23** | **40** | **5** | **4** |

From the above **Table 2,** it is apparent that the most differences were seen in the immune response towards peptides from EBNA3, EBNA4 and EBNA6. From this, it was concluded that these three peptides are the most suitable markers for the diagnosis of CFS, and that these also allow for the differentiation between CFS patients and depression patients.

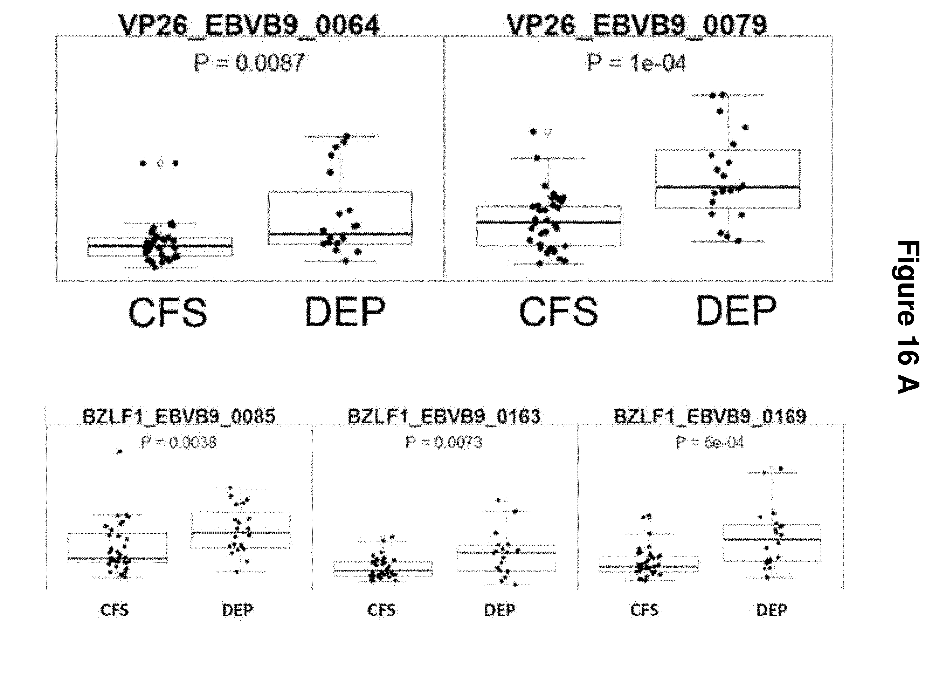

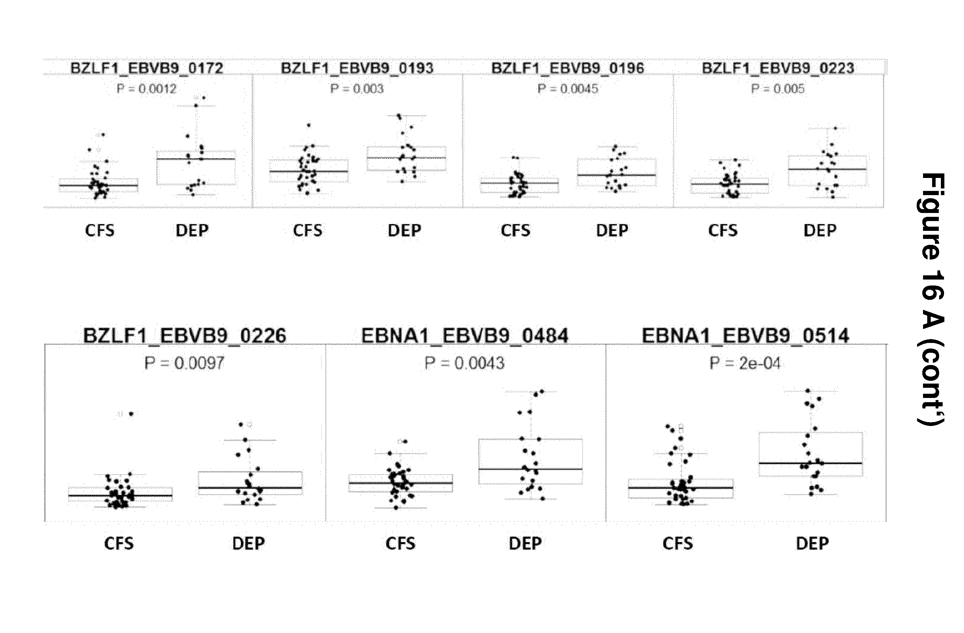

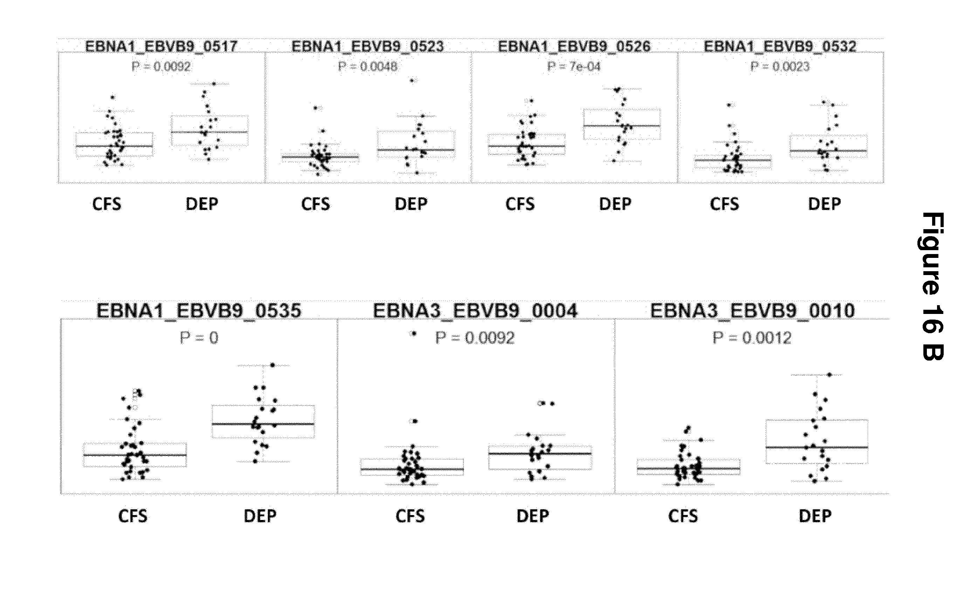

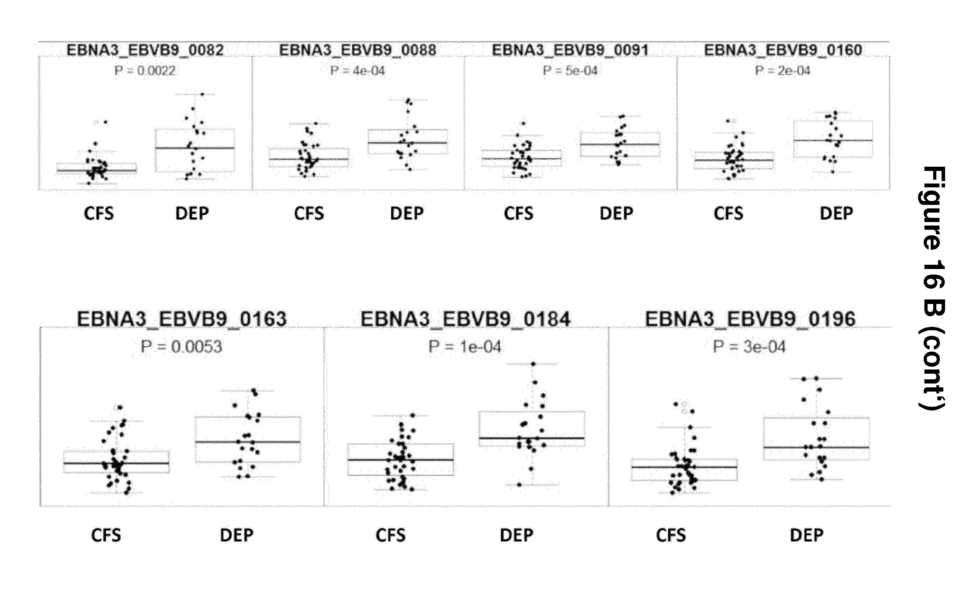

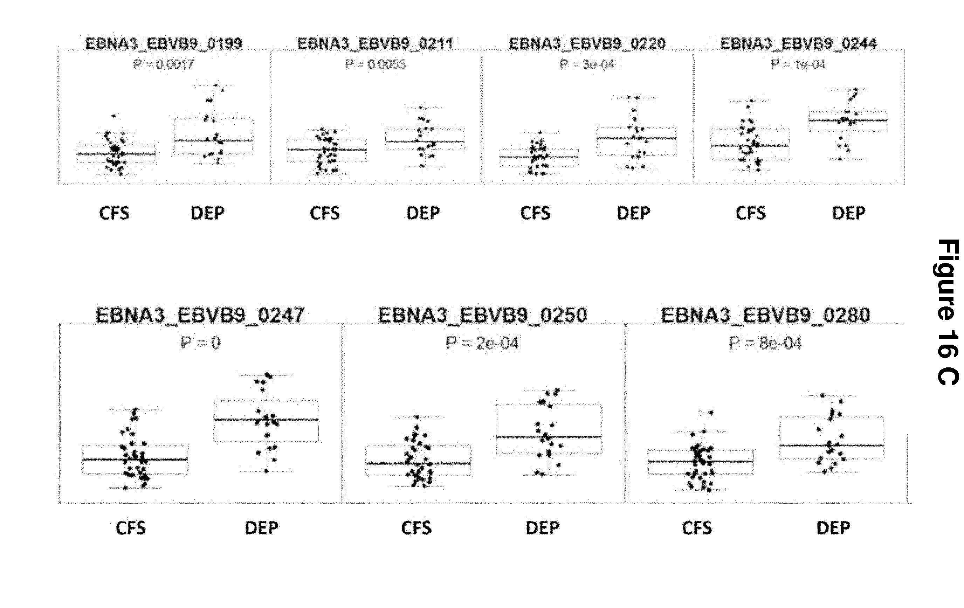

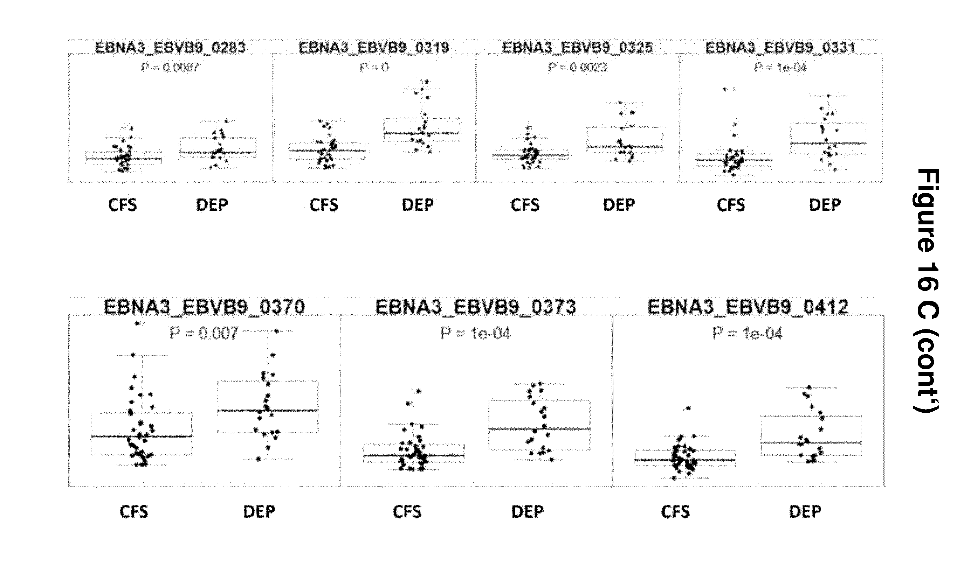

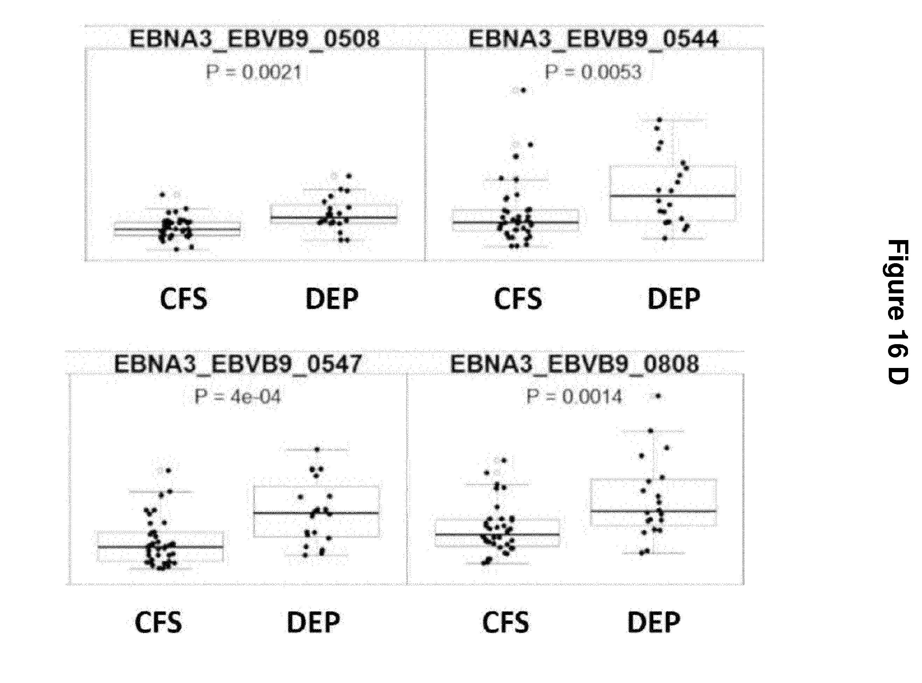

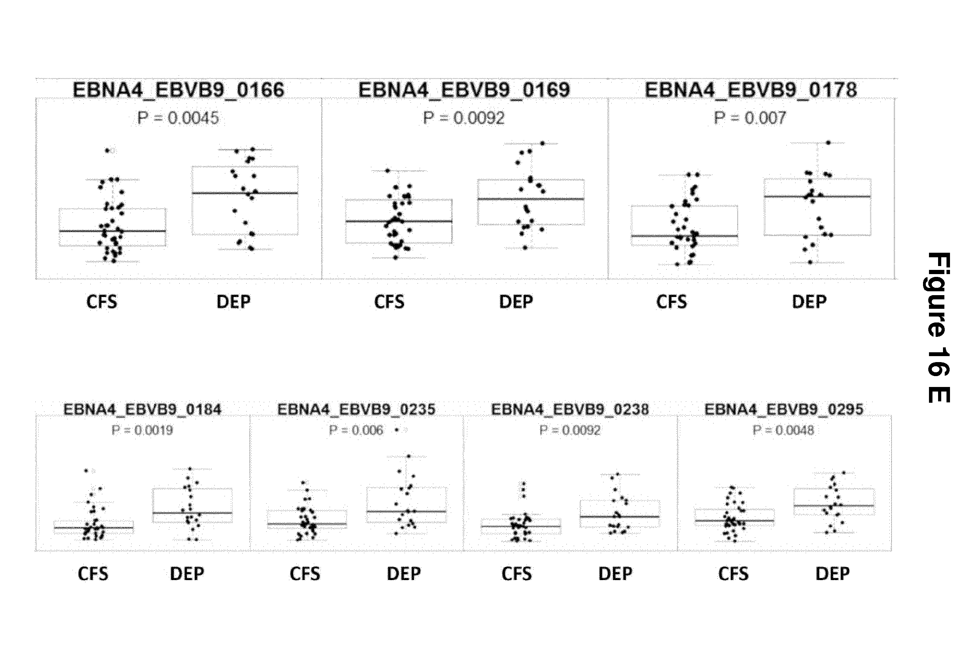

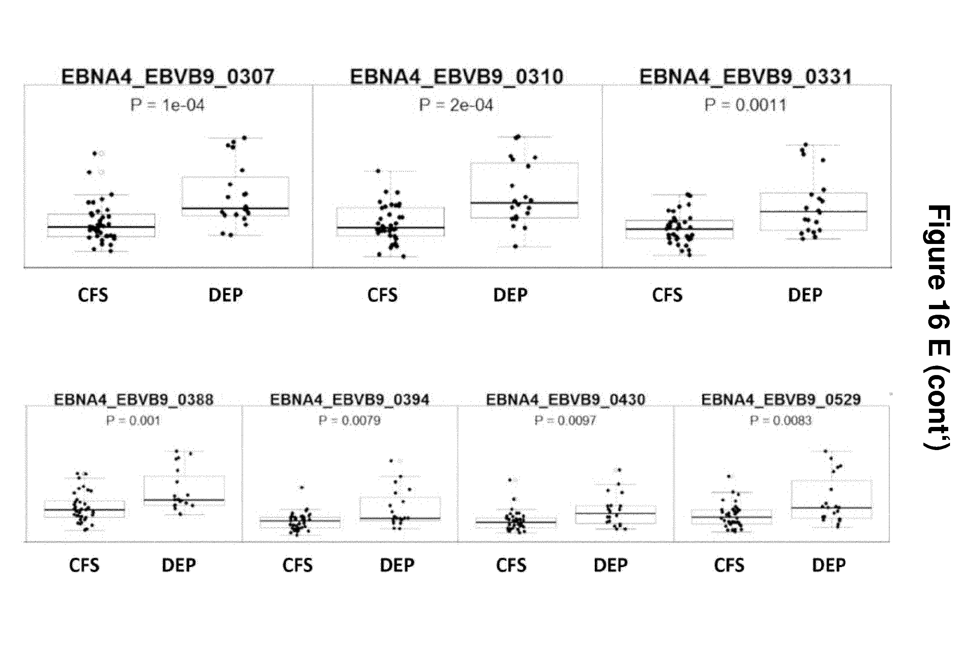

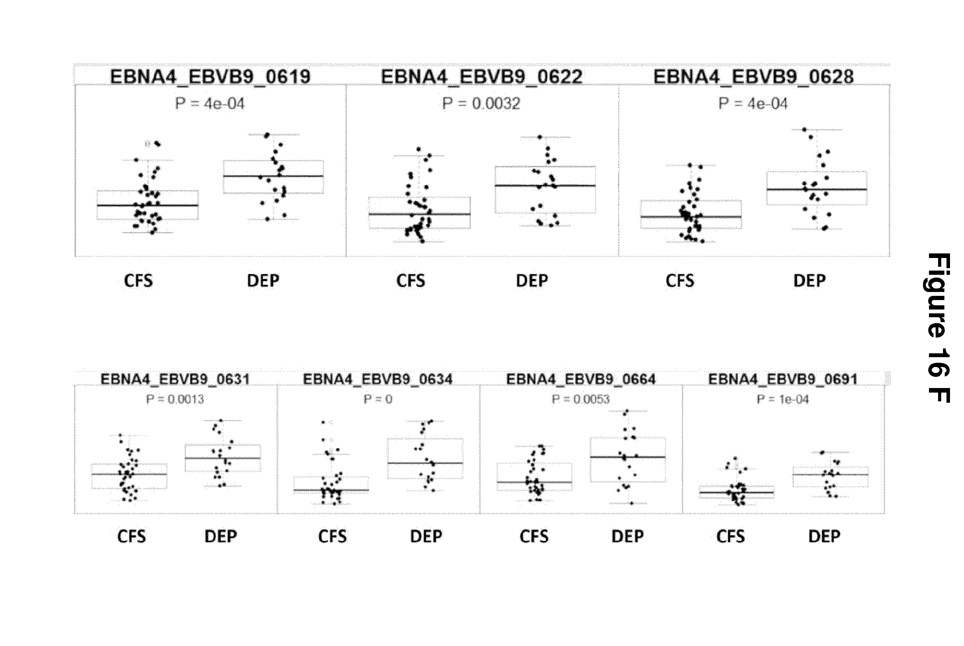

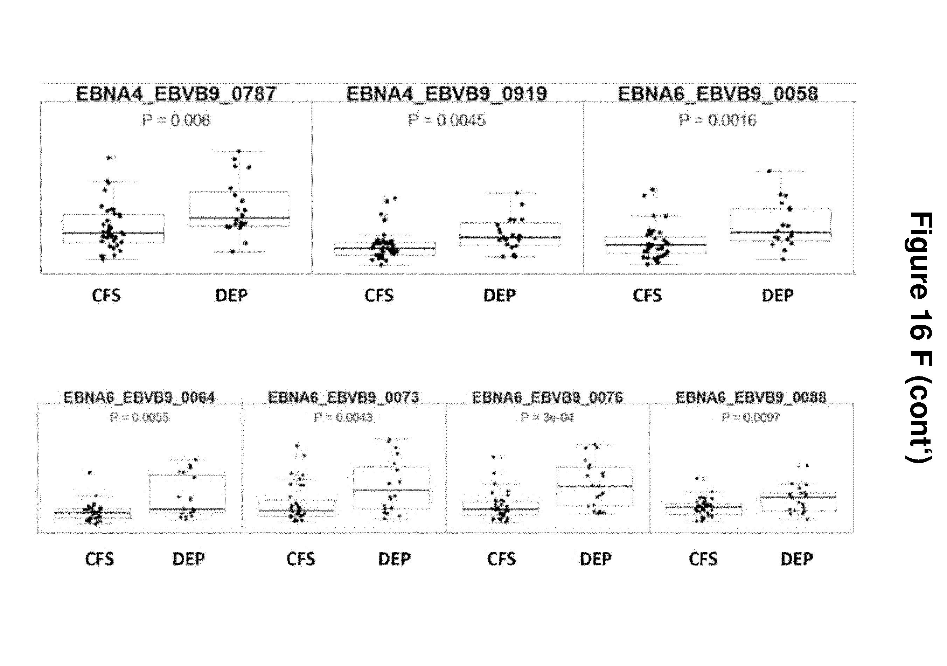

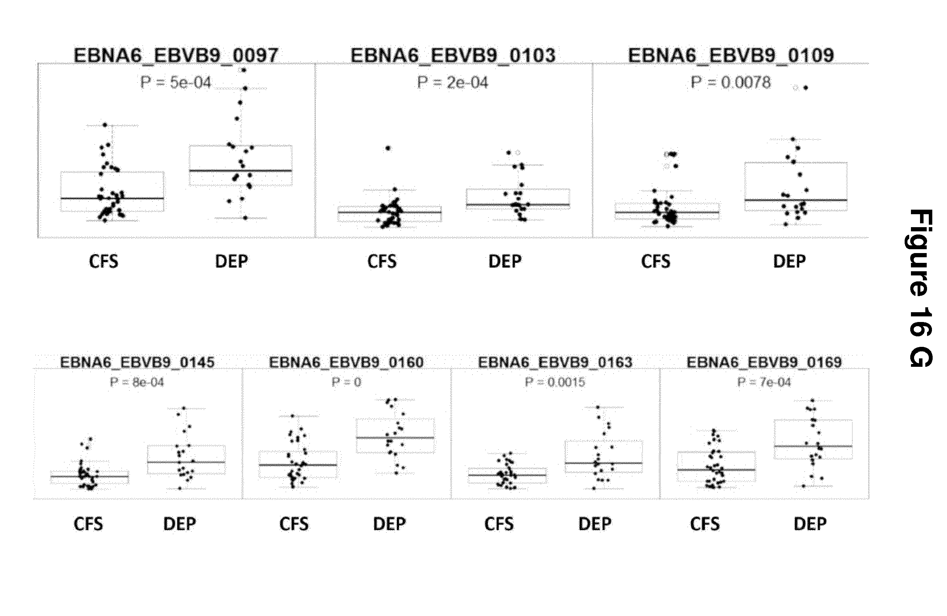

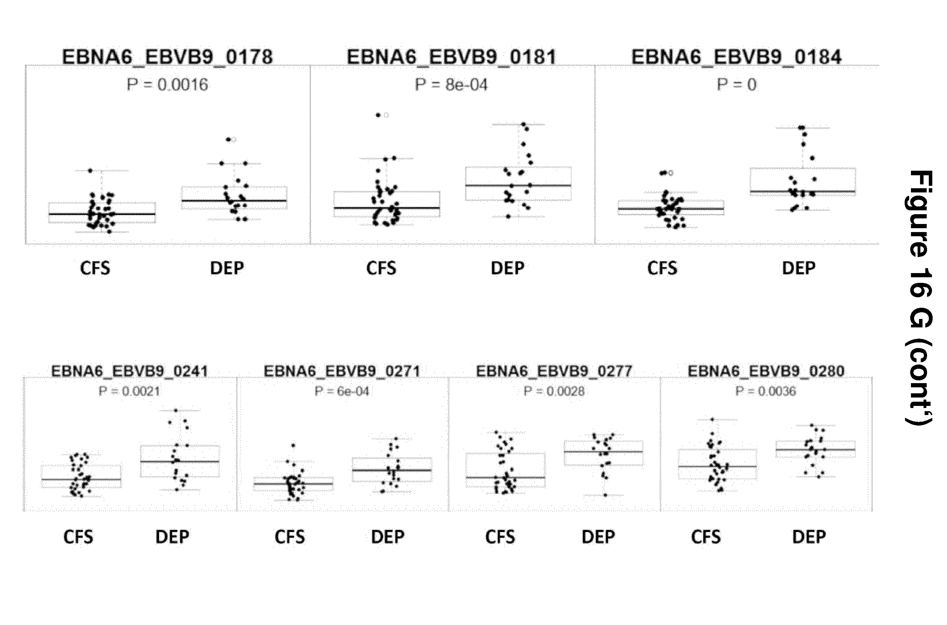

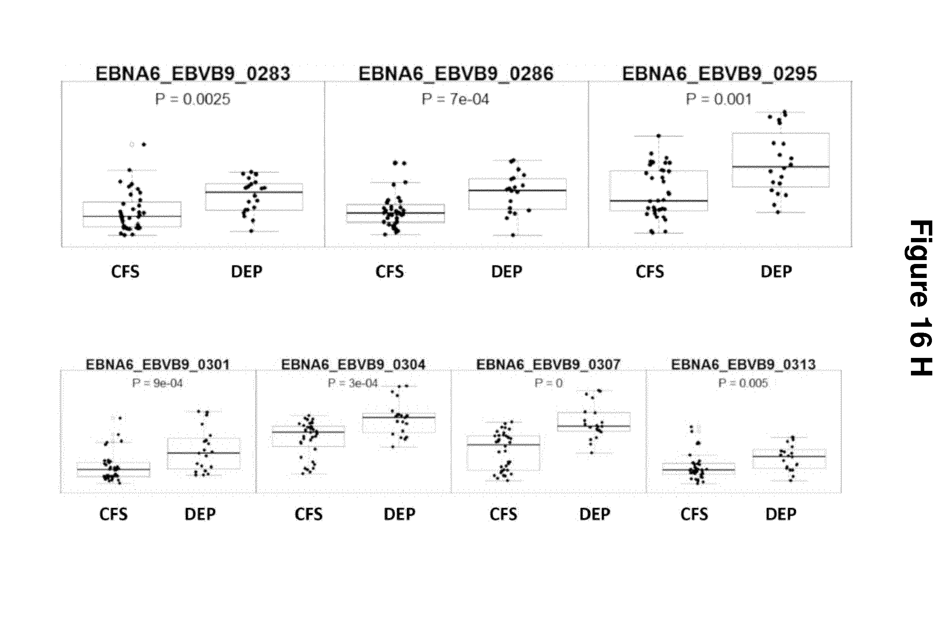

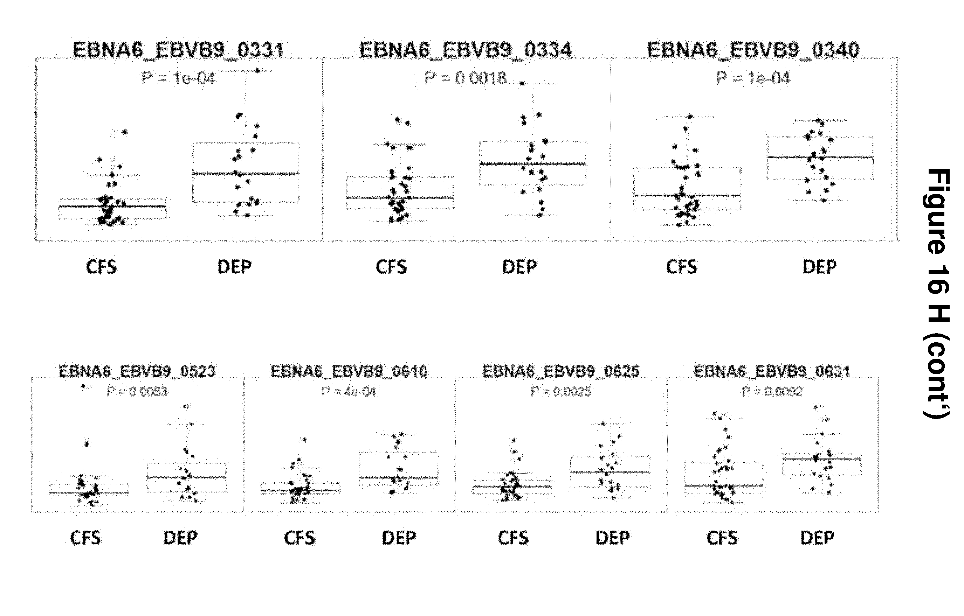

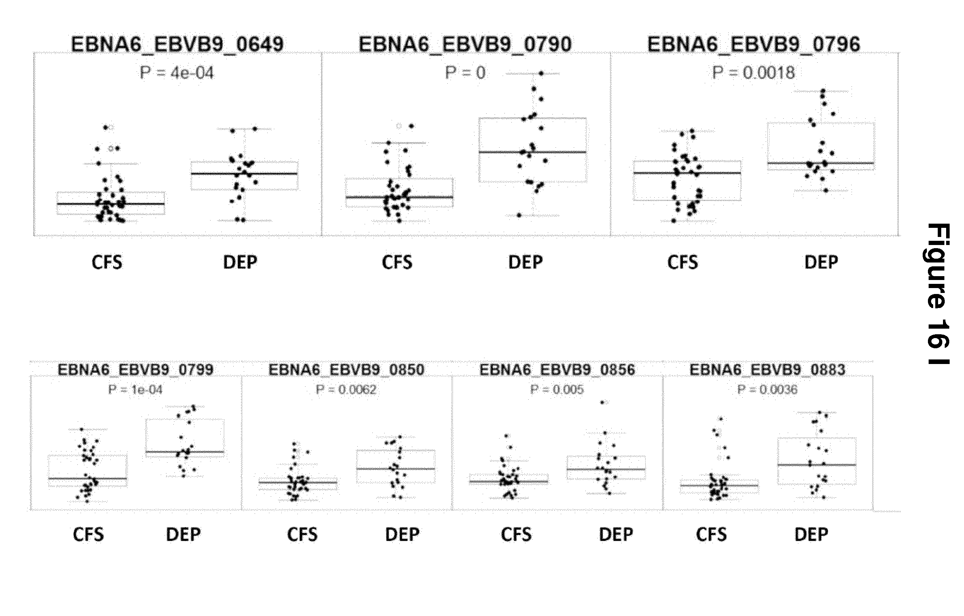

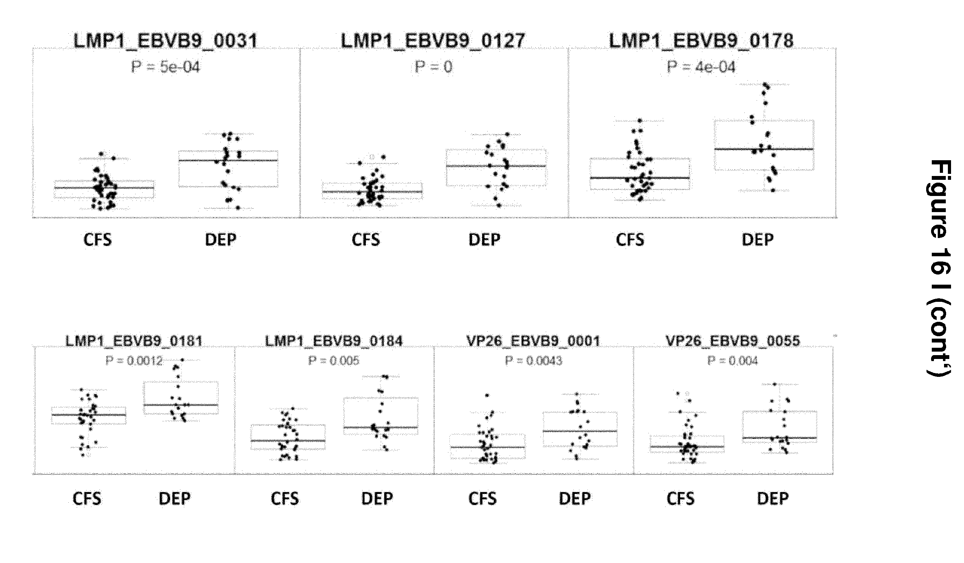

For the evaluation of the foregoing results, Boxplots of signal intensities for individual peptides fragments with a p-value < 0.01: CFS patients vs. Depression patients are shown in **Figure 16A to 16I****.**

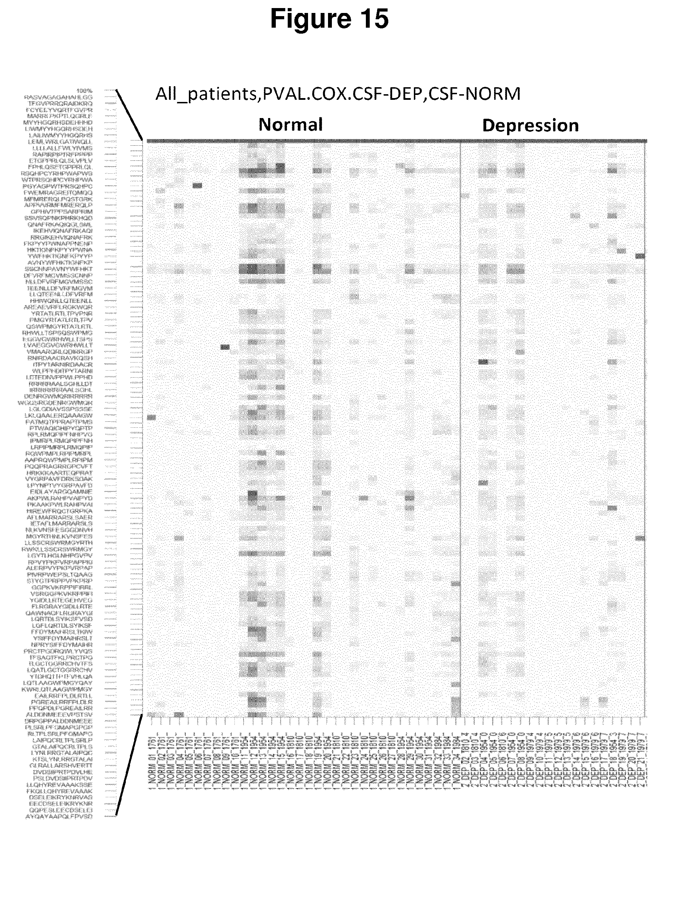

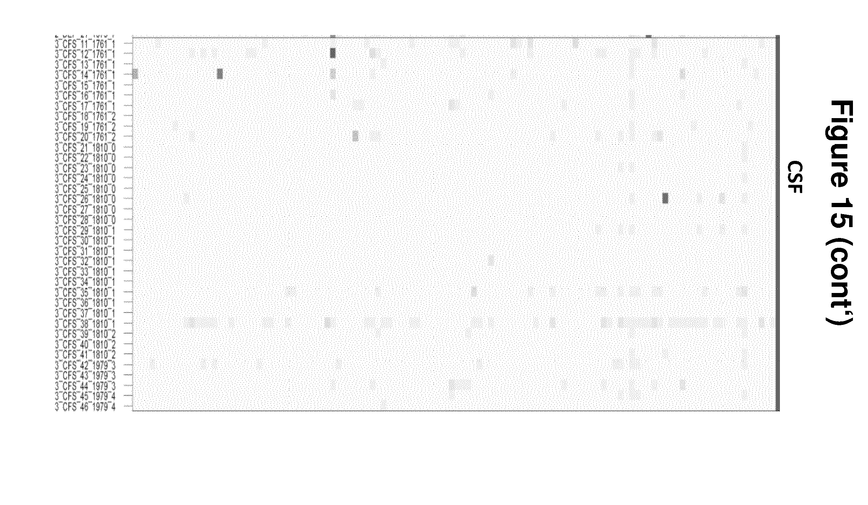

In **Figure 15** a p-value was calculated for each peptide for the null hypothesis that the distribution of signal intensities is equal between patient groups:
a) CFS-Patients vs. healthy volunteers (NORM)
b) CFS-Patients vs. Depression Patients (DEP).

## Claims

1. An *in vitro* method for diagnosing Chronic Fatigue Syndrome, the method comprising determining the presence, absence or the amount of at least one marker characteristic of Epstein-Barr virus (EBV) infection in a sample obtained from the body of an individual, wherein the marker is selected from the group consisting of EBNA1, EBNA3, EBNA4, EBNA6, BZLF1, LMP1 and VP26, and wherein the presence of Chronic Fatigue Syndrome can be concluded from the presence, absence or the amount of the marker in the sample.

2. The method of claim 1, wherein at least one, two or all of the markers EBNA1, EBNA3, EBNA4 and EBNA6 is absent in the sample and the absence indicates the presence of Chronic Fatigue Syndrome.

3. The method of claims 1 or 2, wherein at least one, two or all of the markers BZLF1 and VP26 is present in the sample and the presence indicates the presence of Chronic Fatigue Syndrome.

4. The method according to any one of the preceding claims, wherein the presence, absence or amount of immune response to at least one further marker, which is characteristic of Epstein-Barr virus infection, is determined.

5. The method according to any one of the preceding claims, wherein a distinction between Chronic Fatigue Syndrome and a physical disorder can be made by determining the presence, absence or amount of at least one of the markers EBNA3, EBNA4, and EBNA6, wherein the physical disorder can be depression or burnout syndrome.

6. The method according to any one of the preceding claims, wherein the presence, absence of the amount of the marker can be determined directly or indirectly by an assay suitable to detect the presence, absence or the amount of the marker.

7. The method according to claim 6, wherein the assay can determine the presence, absence or amount of each marker directly by measuring the presence, absence or amount of the corresponding protein or a fragment thereof or the corresponding nucleotide encoding the protein, or wherein the presence, absence or the amount of the marker can be determined indirectly by measuring the presence, absence of amount of an immune response to the corresponding protein or a fragment thereof.

8. The method according to claim 7, wherein the immune response is determined by an immunoassay.

9. The method according to claim 8, wherein the immunoassay comprises at least one protein fragment of 4 to 70 amino acids in length from the specified regions of the following sequences: amino acid 80 - 105 of SEQ ID NO.: 1; amino acid 158 - 245 of SEQ ID NO.: 1; amino acid 80 - 105 of SEQ ID NO.: 2; amino acid 158 - 245 of SEQ ID NO.: 2; amino acid 80 - 105 of SEQ ID NO.: 3; amino acid 158 - 245 of SEQ ID NO.: 3; amino acid 479 - 555 of SEQ ID NO.: 4; amino acid 479 - 555 of SEQ ID NO.: 5; amino acid 479 - 555 of SEQ ID NO.: 6; amino acid 1 - 30 of SEQ ID NO.: 7; amino acid 76 - 110 of SEQ ID NO.: 7; amino acid 154 - 431 of SEQ ID NO.: 7; amino acid 489 - 548 of SEQ ID NO.: 7; amino acid 784 - 828 of SEQ ID NO.: 7; amino acid 1 - 30 of SEQ ID NO.: 8; amino acid 77 - 111 of SEQ ID NO.: 8; amino acid 155 - 432 of SEQ ID NO.: 8; amino acid 503 - 567 of SEQ ID NO.: 8; amino acid 803 - 828 of SEQ ID NO.: 8; amino acid 1 - 30 of SEQ ID NO.: 9; amino acid 77 - 111 of SEQ ID NO.: 9; amino acid 155 - 432 of SEQ ID NO.: 9; amino acid 503 - 567 of SEQ ID NO.: 9; amino acid 794 - 819 of SEQ ID NO.: 9; amino acid 161 - 204 of SEQ ID NO.: 10; amino acid 230 - 259 of SEQ ID NO.: 10; amino acid 290 - 351 of SEQ ID NO.: 10; amino acid 383 - 450 of SEQ ID NO.: 10; amino acid 524 - 549 of SEQ ID NO.: 10; amino acid 614 - 719 of SEQ ID NO.: 10; amino acid 790 - 815 of SEQ ID NO.: 10; amino acid 922 - 946 of SEQ ID NO.: 10; amino acid 161 - 204 of SEQ ID NO.: 11; amino acid 230 - 259 of SEQ ID NO.: 11; amino acid 290 - 351 of SEQ ID NO.: 11; amino acid 383 - 450 of SEQ ID NO.: 11; amino acid 524 - 549 of SEQ ID NO.: 11; amino acid 614 - 711 of SEQ ID NO.: 11; amino acid 782 - 807 of SEQ ID NO.: 11; amino acid 914 - 938 of SEQ ID NO.: 11; amino acid 161 - 204 of SEQ ID NO.: 12; amino acid 230 - 259 of SEQ ID NO.: 12; amino acid 290 - 351 of SEQ ID NO.: 12; amino acid 383 - 450 of SEQ ID NO.: 12; amino acid 524 - 549 of SEQ ID NO.: 12; amino acid 614 - 711 of SEQ ID NO.: 12; amino acid 782 - 807 of SEQ ID NO.: 12; amino acid 914 - 938 of SEQ ID NO.: 12; amino acid 53 - 204 of SEQ ID NO.: 13; amino acid 236 - 306 of SEQ ID NO.: 13; amino acid 519 - 575 of SEQ ID NO.: 13; amino acid 632 - 696 of SEQ ID NO.: 13; amino acid 855 - 980 of SEQ ID NO.: 13; amino acid 53 - 204 of SEQ ID NO.: 14; amino acid 236 - 306 of SEQ ID NO.: 14; amino acid 518 - 543 of SEQ ID NO.: 14; amino acid 605 - 669 of SEQ ID NO.: 14; amino acid 785 - 903 of SEQ ID NO.: 14; amino acid 53 - 204 of SEQ ID NO.: 15; amino acid 236 - 306 of SEQ ID NO.: 15; amino acid 518 - 543 of SEQ ID NO.: 15; amino acid 570 - 634 of SEQ ID NO.: 15; amino acid 802 - 920 of SEQ ID NO.: 15; amino acid 26 - 51 of SEQ ID NO.: 16; amino acid 122 - 147 of SEQ ID NO.: 16; amino acid 173 - 204 of SEQ ID NO.: 16; amino acid 26 - 51 of SEQ ID NO.: 17; amino acid 122 - 147 of SEQ ID NO.: 17; amino acid 173 - 204 of SEQ ID NO.: 17; amino acid 26 - 51 of SEQ ID NO.: 18; amino acid 122 - 147 of SEQ ID NO.: 18; amino acid 173 - 204 of SEQ ID NO.: 18; amino acid 26 - 51 of SEQ ID NO.: 19; amino acid 122 - 147 of SEQ ID NO.: 19; amino acid 173 - 204 of SEQ ID NO.: 19; amino acid 26 - 51 of SEQ ID NO.: 20; amino acid 122 - 147 of SEQ ID NO.: 20; amino acid 173 - 204 of SEQ ID NO.: 20; amino acid 1 - 21 of SEQ ID NO.: 21; or amino acid 50 - 99 of SEQ ID NO.: 21., or any one of SEQ ID NOs.:22-135.

10. The method according to claim 9, wherein the protein fragments are immobilized on a solid phase and brought into contact with the sample obtained from the body of the individual, to determine the immune response (antibody response) of the individual to at least one marker which are selected from the group consisting of EBNA1, EBNA3, EBNA4, EBNA6, BZLF1, LMP1 and VP26.

11. The method according to any one of the preceding claims, wherein the method further comprises determining the number of memory B cells of the individual, wherein the total number of memory B cells and the number of EBV-specific memory B cells is determined, wherein a reduction in the number of EBV-specific memory B cells relative to the number of EBV specific memory B-cells from a EBV seropositive control group of healthy individuals indicates the presence of Chronic Fatigue Syndrome.

12. The method according to any one of the preceding claims, wherein the method further comprises determining the response of TNF-alpha or interferon gamma-producing memory T cells to EBV protein or peptides, wherein a reduction of TNF-alpha or interferon gamma-producing memory T cells specific to EBV relative to the number of TNF-alpha or interferon gamma-producing memory T cells of an EBV seropositive control group of healthy individuals indicates the presence of Chronic Fatigue Syndrome.

13. A device for the diagnosis of Chronic Fatigue Syndrome, wherein the device comprises a solid phase having immobilized thereon a protein comprising or consisting of at least one marker or protein fragment thereof, wherein the marker is selected from the group consisting of EBNA1, EBNA3, EBNA4, EBNA6, BZLF1, LMP1 and VP26.

14. A protein comprising or consisting of at least one marker or protein fragment thereof for use in the diagnosis of Chronic Fatigue Syndrome, wherein the marker is selected from the group consisting of EBNA1 EBNA3, EBNA4, EBNA6, BZLF1, LMP1 and VP26.

15. The use of claims 1 to 12, the device of claim 13 or the protein of claim 14, wherein at least one of the markers is EBNA3, EBNA4 or EBNA6 or a fragment thereof.
